# EUROPEAN PATENT APPLICATION

(11) **EP 2 746 274 A1**
(43) Date of publication of application: **25.06.2014**
(21) Application number: 12198083.3
(22) Date of filing: 19.12.2012
(51) Int. Cl.: C07D 405/06, A01N 43/50, A01N 43/653

(54) **Substituted [1,2,4]triazole compounds**

(71) Applicant: BASF SE, 67056 Ludwigshafen (DE)
(72) Inventor: Craig, Ian Robert, 67063 Ludwigshafen (DE); Grammenos, Wassilios, 67071 Ludwigshafen (DE); Boudet, Nadege, 69502 Hemsbach (DE); Müller, Bernd, 67227 Frankenthal (DE); Dietz, Jochen, 76227 Karlsruhe (DE); Lauterwasser, Erica May Wilson, 47157 Wachenheim (DE); Lohmann, Jan Klaas, 67245 Lambsheim (DE); Grote, Thomas, 67157 Wachenheim (DE); Haden, Egon, 67346 Speyer (DE); Escribano Cuesta, Ana, 68167 Mannheim (DE)

(57) **Abstract**

The present invention relates to compounds of the formula I 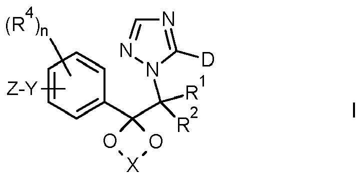 wherein the substituents are defined in the description and claims, their preparation and uses thereof.

## Description

The present invention relates to substituted [1,2,4]triazol compounds and the N-oxides and the salts thereof for combating phytopathogenic fungi, and to the use and methods for combating phytopathogenic fungi and to seeds coated with at least one such compound. The invention also relates to processes for preparing these compounds, intermediates, processes for preparing such intermediates, and to compositions comprising at least one compound I.

EP 0 029 355 relates to azolyl ketals, their preparation and use and microbicidal agents containing them. US 4,479,004 relates to 1-[2-(4-diphenyl)-1,3-dioxolan-2-yl-methyl]-1H-triazoles and their use for combatting microorganisms.

In many cases, in particular at low application rates, the fungicidal activity of the known fungicidal compounds is unsatisfactory. Based on this, it was an object of the present invention to provide compounds having improved activity and/or a broader activity spectrum against phytopathogenic harmful fungi.

Surprisingly, this object is achieved by the use of the inventive substituted [1,2,4]triazol and imidazole compounds of formula I having favorable fungicidal activity against phytopathogenic fungi.

Accordingly, the present invention relates, to the compounds of the formula I 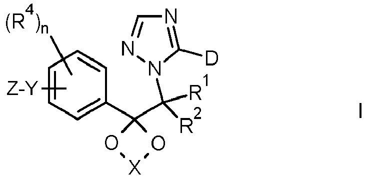 in which
D is H, halogen or SR^{D}, wherein
R^{D} is hydrogen, C₁-C₆-alkyl, C₁-C₆-haloalkyl, C₂-C₆-alkenyl, C₂-C₆-haloalkenyl, C₂-C₆-alkynyl, C₂-C₆-haloalkynyl or CN;
R¹,R² are hydrogen;
X is -CR⁵R⁶-CR⁷R⁸- or -CR⁵R⁶-CR⁷R⁸-CR⁹R¹⁰-, wherein
R⁵,R⁶,R⁷,R⁸,R⁹,R¹⁰ are independently selected from hydrogen, halogen, C₁-C₆-alkyl, C₂-C₆-alkenyl, C₂-C₆-alkynyl, C₁-C₆-alkoxy and phenyl; wherein these substituents are unsubstituted or substituted by one, two, three or up to the maximum possible number of R^{x}; wherein
R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy
Y is a direct bond or a divalent group selected from the group consisting of -O-, -S-, -SO-, - SO₂-, -NH-, -N(C₁-C₄-alkyl)-, -CR¹²R¹³-, -CR¹²R¹³-CR¹⁴R¹⁵-, -CR¹⁶=CR¹⁷-and -C≡C-;
wherein
R¹²,R¹³R¹⁴,R¹⁵,R¹⁶,R¹⁷ are independently selected from hydrogen, halogen, CN, nitro, OH, C₁-C₄-alkyl, C₁-C₄-halogenalkyl, C₁-C₄-alkoxy and C₁-C₄-halogenalkoxy;
Z is five or six-membered heteroaryl or phenyl, wherein the heteroaryl contains 1, 2, 3 or 4 heteroatoms selected from the group consisting of O, N and S, and wherein the heteroaryl or phenyl is unsubstituted (m=0) or substituted by (R^{L})ₘ, wherein
m is 0, 1, 2, 3 or 4; and wherein
R^{L} is independently selected from halogen, CN, NO₂, OH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyl-C₁-C₄-alkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl and phenyl-C₁-C₄-alkyl, wherein the aliphatic, alicyclic and aromatic moieties of R^{L} are unsubstituted or substituted by one, two, three or four or up to the maximum possible number of R^{La}; wherein
R^{La} is independently selected from halogen, CN, NO₂, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio and C₁-C₆-haloalkylthio;
R⁴ is independently selected from halogen, CN, NO₂, OH, SH, C₁-C₄-alkyl, C₁C₁-C₄-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyl-C₁-C₄-alkyl, C₃-C₈-cycloalkyloxy, phenyl, phenoxy, 5- or 6-membered heteroaryl, 5- or 6-membered heteroaryloxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl) and C(=O)-N(C₃-C₆-cycloalkyl)₂; wherein the aliphatic, alicyclic and aromatic moieties of R⁴ are unsubstituted or substituted by one, two, three or four or up to the maximum possible number of R^{4a}; wherein
R^{4a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-halogenalkoxy;
n is 0, 1, 2, 3 or 4;
with the proviso, that
if Z is phenyl, Y is a direct bond, n = 0 and D=H, the phenyl is substituted by (R^{L})ₘ with m=1, 2, 3 or 4; and
if Z is phenyl and Y is a divalent group selected from the group consisting of -O-, -S-, -NH- and - N(C₁-C₄-alkyl)-, Z-Y is not in the para-position in relation to the attachment of the phenyl group in formula I to the remaining backbone structure;
and the N-oxides and the agriculturally acceptable salts thereof.

Compounds I can be synthesized starting from acetophenones II carrying halogen=X that is commercially available or can be provided by the skilled person by methods known in the art. Compound II can be transformed to compound III using transition metal catalysis. The X (I,Br) in II can be transformed to compounds III with Y being and amino chain (Synlett, (8), 1137-1142; 2011, European Journal of Organic Chemistry, (17), 3219-3223, S3219/1-S3219/38; 2010; Advanced Synthesis & Catalysis, 350(3), 395-398; 2008), Y=-S- (Organic & Biomolecular Chemistry (2012), 10(13), 2562-2568; Organic Letters (2011), 13(15), 4100-4103; ), Y=-O (Organic Letters, 14(1), 170-173; 2012; Journal of Organic Chemistry, 74(18), 7187-7190; 2009; Synlett (2011),(2), 268-272), Y=-C(R)=C(R)-(Tetrahedron, 68(36), 7309-7316; 2012; Journal of Organometallic Chemistry, 344(2), 253-9; 1988; WO 2004046068 A2; Tetrahedron, 61(1), 259-266; 2004), -C≡C- (EP 648723 A1; WO 2010099527; Organic Letters (2002), 4(24), 4305-4307). Halogenation of the ketone leads to halo ketons IV, that can be subsequently transformed into compounds V using an azole compound and a base.

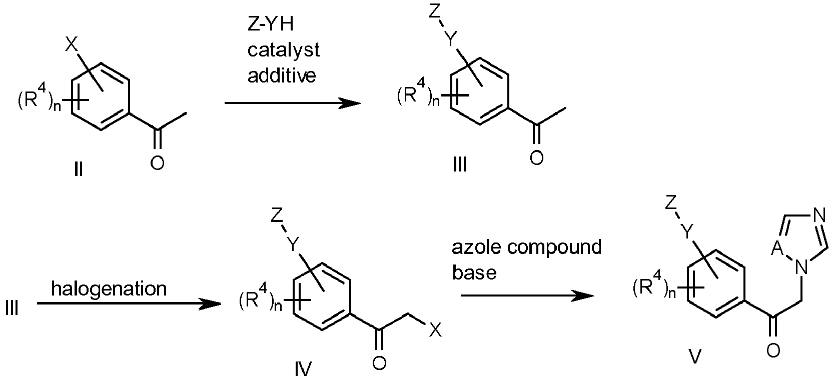

Compounds V can be transformed to substituted ketones VI (DE 3000244 A1, Ger. Offen., 3440114, 07 May 1986; Journal of Medicinal Chemistry, 30(8), 1497-502; 1987; Gaodeng Xuexiao Huaxue Xuebao, 13(8), 1084-6; 1992; Gaodeng Xuexiao Huaxue Xuebao, 16(9), 1396-9; 1995; Gaodeng Xuexiao Huaxue Xuebao, 24(3), 431-435; 2003; Faming Zhuanli Shenqing, 101323600, 17 Dec 2008; Heterocycles (2001), 55(11), 2059-2074; Journal of Medicinal Chemistry (1987), 30(8), 1497-502; DE3140276A1; Jpn. Kokai Tokkyo Koho, 2011251945, 15 Dec 2011, DE 3019028 A1)

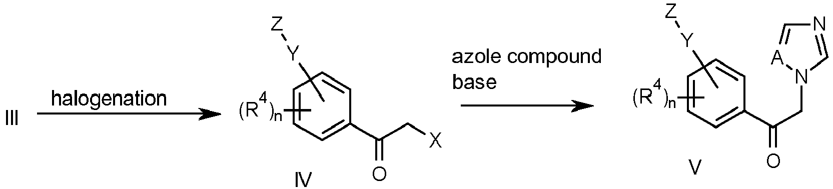

Ketalization with an approtiate diol compound leads to compounds 1.1 (Arzneimittel-Forschung, 53(11), 758-762; 2003; Journal of Medicinal Chemistry, 48(6), 2184-2193; 2005; Bioorganic & Medicinal Chemistry Letters (2012), 22(4), 1625-1628; WO 2010146114 A1; CN 1631888 A1)

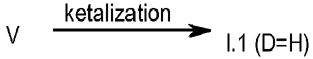

Compounds I.1 can be transformed into I, wherein D is different from H using a strong base (e.g. BuLi, LDA, LHMDS, KHMDS, BuLi, LTMP, Zn-TMP) an an electrophile E+(S₈, I₂, ICI, C₂F₄Br₂) to obtain substituted azole compounds (WO 2012025506 A1, Synthesis, (1), 100-106; 1999).

The N-oxides may be prepared from the inventive compounds according to conventional oxidation methods, e. g. by treating compounds I with an organic peracid such as metachloroper-benzoic acid (cf. WO 03/64572 or J. Med. Chem. 38(11), 1892-903, 1995); or with inorganic oxidizing agents such as hydrogen peroxide (cf. J. Heterocyc. Chem. 18(7), 1305-8, 1981) or oxone (cf. J. Am. Chem. Soc. 123(25), 5962-5973, 2001). The oxidation may lead to pure mono-N-oxides or to a mixture of different N-oxides, which can be separated by conventional methods such as chromatography.

If the synthesis yields mixtures of isomers, a separation is generally not necessarily required since in some cases the individual isomers can be interconverted during work-up for use or during application (e. g. under the action of light, acids or bases). Such conversions may also take place after use, e. g. in the treatment of plants in the treated plant, or in the harmful fungus to be controlled.

In the following, the intermediate compounds are further described. A skilled person will readily understand that the preferences for the substituents, also in particular the ones given in the tables below for the respective substituents, given herein in connection with compounds I apply for the intermediates accordingly. Thereby, the substituents in each case have independently of each other or more preferably in combination the meanings as defined herein.

Compounds of formula III are at least partially new. Consequently, a further embodiment of the present invention are compounds of formula III (see above), wherein the variables are as defined and preferably defined for formula I herein.

Compounds of formula IV are at least partially new. Consequently, a further embodiment of the present invention are compounds of formula III (see above), wherein the variables are as defined and preferably defined for formula I herein.

Compounds of formula V are at least partially new. Consequently, a further embodiment of the present invention are compounds of formula V (see above), wherein the variables are as defined and preferably defined for formula I herein.

In the definitions of the variables given above, collective terms are used which are generally representative for the substituents in question. The term "Cₙ-Cₘ" indicates the number of carbon atoms possible in each case in the substituent or substituent moiety in question.

The term "halogen" refers to fluorine, chlorine, bromine and iodine.

The term "C₁-C₆-alkyl" refers to a straight-chained or branched saturated hydrocarbon group having 1 to 6 carbon atoms, e.g. methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl. Likewise, the term "C₂-C₄-alkyl" refers to a straight-chained or branched alkyl group having 2 to 4 carbon atoms, such as ethyl, propyl (n-propyl), 1-methylethyl (iso-propoyl), butyl, 1-methylpropyl (sec.-butyl), 2-methylpropyl (iso-butyl), 1,1-dimethylethyl (tert.-butyl).
The term "C₁-C₆-haloalkyl" refers to an alkyl group having 1 or 6 carbon atoms as defined above, wherein some or all of the hydrogen atoms in these groups may be replaced by halogen atoms as mentioned above. Examples are "C₁-C₂-haloalkyl" groups such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl or pentafluoroethyl.

The term "C₁-C₆-hydroxyalkyl" refers to an alkyl group having 1 or 6 carbon atoms as defined above, wherein some or all of the hydrogen atoms in these groups may be replaced by OH groups.

The term "C₂-C₆-alkenyl" refers to a straight-chain or branched unsaturated hydrocarbon radical having 2 to 6 carbon atoms and a double bond in any position. Examples are "C₂-C₄-alkenyl" groups, such as ethenyl, 1-propenyl, 2-propenyl (allyl), 1-methylethenyl, 1-butenyl, 2-butenyl, 3-butenyl, 1-methyl-1-propenyl, 2-methyl-1-propenyl, 1-methyl-2-propenyl, 2-methyl-2-propenyl. The term "C₂-C₆-alkynyl" refers to a straight-chain or branched unsaturated hydrocarbon radical having 2 to 6 carbon atoms and containing at least one triple bond. Examples are "C₂-C₄-alkynyl" groups, such as ethynyl, prop-1-ynyl, prop-2-ynyl (propargyl), but-1-ynyl, but-2-ynyl, but-3-ynyl, 1-methyl-prop-2-ynyl.

The term "C₃-C₈-cycloalkyl" refers to monocyclic saturated hydrocarbon radicals having 3 to 8 carbon ring members, such as cyclopropyl, cyclobutyl, cyclopentyl, cyclohexyl, cycloheptyl or cyclooctyl.

The term "C₃-C₈-cycloalkyl-C₁-C₄-alkyl" refers to alkyl having 1 to 4 carbon atoms (as defined above), wherein one hydrogen atom of the alkyl radical is replaced by a cycloalkyl radical having 3 to 8 carbon atoms (as defined above).

The term "C₁-C₆-alkoxy" refers to a straight-chain or branched alkyl group having 1 to 6 carbon atoms which is bonded via an oxygen, at any position in the alkyl group. Examples are "C₁-C₄-alkoxy" groups, such as methoxy, ethoxy, n-propoxy, 1-methylethoxy, butoxy, 1-methyl¬propoxy, 2-methylpropoxy or 1,1-dimethylethoxy.

The term "C₁-C₆-haloalkoxy" refers to a C₁-C₆-alkoxy radical as defined above, wherein some or all of the hydrogen atoms in these groups may be replaced by halogen atoms as mentioned above. Examples are "C₁-C₄-haloalkoxy" groups, such as OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloro¬ethoxy, OC₂F₅, 2-fluoropropoxy, 3-fluoropropoxy, 2,2-difluoropropoxy, 2,3-difluoro¬propoxy, 2 chloropropoxy, 3-chloropropoxy, 2,3-dichloropropoxy, 2-bromo¬propoxy, 3 bromopropoxy, 3,3,3-trifluoropropoxy, 3,3,3-trichloropropoxy, OCH₂-C₂F₅, OCF₂-C₂F₅, 1-fluoromethyl-2-fluoroethoxy, 1-chloromethyl-2-chloroethoxy, 1-bromomethyl-2-bromo¬ethoxy, 4-fluorobutoxy, 4-chlorobutoxy, 4-bromobutoxy or nonafluorobutoxy.

The term "phenyl-C₁-C₆-alkyl" refers to alkyl having 1 to 6 carbon atoms (as defined above), wherein one hydrogen atom of the alkyl radical is replaced by a phenyl radical. Likewise, the terms "phenyl-C₂-C₆-alkenyl" and "phenyl-C₂-C₆-alkynyl" refer to alkenyl and alkynyl, respectively, wherein one hydrogen atom of the aforementioned radicals is replaced by a phenyl radical. The term "C₁-C₄-alkoxy-C₁-C₄-alkyl" refers to alkyl having 1 to 4 carbon atoms (as defined above), wherein one hydrogen atom of the alkyl radical is replaced by a C₁-C₄-alkoxy group (as defined above). Likewise, the term "C₁-C₆-alkoxy-C₁-C₄-alkyl" refers to alkyl having 1 to 4 carbon atoms (as defined above), wherein one hydrogen atom of the alkyl radical is replaced by a C₁-C₆-alkoxy group (as defined above).

The term "C₁-C₆-alkylthio" as used herein refers to straight-chain or branched alkyl groups having 1 to 6 carbon atoms (as defined above) bonded via a sulfur atom. Accordingly, the term "C₁-C₆-haloalkylthio" as used herein refers to straight-chain or branched haloalkyl group having 1 to 6 carbon atoms (as defined above) bonded through a sulfur atom, at any position in the haloalkyl group.

The term "C₁-C₆-alkylsulfinyl" refers to straight-chain or branched alkyl groups having 1 to 6 carbon atoms (as defined above) bonded through a -S(=O)- moiety, at any position in the alkyl group, for example methylsulfinyl and ethylsulfinyl, and the like. Accordingly, the term "C₁-C₆-haloalkylsulfinyl" refers to straight-chain or branched haloalkyl group having 1 to 6 carbon atoms (as defined above), bonded through a -S(=O)- moiety, at any position in the haloalkyl group. The term "C₁-C₆-alkylsulfonyl" refers to straight-chain or branched alkyl groups having 1 to 6 carbon atoms (as defined above), bonded through a -S(=O)₂- moiety, at any position in the alkyl group, for example methylsulfonyl. Accordingly, the term "C₁-C₆-haloalkylsulfonyl" refers to straight-chain or branched haloalkyl group having 1 to 6 carbon atoms (as defined above), bonded through a -S(=O)₂- moiety, at any position in the haloalkyl group.

The term "C₃-C₈-cycloalkyl-C₃-C₈-cycloalkyl" refers to a cycloalkyl radical having 3 to 8 carbon atoms (as defined above), which is substituted by a further cycloalkyl radical having 3 to 8 carbon atoms.

The term "C₃-C₈-cycloalkoxy" refers to a cycloalkyl radical having 3 to 8 carbon atoms (as defined above), which is bonded via an oxygen.

The term "C(=O)-C₁-C₄-alkyl" refers to a radical which is attached through the carbon atom of the group C(=O) as indicated by the number valence of the carbon atom. The number of valence of carbon is 4, that of nitrogen is 3. Likewise the following terms are to be construed: NH(C₁-C₄-alkyl), N(C₁-C₄-alkyl)₂, NH(C₃-C₆-cycloalkyl), N(C₃-C₆-cycloalkyl)₂, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂.

The term "saturated or partially unsaturated 3-, 4- 5-, 6- or 7-membered carbocycle" is to be understood as meaning both saturated or partially unsaturated carbocycles having 3, 4, 5, 6 or 7 ring members. Examples include cyclopropyl, cyclopentyl, cyclopentenyl, cyclopentadienyl, cyclohexyl, cyclohexenyl, cyclohexadienyl, cycloheptyl, cycloheptenyl, cycloheptadienyl, and the like.

The term "saturated or partially unsaturated 3-, 4-, 5-, 6-, or 7-membered heterocycle, wherein the ring member atoms of the heterocycle include besides carbon atoms 1, 2, 3 or 4 heteroatoms independently selected from the group of N, O and S", is to be understood as meaning both saturated and partially unsaturated heterocycles, for example:
a 3- or 4-membered saturated heterocycle which contains 1 or 2 heteroatoms from the group consisting of N, O and S as ring members such as oxirane, aziridine, thiirane, oxetane, azetidine, thiethane, [1,2]dioxetane, [1,2]dithietane, [1,2]diazetidine; and
a 5- or 6-membered saturated or partially unsaturated heterocycle which contains 1, 2 or 3 heteroatoms from the group consisting of N, O and S as ring members such as 2-tetrahydrofuranyl, 3-tetrahydrofuranyl, 2-tetrahydrothienyl, 3-tetrahydrothienyl, 2-pyrrolidinyl, 3-pyrrolidinyl,
3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 1,2,4-oxadiazolidin-3-yl, 1,2,4-oxadiazolidin-5-yl, 1,2,4-thiadiazolidin-3-yl, 1,2,4-thiadiazolidin-5-yl, 1,2,4-triazolidin-3-yl, 1,3,4-oxadiazolidin-2-yl, 1,3,4-thiadiazolidin-2-yl, 1,3,4-triazolidin-2-yl, 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 2-pyrrolin-2-yl, 2-pyrrolin-3-yl, 3-pyrrolin-2-yl, 3-pyrrolin-3-yl, 2-isoxazolin-3-yl, 3-isoxazolin-3-yl, 4-isoxazolin-3-yl, 2-isoxazolin-4-yl, 3-isoxazolin-4-yl, 4-isoxazolin-4-yl, 2-isoxazolin-5-yl, 3-isoxazolin-5-yl, 4-isoxazolin-5-yl, 2-isothiazolin-3-yl, 3-isothiazolin-3-yl, 4-isothiazolin-3-yl, 2-isothiazolin-4-yl, 3-isothiazolin-4-yl, 4-isothiazolin-4-yl, 2-isothiazolin-5-yl, 3-isothiazolin-5-yl, 4-isothiazolin-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 3,4-dihydrooxazol-2-yl, 3,4-dihydrooxazol-3-yl, 3,4-dihydrooxazol-4-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, 1,3,5-hexahydrotriazin-2-yl and 1,2,4-hexahydrotriazin-3-yl and also the corresponding -ylidene radicals; and
a 7-membered saturated or partially unsaturated heterocycle such as tetra- and hexahydroazepinyl, such as 2,3,4,5-tetrahydro[1H]azepin-1-,-2-,-3-,-4-,-5-,-6- or-7-yl, 3,4,5,6-tetra-hydro[2H]azepin-2-,-3-,-4-,-5-,-6- or-7-yl, 2,3,4,7-tetrahydro[1H]azepin-1-,-2-,-3-,-4-,-5-,-6- or-7-yl, 2,3,6,7-tetrahydro[1H]azepin-1-,-2-,-3-,-4-,-5-,-6- or-7-yl, hexahydroazepin-1-,-2-,-3- or-4-yl, tetra- and hexahydrooxepinyl such as 2,3,4,5-tetrahydro[1H]oxepin-2-,-3-,-4-,-5-,-6- or-7-yl, 2,3,4,7-tetrahydro[1H]oxepin-2-,-3-,-4-,-5-,-6- or-7-yl, 2,3,6,7-tetrahydro[1H]oxepin-2-, -3-,-4-,-5-,-6- or-7-yl, hexahydroazepin-1-,-2-,-3- or-4-yl, tetra- and hexahydro-1,3-diazepinyl, tetra- and hexahydro-1,4-diazepinyl, tetra- and hexahydro-1,3-oxazepinyl, tetra- and hexahydro-1,4-oxazepinyl, tetra- and hexahydro-1,3-dioxepinyl, tetra- and hexahydro-1,4-dioxepinyl and the corresponding -ylidene radicals; and

The term "5-or 6-membered heteroaryl" refers to aromatic ring systems incuding besides carbon atoms, 1, 2, 3 or 4 heteroatoms independently selected from the group consisting of N, O and S, for example,
a 5-membered heteroaryl such as pyrrol-1-yl, pyrrol-2-yl, pyrrol-3-yl, thien-2-yl, thien-3-yl, furan-2-yl, furan-3-yl, pyrazol-1-yl, pyrazol-3-yl, pyrazol-4-yl, pyrazol-5-yl, imidazol-1-yl, imidazol-2-yl, imidazol-4-yl, imidazol-5-yl, oxazol-2-yl, oxazol-4-yl, oxazol-5-yl, isoxazol-3-yl, isoxazol-4-yl, isoxazol-5-yl, thiazol-2-yl, thiazol-4-yl, thiazol-5-yl, isothiazol-3-yl, isothiazol-4-yl, isothiazol-5-yl, 1,2,4-triazolyl-1-yl, 1,2,4-triazol-3-yl 1,2,4-triazol-5-yl, 1,2,4-oxadiazol-3-yl, 1,2,4-oxadiazol-5-yl and 1,2,4-thiadiazol-3-yl, 1,2,4-thiadiazol-5-yl; or
a 6-membered heteroaryl, such as pyridin-2-yl, pyridin-3-yl, pyridin-4-yl, pyridazin-3-yl, pyridazin-4-yl, pyrimidin-2-yl, pyrimidin-4-yl, pyrimidin-5-yl, pyrazin-2-yl and 1,3,5-triazin-2-yl and 1,2,4-triazin-3-yl.

Agriculturally acceptable salts of the inventive compounds encompass especially the salts of those cations or the acid addition salts of those acids whose cations and anions, respectively, have no adverse effect on the fungicidal action of said compounds. Suitable cations are thus in particular the ions of the alkali metals, preferably sodium and potassium, of the alkaline earth metals, preferably calcium, magnesium and barium, of the transition metals, preferably manganese, copper, zinc and iron, and also the ammonium ion which, if desired, may carry one to four C₁-C₄-alkyl substituents and/or one phenyl or benzyl substituent, preferably diisopropylammonium, tetramethylammonium, tetrabutylammonium, trimethylbenzylammonium, furthermore phosphonium ions, sulfonium ions, preferably tri(C₁-C₄-alkyl)sulfonium, and sulfoxonium ions, preferably tri(C₁-C₄-alkyl)sulfoxonium. Anions of useful acid addition salts are primarily chloride, bromide, fluoride, hydrogensulfate, sulfate, dihydrogenphosphate, hydrogenphosphate, phosphate, nitrate, bicarbonate, carbonate, hexafluorosilicate, hexafluorophosphate, benzoate, and the anions of C₁-C₄-alkanoic acids, preferably formate, acetate, propionate and butyrate. They can be formed by reacting such inventive compound with an acid of the corresponding anion, preferably of hydrochloric acid, hydrobromic acid, sulfuric acid, phosphoric acid or nitric acid.
The inventive compounds can be present in atropisomers arising from restricted rotation about a single bond of asymmetric groups. They also form part of the subject matter of the present invention.

Depending on the substitution pattern, the compounds of formula I and their N-oxides may have one or more centers of chirality, in which case they are present as pure enantiomers or pure diastereomers or as enantiomer or diastereomer mixtures. Both, the pure enantiomers or diastereomers and their mixtures are subject matter of the present invention.

In the following, particular embodiments of the inventive compounds are described. Therein, specific meanings of the respective substituents are further detailled, wherein the meanings are in each case on their own but also in any combination with one another, particular embodiments of the present invention.

Furthermore, in respect of the variables, generally, the embodiments of the compounds I also apply to the intermediates.

D according to the present invention is hydrogen, halogen or SR^{D}, wherein R^{D} is hydrogen, CN, C₁-C₆-alkyl, C₁-C₆-haloalkyl, C₂-C₆-alkenyl, C₂-C₆-haloalkenyl, C₂-C₆-alkynyl or C₂-C₆-haloalkynyl.

In a preferred embodiment D is hydrogen, halogen, SH, SCN or S-CH₂-CH=CH₂ (S-allyl). According to one embodiment D is hydrogen. According to a further embodiment, D is halogen, in particular iodine. According to another preferred embodiment D is SR^{D}. According to a particular embodiment, R^{D} is H. In yet another preferred embodiment R^{D} is CN. In a further preferred embodiment R^{D} is -CH₂-CH=CH₂.

R¹ and R² according to the present invention are hydrogen.

X according to the invention is -CR⁵R⁶-CR⁷R⁸- or -CR⁵R⁶-CR⁷R⁸-CR⁹R¹⁰-, wherein
R⁵,R⁶,R⁷,R⁸,R⁹ and R¹⁰ are independently selected from hydrogen, halogen, C₁-C₆-alkyl, C₂-C₆-alkenyl, C₂-C₆-alkynyl, C₁-C₆-alkoxy and phenyl; wherein these substituents are unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy.

In one embodiment of the invention, X is -CR⁵R⁶-CR⁷R⁸-, wherein R⁵, R⁶, R⁷ and R⁸ are independently selected from the substituents and preferred substituents as defined below.

For every R⁵, R⁶, R⁷ and/or R⁸ that is present in -CR⁵R⁶-CR⁷R⁸-, the following embodiments and preferences apply independently of the meaning of any other R⁵, R⁶, R⁷ and/or R⁸ that may be present in the chain.

According to one embodiment, R⁵ is hydrogen.
According to a further embodiment, R⁵ is halogen, in particular Cl or F.
According to a further embodiment, R⁵ is C₁-C₆-alkyl, in particular C₁-C₄-alkyl, wherein the alkyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R⁵ is C₁-C₆-alkyl, in particular C₁-C₄-alkyl, such as methyl, ethyl or n-propyl. According to a further embodiment thereof, R⁵ is C₁-C₆-haloalkyl, in particular C₁-C₄-haloalkyl, more particularly C₁-C₂-haloalkyl. According to a further embodiment thereof, R⁵ is C₁-C₆-hydroxyalkyl, in particular C₁-C₄-hydroxyalkyl, more particularly C₁-C₂-hydroxyalkyl. According to a further embodiment thereof, R⁵ is C₁-C₆-phenylalkyl, in particular C₁-C₄-phenylalkyl, more particularly C₁-C₂-phenylalkyl. According to a further embodiment thereof, R⁵ is C₁-C₆-phenoxyalkyl, in particular C₁-C₄-phenoxyalkyl, more particularly C₁-C₂-phenoxyalkyl.

According to a further embodiment, R⁵ is C₂-C₆-alkenyl, in particular C₂-C₄-alkenyl, wherein the alkenyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R⁵ is C₂-C₆-alkenyl, in particular C₂-C₄-alkenyl, such as C₂-alkenyl. According to a further embodiment thereof, R⁵ is C₂-C₆-haloalkenyl, in particular C₂-C₄-haloalkenyl, more particularly C₂-haloalkenyl. According to a further embodiment thereof, R⁵ is C₂-C₆-hydroxyalkenyl, in particular C₂-C₄-hydroxyalkenyl, more particularly C₂-hydroxyalkenyl. According to a further embodiment thereof, R⁵ is C₂-C₆-phenylalkenyl, in particular C₂-C₄-phenylalkenyl, more particularly C₂-phenylalkenyl. According to a further embodiment thereof, R⁵ is C₂-C₆-phenoxyalkenyl, in particular C₂-C₄-phenoxyalkenyl, more particularly C₂-phenoxyalkenyl.

According to a further embodiment, R⁵ is C₂-C₆-alkynyl, in particular C₂-C₄-alkynyl, wherein the alkynyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R⁵ is C₂-C₆-alkynyl, in particular C₂-C₄-alkynyl, such as C₂-alkynyl. According to a further embodiment thereof, R⁵ is C₂-C₆-haloalkynyl, in particular C₂-C₄-haloalkynyl, more particularly C₂-haloalkynyl. According to a further embodiment thereof, R⁵ is C₂-C₆-hydroxyalkynyl, in particular C₂-C₄-hydroxyalkynyl, more particularly C₂-hydroxyalkynyl. According to a further embodiment thereof, R⁵ is C₂-C₆-phenylalkynyl, in particular C₂-C₄-phenylalkynyl, more particularly C₂-phenylalkynyl. According to a further embodiment thereof, R⁵ is C₂-C₆-phenoxyalkynyl, in particular C₂-C₄-phenoxyalkynyl, more particularly C₂-phenoxyalkynyl.

According to a further embodiment, R⁵ is C₁-C₆-alkoxy, in particular C₁-C₄-alkoxy, more specifically C₁-C₂-alkoxy.

According to a further embodiment, R⁵ is phenyl that is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₂-haloalkyl, C₃-C₆-cycloalkyl, C₃-C₆-halocycloalkyl, C₁-C₂-alkoxy and C₁-C₂-haloalkoxy.

According to one embodiment, R⁶ is hydrogen.
According to a further embodiment, R⁶ is halogen, in particular Cl or F.
According to a further embodiment, R⁶ is C₁-C₆-alkyl, in particular C₁-C₄-alkyl, wherein the alkyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R⁶ is C₁-C₆-alkyl, in particular C₁-C₄-alkyl, such as methyl, ethyl or n-propyl. According to a further embodiment thereof, R⁶ is C₁-C₆-haloalkyl, in particular C₁-C₄-haloalkyl, more particularly C₁-C₂-haloalkyl. According to a further embodiment thereof, R⁶ is C₁-C₆-hydroxyalkyl, in particular C₁-C₄-hydroxyalkyl, more particularly C₁-C₂-hydroxyalkyl. According to a further embodiment thereof, R⁶ is C₁-C₆-phenylalkyl, in particular C₁-C₄-phenylalkyl, more particularly C₁-C₂-phenylalkyl. According to a further embodiment thereof, R⁶ is C₁-C₆-phenoxyalkyl, in particular C₁-C₄-phenoxyalkyl, more particularly C₁-C₂-phenoxyalkyl.

According to a further embodiment, R⁶ is C₂-C₆-alkenyl, in particular C₂-C₄-alkenyl, wherein the alkenyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R⁶ is C₂-C₆-alkenyl, in particular C₂-C₄-alkenyl, such as C₂-alkenyl. According to a further embodiment thereof, R⁶ is C₂-C₆-haloalkenyl, in particular C₂-C₄-haloalkenyl, more particularly C₂-haloalkenyl. According to a further embodiment thereof, R⁶ is C₂-C₆-hydroxyalkenyl, in particular C₂-C₄-hydroxyalkenyl, more particularly C₂-hydroxyalkenyl. According to a further embodiment thereof, R⁶ is C₂-C₆-phenylalkenyl, in particular C₂-C₄-phenylalkenyl, more particularly C₂-phenylalkenyl. According to a further embodiment thereof, R⁶ is C₂-C₆-phenoxyalkenyl, in particular C₂-C₄-phenoxyalkenyl, more particularly C₂-phenoxyalkenyl.

According to a further embodiment, R⁶ is C₂-C₆-alkynyl, in particular C₂-C₄-alkynyl, wherein the alkynyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R⁶ is C₂-C₆-alkynyl, in particular C₂-C₄-alkynyl, such as C₂-alkynyl. According to a further embodiment thereof, R⁶ is C₂-C₆-haloalkynyl, in particular C₂-C₄-haloalkynyl, more particularly C₂-haloalkynyl. According to a further embodiment thereof, R⁶ is C₂-C₆-hydroxyalkynyl, in particular C₂-C₄-hydroxyalkynyl, more particularly C₂-hydroxyalkynyl. According to a further embodiment thereof, R⁶ is C₂-C₆-phenylalkynyl, in particular C₂-C₄-phenylalkynyl, more particularly C₂-phenylalkynyl. According to a further embodiment thereof, R⁶ is C₂-C₆-phenoxyalkynyl, in particular C₂-C₄-phenoxyalkynyl, more particularly C₂-phenoxyalkynyl.

According to a further embodiment, R⁶ is C₁-C₆-alkoxy, in particular C₁-C₄-alkoxy, more specifically C₁-C₂-alkoxy.

According to a further embodiment, R⁶ is phenyl that is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, Cₗ-C₄-alkyl, C₁-C₂-haloalkyl, C₃-C₆-cycloalkyl, C₃-C₆-halocycloalkyl, C₁-C₂-alkoxy and C₁-C₂-haloalkoxy.

According to one embodiment, R⁷ is hydrogen.
According to a further embodiment, R⁷ is halogen, in particular Cl or F.
According to a further embodiment, R⁷ is C₁-C₆-alkyl, in particular C₁-C₄-alkyl, wherein the alkyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R⁷ is C₁-C₆-alkyl, in particular C₁-C₄-alkyl, such as methyl, ethylor n-propyl. According to a further embodiment thereof, R⁷ is C₁-C₆-haloalkyl, in particular C₁-C₄-haloalkyl, more particularly C₁-C₂-haloalkyl. According to a further embodiment thereof, R⁷ is C₁-C₆-hydroxyalkyl, in particular C₁-C₄-hydroxyalkyl, more particularly C₁-C₂-hydroxyalkyl. According to a further embodiment thereof, R⁷ is C₁-C₆-phenylalkyl, in particular C₁-C₄-phenylalkyl, more particularly C₁-C₂-phenylalkyl. According to a further embodiment thereof, R⁷ is C₁-C₆-phenoxyalkyl, in particular C₁-C₄-phenoxyalkyl, more particularly C₁-C₂-phenoxyalkyl.

According to a further embodiment, R⁷ is C₂-C₆-alkenyl, in particular C₂-C₄-alkenyl, wherein the alkenyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R⁷ is C₂-C₆-alkenyl, in particular C₂-C₄-alkenyl, such as C₂-alkenyl. According to a further embodiment thereof, R⁷ is C₂-C₆-haloalkenyl, in particular C₂-C₄-haloalkenyl, more particularly C₂-haloalkenyl. According to a further embodiment thereof, R⁷ is C₂-C₆-hydroxyalkenyl, in particular C₂-C₄-hydroxyalkenyl, more particularly C₂-hydroxyalkenyl. According to a further embodiment thereof, R⁷ is C₂-C₆-phenylalkenyl, in particular C₂-C₄-phenylalkenyl, more particularly C₂-phenylalkenyl. According to a further embodiment thereof, R⁷ is C₂-C₆-phenoxyalkenyl, in particular C₂-C₄-phenoxyalkenyl, more particularly C₂-phenoxyalkenyl.

According to a further embodiment, R⁷ is C₂-C₆-alkynyl, in particular C₂-C₄-alkynyl, wherein the alkynyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R⁷ is C₂-C₆-alkynyl, in particular C₂-C₄-alkynyl, such as C₂-alkynyl. According to a further embodiment thereof, R⁷ is C₂-C₆-haloalkynyl, in particular C₂-C₄-haloalkynyl, more particularly C₂-haloalkynyl. According to a further embodiment thereof, R⁷ is C₂-C₆-hydroxyalkynyl, in particular C₂-C₄-hydroxyalkynyl, more particularly C₂-hydroxyalkynyl. According to a further embodiment thereof, R⁷ is C₂-C₆-phenylalkynyl, in particular C₂-C₄-phenylalkynyl, more particularly C₂-phenylalkynyl. According to a further embodiment thereof, R⁷ is C₂-C₆-phenoxyalkynyl, in particular C₂-C₄-phenoxyalkynyl, more particularly C₂-phenoxyalkynyl.

According to a further embodiment, R⁷ is C₁-C₆-alkoxy, in particular C₁-C₄-alkoxy, more specifically C₁-C₂-alkoxy.

According to a further embodiment, R⁷ is phenyl that is unsubstituted or substituted by one, two, three or up to the maximum possible number of R^{x}; wherein R^{x} is independently selected from halogen, CN, OH, SH, NH_{2,} C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₂-haloalkyl, C₃-C₆-cycloalkyl, C₃-C₆-halocycloalkyl, C₁-C₂-alkoxy and C₁-C₂-haloalkoxy.

According to one embodiment, R⁸ is hydrogen.
According to a further embodiment, R⁸ is halogen, in particular Cl or F.
According to a further embodiment, R⁸ is C₁-C₆-alkyl, in particular C₁-C₄-alkyl, wherein the alkyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R⁸ is C₁-C₆-alkyl, in particular C₁-C₄-alkyl, such as methyl, ethyl or n-propyl. According to a further embodiment thereof, R⁸ is C₁-C₆-haloalkyl, in particular C₁-C₄-haloalkyl, more particularly C₁-C₂-haloalkyl. According to a further embodiment thereof, R⁸ is C₁-C₆-hydroxyalkyl, in particular C₁-C₄-hydroxyalkyl, more particularly C₁-C₂-hydroxyalkyl. According to a further embodiment thereof, R⁸ is C₁-C₆-phenylalkyl, in particular C₁-C₄-phenylalkyl, more particularly C₁-C₂-phenylalkyl. According to a further embodiment thereof, R⁸ is C₁-C₆-phenoxyalkyl, in particular C₁-C₄-phenoxyalkyl, more particularly C₁-C₂-phenoxyalkyl.

According to a further embodiment, R⁸ is C₂-C₆-alkenyl, in particular C₂-C₄-alkenyl, wherein the alkenyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R⁸ is C₂-C₆-alkenyl, in particular C₂-C₄-alkenyl, such as C₂-alkenyl. According to a further embodiment thereof, R⁸ is C₂-C₆-haloalkenyl, in particular C₂-C₄-haloalkenyl, more particularly C₂-haloalkenyl. According to a further embodiment thereof, R⁸ is C₂-C₆-hydroxyalkenyl, in particular C₂-C₄-hydroxyalkenyl, more particularly C₂-hydroxyalkenyl. According to a further embodiment thereof, R⁸ is C₂-C₆-phenylalkenyl, in particular C₂-C₄-phenylalkenyl, more particularly C₂-phenylalkenyl. According to a further embodiment thereof, R⁸ is C₂-C₆-phenoxyalkenyl, in particular C₂-C₄-phenoxyalkenyl, more particularly C₂-phenoxyalkenyl.

According to a further embodiment, R⁸ is C₂-C₆-alkynyl, in particular C₂-C₄-alkynyl, wherein the alkynyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R⁸ is C₂-C₆-alkynyl, in particular C₂-C₄-alkynyl, such as C₂-alkynyl. According to a further embodiment thereof, R⁸ is C₂-C₆-haloalkynyl, in particular C₂-C₄-haloalkynyl, more particularly C₂-haloalkynyl. According to a further embodiment thereof, R⁸ is C₂-C₆-hydroxyalkynyl, in particular C₂-C₄-hydroxyalkynyl, more particularly C₂-hydroxyalkynyl. According to a further embodiment thereof, R⁸ is C₂-C₆-phenylalkynyl, in particular C₂-C₄-phenylalkynyl, more particularly C₂-phenylalkynyl. According to a further embodiment thereof, R⁸ is C₂-C₆-phenoxyalkynyl, in particular C₂-C₄-phenoxyalkynyl, more particularly C₂-phenoxyalkynyl.

According to a further embodiment, R⁸ is C₁-C₆-alkoxy, in particular C₁-C₄-alkoxy, more specifically C₁-C₂-alkoxy.

According to a further embodiment, R⁸ is phenyl that is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₂-haloalkyl, C₃-C₆-cycloalkyl, C₃-C₆-halocycloalkyl, C₁-C₂-alkoxy and C₁-C₂-haloalkoxy.

According to one specific embodiment R⁵, R⁶, R⁷ and R⁸ are independently selected from hydrogen, C₁-C₆-alkyl, C₁-C₆-haloalkyl, C₁-C₆-hydroxyalkyl and C₁-C₆-phenylalkyl and as particularly defined above. According to one specific embodiment thereof, R⁵ and R⁶ are hydrogen, and in a particular embodiment, at the same time, both R⁷ and R⁸ are selected from methyl, ethyl, n-propyl, in particular methyl. According to a further specific embodiment thereof, R⁵ and R⁷ are hydrogen, and in a particular embodiment, at the same time, both R⁶ and R⁸ are selected from methyl, ethyl, n-propyl, in particular methyl. According to a further specific embodiment thereof R⁵, R⁶ and R⁷ are hydrogen, and in a particular embodiment, at the same time, R⁸ are selected from methyl, ethyl, n-propyl, in particular methyl. According to a further specific embodiment thereof R⁵, R⁶, R⁷ and R⁸ are hydrogen.

In one further embodiment X is -CR⁵H-CR⁷H-.
In still one further embodiment X is -CH₂-CR⁷H-.
In still one further embodiment X is -CR⁵H-CR⁷R⁸-.
In still one further embodiment X is -CH₂-CR⁷R⁸-.

According to one embodiment, -CR⁵R⁶-CR⁷R⁸- contains at least one substituent, such as one, two, three or four substituents, in particular exactly one substituent, as defined and preferably defined above.

According to a further embodiment, -CR⁵R⁶-CR⁷R⁸- contains at least two substituents, such as two, three or four, in particular exactly two substituents, as defined and preferably defined above.

According to a further embodiment, -CR⁵R⁶-CR⁷R⁸- contains at least three substituents, such as three or four substituents, in particular exactly three substituents, as defined and preferably defined above.

According to still a further embodiment, -CR⁵R⁶-CR⁷R⁸- contains four substituents as defined and preferably defined above.

Particularly preferred embodiments of the combinations of R⁵, R⁶, R⁷ and R⁸ according to the invention are in Table X1 below, wherein each line of lines X1-1 to X1-43 corresponds to one particular embodiment of the invention, wherein X1-1 to X1-43 are also in any combination a preferred embodiment of the present invention.

**Table X1:**

| **line** | **R⁵** | **R⁶** | **R⁷** | **R⁸** | | **line** | **R⁵** | **R⁶** | **R⁷** | **R⁸** |
|---|---|---|---|---|---|---|---|---|---|---|
| X1-1 | H | H | H | H | | X1-14 | CH₃ | H | CH(CH₃)₂ | H |
| X1-2 | CH₃ | H | H | H | | X1-15 | CH₃ | H | C(CH₃)₃ | H |
| X1-3 | CH₃ | CH₃ | H | H | | X1-16 | C(CH₃)₃ | H | H | H |
| X1-4 | CH₃ | H | CH₃ | H | | X1-17 | F | H | H | H |
| X1-5 | CH₃ | CH₃ | CH₃ | CH₃ | | X1-18 | F | F | H | H |
| X1-6 | CH₂CH₃ | H | H | H | | X1-19 | F | H | F | H |
| X1-7 | CH₂CH₃ | H | CH₂CH₃ | H | | X1-20 | F | H | Cl | H |
| X1-8 | CH₂CH₂CH₃ | H | H | H | | X1-21 | F | H | Br | H |
| X1-9 | CH₂CH₂CH₃ | H | CH₂CH₂CH₃ | H | | X1-22 | Cl | H | Br | H |
| X1-10 | CH(CH₃)2 | H | H | H | | X1-23 | Cl | H | H | H |
| X1-11 | CH(CH₃)2 | H | CH(CH₃)₂ | H | | X1-24 | Cl | Cl | H | H |
| X1-12 | CH₃ | H | CH₂CH₃ | H | | X1-25 | Cl | H | Cl | H |
| X1-13 | CH₃ | H | CH₂CH₂CH₃ | H | | X1-26 | Br | H | H | H |
| X1-27 | Br | Br | H | H | | X1-36 | CCl₃ | H | H | H |
| X1-28 | Br | H | Br | H | | X1-37 | CCl3 | H | CCl₃ | H |
| X1-29 | C₆H₅ | H | H | H | | X1-38 | CHF₂ | H | H | H |
| X1-30 | OCH₃ | H | H | H | | X1-39 | CH₂F | H | H | H |
| X1-31 | OCH₃ | H | OCH₃ | H | | X1-40 | CHCl₂ | H | H | H |
| X1-32 | OCH₂CH₃ | H | H | H | | X1-41 | CH₂Cl | H | H | H |
| X1-33 | OCH₂CH₃ | H | OCH₂CH₃ | H | | X1-42 | CH₂CH=CH₂ | H | H | H |
| X1-34 | CF₃ | H | H | H | | X1-43 | C≡CCH₃ | H | H | H |
| X1-35 | CF₃ | H | CF₃ | H | | | | | | |

In a further embodiment of the invention, X is -CR⁵R⁶-CR⁷R⁸-CR⁹R¹⁰-, wherein R⁵, R⁶, R⁷, R⁸, R⁹ and R¹⁰ are independently selected from the substituents and preferred substituents as defined below.

For every R⁵, R⁶, R⁷, R⁸, R⁹ and/or R¹⁰ that is present in -CR⁵R⁶-CR⁷R⁸-CR⁹R¹⁰-, the following embodiments and preferences apply independently of the meaning of any other R⁵, R⁶, R⁷, R⁸, R⁹ and/or R¹⁰ that may be present in the chain.
R⁵, R⁶, R⁷ and R⁸, are defined above.

According to one embodiment, R⁹ is hydrogen.
According to a further embodiment, R⁹ is halogen, in particular Cl or F.
According to a further embodiment, R⁹ is C₁-C₆-alkyl, in particular C₁-C₄-alkyl, wherein the alkyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆₋cycloalkyl, C₃-C₆-halocycloalkyl, C₁-C₄-alkoxy, C₁-C₄-haloalkoxy, phenyl and phenoxy. According to one embodiment thereof, R⁹ is C₁-C₆-alkyl, in particular C₁-C₄-alkyl, such as methyl, ethyl or n-propyl. According to a further embodiment thereof, R⁹ is C₁-C₆-haloalkyl, in particular C₁-C₄-haloalkyl, more particularly C₁-C₂-haloalkyl. According to a further embodiment thereof, R⁹ is C₁-C₆-hydroxyalkyl, in particular C₁-C₄-hydroxyalkyl, more particularly C₁-C₂-hydroxyalkyl. According to a further embodiment thereof, R⁹ is C₁-C₆-phenylalkyl, in particular C₁-C₄-phenylalkyl, more particularly C₁-C₂-phenylalkyl. According to a further embodiment thereof, R⁹ is C₁-C₆-phenoxyalkyl, in particular C₁-C₄-phenoxyalkyl, more particularly C₁-C₂-phenoxyalkyl.

According to a further embodiment, R⁹ is C₂-C₆-alkenyl, in particular C₂-C₄-alkenyl, wherein the alkenyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R⁹ is C₂-C₆-alkenyl, in particular C₂-C₄-alkenyl, such as C₂-alkenyl. According to a further embodiment thereof, R⁹ is C₂-C₆-haloalkenyl, in particular C₂-C₄-haloalkenyl, more particularly C₂-haloalkenyl. According to a further embodiment thereof, R⁹ is C₂-C₆-hydroxyalkenyl, in particular C₂-C₄-hydroxyalkenyl, more particularly C₂-hydroxyalkenyl. According to a further embodiment thereof, R⁹ is C₂-C₆-phenylalkenyl, in particular C₂-C₄-phenylalkenyl, more particularly C₂-phenylalkenyl. According to a further embodiment thereof, R⁹ is C₂-C₆-phenoxyalkenyl, in particular C₂-C₄-phenoxyalkenyl, more particularly C₂-phenoxyalkenyl.

According to a further embodiment, R⁹ is C₂-C₆-alkynyl, in particular C₂-C₄-alkynyl, wherein the alkynyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R⁹ is C₂-C₆-alkynyl, in particular C₂-C₄-alkynyl, such as C₂-alkynyl. According to a further embodiment thereof, R⁹ is C₂-C₆-haloalkynyl, in particular C₂-C₄-haloalkynyl, more particularly C₂-haloalkynyl. According to a further embodiment thereof, R⁹ is C₂-C₆-hydroxyalkynyl, in particular C₂-C₄-hydroxyalkynyl, more particularly C₂-hydroxyalkynyl. According to a further embodiment thereof, R⁹ is C₂-C₆-phenylalkynyl, in particular C₂-C₄-phenylalkynyl, more particularly C₂-phenylalkynyl. According to a further embodiment thereof, R⁹ is C₂-C₆-phenoxyalkynyl, in particular C₂-C₄-phenoxyalkynyl, more particularly C₂-phenoxyalkynyl.

According to a further embodiment, R⁹ is C₁-C₆-alkoxy, in particular C₁-C₄-alkoxy, more specifically C₁-C₂-alkoxy.

According to a further embodiment, R⁹ is phenyl that is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₂-haloalkyl, C₃-C₆-cycloalkyl, C₃-C₆-halocycloalkyl, C₁-C₂-alkoxy and C₁-C₂-haloalkoxy.

According to one embodiment, R¹⁰ is hydrogen.
According to a further embodiment, R¹⁰ is halogen, in particular Cl or F.
According to a further embodiment, R¹⁰ is C₁-C₆-alkyl, in particular C₁-C₄-alkyl, wherein the alkyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R¹⁰ is C₁-C₆-alkyl, in particular C₁-C₄-alkyl, such as methyl, ethyl or n-propyl. According to a further embodiment thereof, R¹⁰ is C₁-C₆-haloalkyl, in particular C₁-C₄-haloalkyl, more particularly C₁-C₂-haloalkyl. According to a further embodiment thereof, R¹⁰ is C₁-C₆-hydroxyalkyl, in particular C₁-C₄-hydroxyalkyl, more particularly C₁-C₂-hydroxyalkyl. According to a further embodiment thereof, R¹⁰ is C₁-C₆-phenylalkyl, in particular C₁-C₄-phenylalkyl, more particularly C₁-C₂-phenylalkyl. According to a further embodiment thereof, R¹⁰ is C₁-C₆-phenoxyalkyl, in particular C₁-C₄-phenoxyalkyl, more particularly C₁-C₂-phenoxyalkyl.

According to a further embodiment, R¹⁰ is C₂-C₆-alkenyl, in particular C₂-C₄-alkenyl, wherein the alkenyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R¹⁰ is C₂-C₆-alkenyl, in particular C₂-C₄-alkenyl, such as C₂-alkenyl. According to a further embodiment thereof, R¹⁰ is C₂-C₆-haloalkenyl, in particular C₂-C₄-haloalkenyl, more particularly C₂-haloalkenyl. According to a further embodiment thereof, R¹⁰ is C₂-C₆-hydroxyalkenyl, in particular C₂-C₄-hydroxyalkenyl, more particularly C₂-hydroxyalkenyl. According to a further embodiment thereof, R¹⁰ is C₂-C₆-phenylalkenyl, in particular C₂-C₄-phenylalkenyl, more particularly C₂-phenylalkenyl. According to a further embodiment thereof, R¹⁰ is C₂-C₆-phenoxyalkenyl, in particular C₂-C₄-phenoxyalkenyl, more particularly C₂-phenoxyalkenyl.

According to a further embodiment, R¹⁰ is C₂-C₆-alkynyl, in particular C₂-C₄-alkynyl, wherein the alkynyl is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₄-haloalkyl, C₃-C₆-cycloalkyl, C₃-C₆-halocycloalkyl, C₁-C₄-alkoxy, C₁-C₄-haloalkoxy, phenyl and phenoxy. According to one embodiment thereof, R¹⁰ is C₂-C₆-alkynyl, in particular C₂-C₄-alkynyl, such as C₂-alkynyl. According to a further embodiment thereof, R¹⁰ is C₂-C₆-haloalkynyl, in particular C₂-C₄-haloalkynyl, more particularly C₂-haloalkynyl. According to a further embodiment thereof, R¹⁰ is C₂-C₆-hydroxyalkynyl, in particular C₂-C₄-hydroxyalkynyl, more particularly C₂-hydroxyalkynyl. According to a further embodiment thereof, R¹⁰ is C₂-C₆-phenylalkynyl, in particular C₂-C₄-phenylalkynyl, more particularly C₂-phenylalkynyl. According to a further embodiment thereof, R¹⁰ is C₂-C₆-phenoxyalkynyl, in particular C₂-C₄-phenoxyalkynyl, more particularly C₂-phenoxyalkynyl.

According to a further embodiment, R¹⁰ is C₁-C₆-alkoxy, in particular C₁-C₄-alkoxy, more specifically C₁-C₂-alkoxy.

According to a further embodiment, R¹⁰ is phenyl that is unsubstituted or substituted by one, two, three or up to the maximum possible number of R^{x}; wherein R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy, in particular selected from Cl, F, CN, OH, C₁-C₄-alkyl, C₁-C₂-haloalkyl, C₃-C₆-cycloalkyl, C₃-C₆-halocycloalkyl, C₁-C₂-alkoxy and C₁-C₂-haloalkoxy.

According to one specific embodiment, R⁵, R⁶, R⁷, R⁸, R⁹ and R¹⁰ are independently selected from hydrogen, C₁-C₆-alkyl, C₁-C₆-haloalkyl, C₁-C₆-hydroxyalkyl and C₁-C₆-phenylalkyl as defined above. According to one specific embodiment thereof, R⁵ and R⁶ and R⁹ and R¹⁰ are hydrogen, in a particular embodiment, at the same time, both R⁷ and R⁸ are selected from methyl, ethyl, n-propyl, in particular methyl. According to a further specific embodiment thereof, R⁵, R⁷, R⁹ and R¹⁰ are hydrogen, in a particular embodiment, at the same time, both R⁶ and R⁸ are selected from methyl, ethyl, n-propyl, in particular methyl. According to a further specific embodiment thereof R⁵, R⁶, R⁷, R⁹ and R¹⁰ are hydrogen, and in a particular embodiment, at the same time, R⁸ selected from methyl, ethyl, n-propyl, in particular methyl. According to a further specific embodiment thereof R⁵, R⁶, R⁷, R⁸, R⁹ and R¹⁰ are hydrogen.

In one further embodiment, X is -CR⁵H-CH₂-CR⁹H-.
In still one further embodiment, X is -CH₂-CR⁷H-CH₂-.
In still one further embodiment, X is -CH₂-CR⁷R⁸-CH₂-.

According to one embodiment, -CR⁵R⁶-CR⁷R⁸-CR⁹R¹⁰- contains at least one substituent, in particular exactly one substituent, as defined and preferably defined above.

According to a further embodiment, -CR⁵R⁶-CR⁷R⁸-CR⁹R¹⁰- contains at least two substituents, in particular exactly two substituents, as defined and preferably defined above.

According to a further embodiment, -CR⁵R⁶-CR⁷R⁸-CR⁹R¹⁰- contains at least three substituents, in particular exactly three substituents, as defined and preferably defined above.

According to still a further embodiment, -CR⁵R⁶-CR⁷R⁸-CR⁹R¹⁰- contains at least four substituents, in particular exactly four substituents, as defined and preferably defined above.

According to still a further embodiment, -CR⁵R⁶-CR⁷R⁸-CR⁹R¹⁰- contains at least five substituents, in particular exactly five substituents, as defined and preferably defined above.

According to still a further embodiment, -CR⁵R⁶-CR⁷R⁸-CR⁹R¹⁰- contains six substituents, as defined and preferably defined above.

Particularly preferred embodiments of the combinations of R⁵, R⁶, R⁷, R⁸, R⁹ and R¹⁰ according to the invention are in Table X2 below, wherein each line of lines X2-1 to X2-78 corresponds to one particular embodiment of the invention, wherein X2-1 to X2-78 are also in any combination a preferred embodiment of the present invention.

**Table X2:**

| **line** | **R⁵** | **R⁶** | **R⁷** | **R⁸** | **R⁹** | **R¹⁰** |
|---|---|---|---|---|---|---|
| X2-1 | H | H | H | H | H | H |
| X2-2 | H | H | CH₃ | H | H | H |
| X2-3 | H | H | CH₃ | CH₃ | H | H |
| X2-4 | CH₃ | H | H | H | H | H |
| X2-5 | CH₃ | H | CH₃ | H | H | H |
| X2-6 | CH₃ | CH₃ | H | H | H | H |
| X2-7 | CH₃ | H | H | H | CH₃ | H |
| X2-8 | CH₂CH₃ | H | H | H | H | H |
| X2-9 | H | H | CH₂CH₃ | H | H | H |
| X2-10 | CH₂CH₃ | H | H | H | CH₂CH₃ | H |
| X2-11 | CH₂CH₂CH₃ | H | H | H | H | H |
| X2-12 | H | H | CH₂CH₂CH₃ | H | H | H |
| X2-13 | CH₂CH₂CH₃ | H | H | H | CH₂CH₂CH₃ | H |
| X2-14 | CH(CH₃)₂ | H | H | H | H | H |
| X2-15 | H | H | CH(CH₃)₂ | H | H | H |
| X2-16 | CH(CH₃)₂ | H | H | H | CH(CH₃)₂ | H |
| X2-17 | C(CH₃)₃ | H | H | H | H | H |
| X2-18 | H | H | C(CH₃)₃ | H | H | H |
| X2-19 | C(CH₃)₃ | H | H | H | C(CH₃)₃ | H |
| X2-20 | CH₃ | H | CH₂CH₃ | H | H | H |
| X2-21 | CH₃ | H | H | H | CH₂CH₃ | H |
| X2-22 | CH₃ | H | CH₂CH₃ | H | H | H |
| X2-23 | CH₃ | H | H | H | CH₂CH₃ | H |
| X2-24 | CH₃ | H | CH(CH₃)₂ | H | H | H |
| X2-25 | CH₃ | H | H | H | CH(CH₃)₂ | H |
| X2-26 | CH₃ | H | C(CH₃)₃ | H | H | H |
| X2-27 | CH₃ | H | H | H | C(CH₃)₃ | H |
| X2-28 | C₆H₅ | H | H | H | H | H |
| X2-29 | H | H | C₆H₅ | H | H | H |
| X2-30 | OCH₃ | H | H | H | H | H |
| X2-31 | H | H | OCH₃ | H | H | H |
| X2-32 | OCH₂CH₃ | H | H | H | H | H |
| X2-33 | H | H | OCH₂CH₃ | H | H | H |
| X2-34 | H | H | F | H | H | H |
| X2-35 | H | H | F | F | H | H |
| X2-36 | F | H | H | H | H | H |
| X2-37 | F | H | F | H | H | H |
| X2-38 | F | F | H | H | H | H |
| X2-39 | F | H | H | H | F | H |
| X2-40 | H | H | Cl | H | H | H |
| X2-41 | H | H | Cl | Cl | H | H |
| X2-42 | Cl | H | H | H | H | H |
| X2-43 | Cl | H | Cl | H | H | H |
| X2-44 | Cl | Cl | H | H | H | H |
| X2-45 | Cl | H | H | H | Cl | H |
| X2-46 | H | H | Br | H | H | H |
| X2-47 | H | H | Br | Br | H | H |
| X2-48 | Br | H | H | H | H | H |
| X2-49 | Br | H | Br | H | H | H |
| X2-50 | Br | Br | H | H | H | H |
| X2-51 | Br | H | H | H | Br | H |
| X2-52 | H | H | F | Cl | H | H |
| X2-53 | H | H | F | Br | H | H |
| X2-54 | F | H | Cl | H | H | H |
| X2-55 | F | H | Br | H | H | H |
| X2-56 | Cl | H | F | H | H | H |
| X2-57 | Br | H | F | H | H | H |
| X2-58 | Cl | H | Br | H | H | H |
| X2-59 | Br | H | Cl | H | H | H |
| X2-60 | F | H | H | H | Cl | H |
| X2-61 | F | H | H | H | Br | H |
| X2-62 | Cl | H | H | H | Br | H |
| X2-63 | CF₃ | H | H | H | H | H |
| X2-64 | H | H | CF₃ | H | H | H |
| X2-65 | CCl₃ | H | H | H | H | H |
| X2-66 | H | H | CCl₃ | H | H | H |
| X2-67 | CHF₂ | H | H | H | H | H |
| X2-68 | H | H | CHF₂ | H | H | H |
| X2-69 | CH₂F | H | H | H | H | H |
| X2-70 | H | H | CH₂F | H | H | H |
| X2-71 | CHCl₂ | H | H | H | H | H |
| X2-72 | H | H | CHCl₂ | H | H | H |
| X2-73 | CH₂Cl | H | H | H | H | H |
| X2-74 | H | H | CH₂Cl | H | H | H |
| X2-75 | CH₂CH=CH₂ | H | H | H | H | H |
| X2-76 | H | H | CH₂CH=CH₂ | H | H | H |
| X2-77 | C≡CCH₃ | H | H | H | H | H |
| X2-78 | H | H | C≡CCH₃ | H | H | H |

Preferred embodiments for X are listed in Table Q, wherein each line of lines Q-1 to Q-38 corresponds to one particular embodiment for X of the invention, wherein Q-1 to Q-38 are also in any combination a preferred embodiment of the present invention.

**Table Q:**

| **line** | **X** | | | **line** | **X** | |
|---|---|---|---|---|---|---|
| Q-1 | | -CH₂-CH₂- | | Q-20 | | -CH₂CH₂CH₂- |
| Q-2 | | -CH(CH₃)-CH₂- | | Q-21 | | -CH₂CH(CH₃)CH₂- |
| Q-3 | | -C(CH₃)₂-CH₂- | | Q-22 | | -CH₂C(CH₃)₂CH₂- |
| Q-4 | | -CH(CH₃)-CH(CH₃)- | | Q-23 | | -CH(CH₃)CH₂CH₂- |
| Q-5 | | -C(CH₃)₂-C(CH₃)₂- | | Q-24 | | -CH(CH₃)CH(CH₃)CH₂- |
| Q-6 | | -CH(C₂H₅)-CH₂- | | Q-25 | -C(CH₃)₂CH₂CH₂- | |
| Q-7 | | -CH(C₂H₅)-CH(C₂H₅)- | | Q-26 | | -CH(CH₃)CH₂CH(CH₃)- |
| Q-8 | -CH(n-C₃H₇)-CH₂- | | | Q-27 | | -CH(C₂H₅)CH₂CH₂- |
| Q-9 | -CH(n-C₃H₇)-CH(n-C₃H₇)- | | | Q-28 | -CH₂CH(C₂H₅)CH₂- | |
| Q-10 | | -CH(i-C₃H₇)-CH₂- | | Q-29 | | -CH(C₂H₅)CH₂CH(C₂H₅)- |
| Q-11 | | -CH(i-C₃H₇)-CH(i-C₃H₇)- | | Q-30 | -CH(CH₃)CH(C₂H₅)CH₂- | |
| Q-12 | | -CH(CH₃)-CH(C₂H₅)- | | Q-31 | | -CH(CH₃)CH₂CH(C₂H₅)- |
| Q-13 | | -CH(CH₃)-CH(n-C₃H₇)- | | Q-32 | | -CH₂CH(n-C₃H₇)CH₂- |
| Q-14 | | -CHF-CH₂- | | Q-33 | | -CH₂CH(C₆H₅)CH₂- |
| Q-15 | -CHF-CHF- | | | Q-34 | | -CH₂CHFCH₂- |
| Q-16 | -CHCl-CH₂- | | | Q-35 | | -CH₂CH(CF₃)CH₂- |
| Q-17 | | -CH(C₆H₅)-CH₂- | | Q-36 | | -CH₂CHClCH₂- |
| Q-18 | | -CH(CF₃)-CH₂- | | Q-37 | | -CHClCH₂CHCl- |
| Q-19 | | -CH(CH₃)-CH(i-C₃H₇)- | | Q-38 | | -CHClCH₂CH₂- |

According to the invention, Z-Y is bound to the phenyl via Y, and Y is a direct bond or a divalent group selected from the group consisting of -O-, -S-, -So-, -SO₂-, -NH-, -N(C₁-C₄-alkyl)-, - CR¹²R¹³-, -CR¹²R¹³-CR¹⁴R¹⁵-, -CR¹⁶=CR¹⁷-and -C≡C-; wherein R¹²,R¹³,R¹⁴,R¹⁵ R¹⁶ and R¹⁷ are independently selected from hydrogen, halogen, CN, nitro, OH, C₁-C₄-alkyl, C₁-C₄-halogenalkyl, C₁-C₄-alkoxy and C₁-C₄-halogenalkoxy.

According to an embodiment, Y is selected from a direct bond, -O-, -CR¹²R¹³-, -CR¹²R¹³-CR¹⁴R¹⁵-, -CR¹⁶=CR¹⁷-and -C≡C-; wherein R¹²,R¹³,R¹⁴,R¹⁵,R¹⁶ and R¹⁷ are independently selected from hydrogen, halogen, CN, nitro, OH, C₁-C₄-alkyl, C₁-C₄-halogenalkyl, C₁-C₄-alkoxy and C₁-C₄-halogenalkoxy.

According to one embodiment, Z-Y is attached to the ortho-position (2-position).
According to a further embodiment, Z-Y is attached to the meta-position (3-position). According to one embodiment, Z-Y is attached to the para-position (4-position).
According to one embodiment, Y is a direct bond. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

According to a further embodiment, Y is -O-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

According to still a further embodiment, Y is -S-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

According to still a further embodiment, Y is -SO-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

According to still a further embodiment, Y is -SO₂-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

According to still a further embodiment, Y is -NH-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

According to still a further embodiment, Y is -N(C₁-C₄-alkyl)-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

According to still a further embodiment, Y is -CR¹²R¹³-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

R¹² and R¹³ are independently selected from hydrogen, halogen, CN, nitro, OH, C₁-C₄-alkyl, C₁-C₄-halogenalkyl, C₁-C₄-alkoxy and C₁-C₄-halogenalkoxy.

In one preferred embodiment R¹² and R¹³ are independently selected from hydrogen and halogen, in particular hydrogen, fluorine and chlorine. In a further preferred embodiment R¹² and R¹³ are independently selected from hydrogen and C₁-C₄-alkyl, in particular hydrogen, methyl and ethyl. In a preferred embodiment, R¹² and R¹³are independently selected from hydrogen and C₁-C₄-alkoxy, in particular hydrogen, methoxy and ethoxy. In another preferred embodiment, R¹² and R¹³ are independently selected from hydrogen and CN. In yet another preferred embodiment R¹² and R¹³ are independently selected from hydrogen and OH.

According to still a further embodiment, Y is -CR¹²R¹³-CR¹⁴R¹⁵-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

R¹² R¹³ R¹⁴ and R¹⁵ are independently selected from hydrogen, halogen, CN, nitro, OH, C₁-C₄-alkyl, C₁-C₄-halogenalkyl, C₁-C₄-alkoxy and C₁-C₄-halogenalkoxy.

In one preferred embodiment R¹²,R¹³,R¹⁴ and R¹⁵are independently selected from hydrogen and halogen, in particular hydrogen, fluorine and chlorine. In a further preferred embodiment R¹²,R¹³,R¹⁴ and R¹⁵ are independently selected from hydrogen and C₁-C₄-alkyl, in particular hydrogen, methyl and ethyl. In a preferred embodiment, R¹²,R¹³,R¹⁴ and R¹⁵ are independently selected from hydrogen and C₁-C₄-alkoxy, in particular hydrogen, methoxy and ethoxy. In another preferred embodiment, R¹²,R¹³,R¹⁴ and R¹⁵ are independently selected from hydrogen and CN. In yet another preferred embodiment R¹²,R¹³,R¹⁴ and R¹⁵ are independently selected from hydrogen and OH.

According to still a further embodiment, Y is -CR¹⁶=CR¹⁷-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position). R¹⁶ and R¹⁷ are independently selected from hydrogen, halogen, CN, nitro, OH, C₁-C₄-alkyl, C₁-C₄-halogenalkyl, C₁-C₄-alkoxy and C₁-C₄-halogenalkoxy.

In one preferred embodiment R¹⁶ and R¹⁷ are independently selected from hydrogen and halogen, in particular hydrogen, fluorine and chlorine. In a further preferred embodiment R¹⁶ and R¹⁷ are independently selected from hydrogen and C₁-C₄-alkyl, in particular hydrogen, methyl and ethyl. In a preferred embodiment, R¹⁶ and R¹⁷ are independently selected from hydrogen and C₁-C₄-alkoxy, in particular hydrogen, methoxy and ethoxy. In another preferred embodiment, R¹⁶ and R¹⁷ are independently selected from hydrogen and CN. In yet another preferred embodiment R¹⁶ and R¹⁷ are independently selected from hydrogen and OH. According to still a further embodiment, Y is -C≡C-. In a specific embodiment thereof, Z-Y is attached to the ortho-position (2-position). In a further specific embodiment thereof, Z-Y is attached to the meta-position (3-position). In a further specific embodiment thereof, Z-Y is attached to the para-position (4-position).

In general, R¹²,R¹³,R¹⁴,R¹⁵,R¹⁶,R¹⁷ are independently selected from hydrogen, halogen, CN, nitro, OH, C₁-C₄-alkyl, C₁-C₄-halogenalkyl, C₁-C₄-alkoxy and C₁-C₄-halogenalkoxy. In one preferred embodiment of the invention R¹²,R¹³,R¹⁴,R¹⁵,R¹⁶ and R¹⁷ are independently selected from hydrogen and halogen, in particular hydrogen, fluorine and chlorine. In a further preferred embodiment R¹²,R¹³,R¹⁴,R¹⁵,R¹⁶ and R¹⁷ are independently selected from hydrogen and C₁-C₄-alkyl, in particular hydrogen, methyl and ethyl. In a preferred embodiment, R¹²,R¹³,R¹⁴,R¹⁵,R¹⁶ and R¹⁷ are independently selected from hydrogen and C₁-C₄-alkoxy, in particular hydrogen, methoxy and ethoxy. In another preferred embodiment, R¹²,R¹³,R¹⁴,R¹⁵,R¹⁶ and R¹⁷ are independently selected from hydrogen and CN. In yet another preferred embodiment R¹²,R¹³,R¹⁴,R¹⁵,R¹⁶ and R¹⁷ are independently selected from hydrogen and OH.

According to the invention, Z is five or six-membered heteroaryl, wherein the heteroaryl contains 1, 2, 3 or 4 heteroatoms selected from the group consisting of O, N and S, and wherein the heteroaryl is unsubstituted or substituted by 1, 2 or 3 R^{L}, or phenyl that is unsubstituted (m=0) or substituted by (R^{L})ₘ.

According to one embodiment, Z is phenyl that is unsubstituted (m=0) or substituted by (R^{L})ₘ. According to the invention, there can be zero, one, two, three, four or five R^{L} present, namely for m is 0, 1, 2, 3, 4 or 5. In particular, m is 0, 1, 2, 3 or 4.

According to one embodiment, m is 0.
According to a further embodiment, m is 1, 2, 3 or 4, in particular 1, 2 or 3, more specifically 1 or 2. According to one specific embodiment thereof, m is 1, according to a further specific embodiment, m is 2.
According to still a further embodiment, m is 2, 3 or 4.
According to still a further embodiment, m is 3.
According to one embodiment of the invention, one R^{L} is attached to the para-position (4-position).
According to a further embodiment of the invention, one R^{L} is attached to the meta-position (3-position).
According to a further embodiment of the invention, one R^{L} is attached to the ortho-position (2-position).

According to a further embodiment of the invention, two R^{L} are attached in 2,4-position.
According to a further embodiment of the invention, two R^{L} are attached in 2,3-position.
According to a further embodiment of the invention, two R^{L} are attached in 2,5-position.
According to a further embodiment of the invention, two R^{L} are attached in 2,6-position.
According to a further embodiment of the invention, two R^{L} are attached in 3,4-position.
According to a further embodiment of the invention, two R^{L} are attached in 3,5-position.
According to a further embodiment of the invention, three R^{L} are attached in 2,4,6-position.

For every R^{L} that is present in the inventive compounds, the following embodiments and preferences apply independently of the meaning of any other R^{L} that may be present in the phenyl ring. Furthermore, the particular embodiments and preferences given herein for R^{L} apply independently for each of m=1, m=2, m=3, m= 4 and m=5.

Each R^{L} is independently selected from halogen, CN, NO₂, OH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyl-C₁-C₄-alkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl and phenyl-C₁-C₄-alkyl, wherein the aliphatic, alicyclic and aromatic moieties of R^{L} are unsubstituted or substituted by one, two, three or four or up to the maximum possible number of R^{La}; wherein R^{La} is independently selected from halogen, CN, NO₂, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio and C₁-C₆-haloalkylthio.

According to one embodiment, R^{L} is independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)p(C₁-C₄-alkyl) (p=0, 1 or 2), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂); wherein each of R^{L} is unsubstituted or further substituted by one, two, three or four independently selected R^{La}, wherein R^{La} is as defined and preferably defined herein.

According to a further embodiment, R^{L} is independently selected from halogen, CN, NO₂, C₁-C₄-alkyl, C₁-C₄-alkoxy, C₂-C₄-alkenyl, C₂-C₄-alkynyl, C₃-C₆-cycloalkyl, C₃-C₆-cycloalkyloxy, NH₂, NH(C₁-C₄₂-alkyl), N(C₁-C₂-alkyl)₂, S(C₁-C₂-alkyl), S(O)(C₁-C₂-alkyl), S(O)₂(C₁-C₂-alkyl), C(=O)(C₁-C₂-alkyl), C(=O)(OH) and C(=O)(O-C₁-C₂-alkyl), wherein each of R^{L} is unsubstituted or further substituted by one, two, three or four independently selected R^{La}, wherein R^{La} is as defined and preferably defined herein.

According to a further embodiment, R^{L} is independently selected from halogen, CN, NO₂, C₁-C₄-alkyl, C₁-C₄-haloalkyl, C₁-C₄-alkoxy, C₁-C₄-haloalkoxy, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, S(C₁-C₂-alkyl), S(O)(C₁-C₂-alkyl), S(O)₂(C₁-C₂-alkyl), C(=O)(C₁-C₂-alkyl), C(=O)(OH) and C(=O)(O-C₁-C₂-alkyl).

According to a further embodiment, R^{L} is independently selected from halogen, CN, NO₂, C₁-C₂-alkyl, C₁-C₂-haloalkyl, C₁-C₂-alkoxy, C₁-C₂-haloalkoxy, S(C₁-C₂-alkyl), S(O)(C₁-C₂-alkyl), S(O)₂(C₁-C₂-alkyl), C(=O)(OH) and C(=O)(O-C₁-C₂-alkyl).

According to a further embodiment, R^{L} is independently selected from F, Cl, Br, CN, C₁-C₄-alkyl, C₁-C₄-haloalkyl, C₁-C₄-alkoxy, C₁-C₄-haloalkoxy, S(C₁-C₄-alkyl), S(O)(C₁-C₄-alkyl) and S(0)₂(Cₗ-C₄-alkyl).

According to still a further specific embodiment, R^{L} is independently selected from halogen, in particular from Br, F and Cl, more specifically from F and Cl.
According to a further specific embodiment, R^{L} is CN.
According to one further embodiment R^{L} is NO₂.
According to one further embodiment R^{L} is OH.
According to one further embodiment R^{L} is SH.

According to a further specific embodiment, R^{L} is C₁-C₆-alkyl, in particular C₁-C₄-alkyl, such as CH₃. Further appropriate alkyls are ethyl, n-propyl, i-propyl, n-butyl, i-butyl and t-butyl.

According to a further specific embodiment, R^{L} is C₁-C₆-haloalkyl, in particular C₁-C₄-haloalkyl, such as CF₃, CHF₂, CH₂F, CCHl₃, CHCl₂ or CH₂Cl.

According to a further specific embodiment R^{L} is C₁-C₆-alkyl, preferably C₁-C₄-alkyl, substituted by OH, more preferably CH₂OH, CH₂CH₂OH, CH₂CH₂CH₂OH, CH(CH₃)CH₂0H, CH₂CH(CH₃)OH, CH₂CH₂CH₂CH₂OH. In a special embodiment R^{L} is CH₂OH. According to a further specific embodiment R^{L} is C₁-C₆-alkyl, preferably C₁-C₄-alkyl substituted by CN, more preferably CH₂CN, CH₂CH₂CN, CH₂CH₂CH₂CN, CH(CH₃)CH₂CN, CH₂CH(CH₃)CN, CH₂CH-₂CH₂CH₂CN. In a special embodiment R^{L} is CH₂CH₂CN. In a further special embodiment R⁴ is CH(CH₃)CN. According to a further specific embodiment R^{L} is C₁-C₄-alkoxy-C₁-C₆-alkyl, more preferably C₁-C₄-alkoxy-C₁-C₄-alkyl. In a special embodiment R^{L} is CH₂OCH₃. In a further special embodiment R^{L} is CH₂CH₂OCH₃. In a further special embodiment R^{L} is CH(CH₃)OCH₃. In a further special embodiment R^{L} is CH(CH₃)OCH₂CH₃. In a further special embodiment R^{L} is CH₂CH₂OCH₂CH₃. According to a further specific embodiment R^{L} is C₁-C₄-haloalkoxy-Cᵢ-C₆-alkyl, more preferably C₁-C₄-alkoxy-C₁-C₄-alkyl. In a special embodiment R^{L} is CH₂OCF₃. In a further special embodiment R^{L} is CH₂CH₂OCF₃. In a further special embodiment R^{L} is CH₂OCCl₃. In a further special embodiment R^{L} is CH₂CH₂OCCl₃.

According to a further specific embodiment, R^{L} is C₁-C₆-alkoxy, in particular C₁-C₄-alkoxy, more specifically C₁-C₂-alkoxy such as OCH₃ or OCH₂CH₃.

According to a further specific embodiment, R^{L} is C₁-C₆-haloalkoxy, in particular C₁-C₄-haloalkoxy, more specifically C₁-C₂-haloalkoxy such as OCF₃, OCHF₂, OCH₂F, OCCl₃, OCHCl₂ or OCH₂Cl, in particular OCF₃, OCHF₂, OCCl₃ or OCHCl₂.

According to still a further embodiment, R^{L} is C₂-C₆-alkenyl or C₂-C₆-haloalkenyl, in particular C₂-C₄-alkenyl or C₂-C₄-haloalkenyl, such as CH=CH₂, CH₂CH=CH₂, CH=CHCH₃ or C(CH₃)=CH₂.

According to a further specific embodiment R^{L} is C₂-C₆-alkenyl, preferably C₂-C₄-alkenyl, substituted by OH, more preferably, CH=CHOH, CH=CHCH₂OH, C(CH₃)=CHOH, CH=C(CH₃)OH. In a special embodiment R^{L} is CH=CHOH. In a further special embodiment R^{L} is CH=CHCH₂OH. According to a further specific embodiment R^{L} is C₁-C₄-alkoxy-C₂-C₆-alkenyl, more preferably C₁-C₄-alkoxy-C₂-C₄-alkenyl. In a special embodiment R^{L} is CH=CHOCH₃. In a further special embodiment R^{L} is CH=CHCH₂OCH₃. According to a further specific embodiment R^{L} is C₁-C₄-haloalkoxy-C₂-C₆-alkenyl, more preferably C₁-C₄-haloalkoxy-C₂-C₄-alkenyl. In a special embodiment R^{L} is CH=CHOCF₃. In a further special embodiment R^{L} is CH=CHCH₂OCF₃. In a further special embodiment R^{L} is CH=CHOCCl₃. In a further special embodiment R^{L} is CH=CHCH₂OCCl₃. According to a further specific embodiment R^{L} is C₃-C₈-cycloalkyl-C₂-C₆-alkenyl, preferably C₃-C₆-cycloalkyl-C₂-C₄-alkenyl. According to a further specific embodiment R^{L} is C₃-C₆-halocycloalkyl-C₂-C₄-alkenyl, preferably C₃-C₈-halocycloalkyl-C₂-C₆-alkenyl.

According to still a further embodiment, R^{L} is C₂-C₆-alkynyl or C₂-C₆-haloalkynyl, in particular C₂-C₄-alkynyl or C₂-C₄-haloalkynyl, such as C≡CH, CH₂CCH or CH₂CCCH₃.

According to a further specific embodiment R^{L} is C₂-C₆-alkynyl, preferably C₂-C₄-alkynyl, substituted by OH, more preferably, CCOH, CH₂CCOH. In a special embodiment R^{L} is CCOH. In a further special embodiment R^{L} is CH₂CCOH. According to a further specific embodiment R^{L} is C₁-C₄-alkoxy-C₂-C₆-alkynyl, more preferably C₁-C₄-alkoxy-C₂-C₄-alkynyl. In a special embodiment R^{L} is CCOCH₃. In a further special embodiment R^{L} is CH₂CCOCH₃. According to a further specific embodiment R^{L} is C₁-C₄-haloalkoxy-C₂-C₆-alkynyl, more preferably C₁-C₄-haloalkoxy-C₂-C₄-alkynyl. In a special embodiment R^{L} is CCOCF₃. In a further special embodiment R^{L} is CH₂CCOCF₃. In a further special embodiment R^{L} is CCOCCl₃. In a further special embodiment

R^{L} is CH₂CCOCCl₃. According to a further specific embodiment R^{L} is C₃-C₈-cycloalkyl-C₂-C₆-alkynyl, preferably C₃-C₆-cycloalkyl-C₂-C₄-alkynyl. According to a further specific embodiment R^{L} is C₃-C₆-halocycloalkyl-C₂-C₄-alkynyl, preferably C₃-C₈-halocycloalkyl-C₂-C₆-alkynyl.

According to one another embodiment R^{L} is C₃-C₈-cycloalkyl, preferably cyclopropyl, cyclobutyl, cyclopentyl or cyclohexyl, in particular cyclopropyl or cyclobutyl. In a special embodiment R^{L} is cyclopropyl. In a further special embodiment R^{L} is cyclobutyl. In a further special embodiment R⁴ is cyclopentyl. In a further special embodiment R^{L} is cyclohexyl.

According to one another embodiment R^{L} is C₃-C₈-cycloalkoxy, preferably C₃-C₆-cycloalkoxy. In a special embodiment R^{L} is O-cyclopropyl.

According to a specific embodiment R^{L} is C₃-C₈-halocycloalkyl, more preferably fully or partially halogenated C₃-C₆-cycloalkyl. In a special embodiment R^{L} is fully or partially halogenated cyclopropyl. In a further special embodiment R^{L} is 1-Cl-cyclopropyl. In a further special embodiment R^{L} is 2-Cl-cyclopropyl. In a further special embodiment R^{L} is 1-F-cyclopropyl. In a further special embodiment R^{L} is 2-F-cyclopropyl. In a further special embodiment R^{L} is fully or partially halogenated cyclobutyl. In a further special embodiment R^{L} is 1-Cl-cyclobutyl. In a further special embodiment R^{L} is 1-F-cyclobutyl. In a further special embodiment R^{L} is 3,3-Cl₂-cyclobutyl. In a further special embodiment R^{L} is 3,3-F₂-cyclobutyl. According to a specific embodiment R^{L} is C₃-C₈-cycloalkyl substituted by C₁-C₄-alkyl, more preferably is C₃-C₆-cycloalkyl substituted by C₁-C₄-alkyl. In a special embodiment R^{L} is 1-CH₃-cyclopropyl. According to a specific embodiment R^{L} is C₃-C₈-cycloalkyl substituted by CN, more preferably is C₃-C₆-cycloalkyl substituted by CN. In a special embodiment R^{L} is 1-CN-cyclopropyl.According to a further specific embodiment R^{L} is C₃-C₈-cycloalkyl-C₃-C₈-cycloalkyl, preferably C₃-C₆-cycloalkyl-C₃-C₆-cycloalkyl. In a special embodiment R^{L} is cyclopropyl-cyclopropyl. In a special embodiment R^{L} is 2-cyclopropyl-cyclopropyl. According to a further specific embodiment R^{L} is C₃-C₈-cycloalkyl-C₃-C₈-halocycloalkyl, preferably C₃-C₆-cydoalkyl-C₃-C₆-halocycloalkyl.

According to one another embodiment R^{L} is C₃-C₈-cycloalkyl-C₁-C₄-alkyl, preferably C₃-C₆-cycloalkyl-C₁-C₄-alkyl. In a special embodiment R^{L} is CH(CH₃)(cyclopropyl). In a further special embodiment R^{L} is CH₂-(cyclopropyl).

According to a further preferred embodiment R^{L} is C₃-C₈-cycloalkyl-C₁-C₄-alkyl wherein the alkyl moiety can be substituted by one, two, three or up to the maximum possible number of identical or different groups R^{a} as defined and preferably herein and the cycloalkyl moiety can be substituted by one, two, three or up to the maximum possible number of identical or different groups R^{b} as defined and preferably herein.

According to a specific embodiment R^{L} is C₃-C₈-cycloalkyl-C₁-C₄-haloalkyl, C₃-C₆-cycloalkyl-C₁-C₄-haloalkyl. According to a specific embodiment R^{L} is C₃-C₈-halocycloalkyl-C₁-C₄-alkyl, C₃-C₆-halocycloalkyl-C₁-C₄-alkyl. In a special embodiment R^{L} is fully or partially halogenated cyclopropyl-C₁-C₄-alkyl. In a further special embodiment R^{L} is 1-Cl-cyclopropyl-C₁-C₄-alkyl. In a further special embodiment R^{L} is 1-F-cyclopropyl-C₁-C₄-alkyl.

According to one another embodiment R^{L} is NH₂.
According to one another embodiment R^{L} is NH(C₁-C₄-alkyl). According to a specific embodiment R^{L} is NH(CH₃). According to a specific embodiment R^{L} is NH(CH₂CH₃). According to a specific embodiment R^{L} is NH(CH₂CH₂CH₃). According to a specific embodiment R^{L} is NH(CH(CH₃)₂). According to a specific embodiment R^{L} is NH(CH₂CH₂CH₂CH₃). According to a specific embodiment R^{L} is NH(C(CH₃)₃).

According to one another embodiment R^{L} is N(C₁-C₄-alkyl)₂. According to a specific embodiment R^{L} is N(CH₃)₂. According to a specific embodiment R^{L} is N(CH₂CH₃)₂. According to a specific embodiment R^{L} is N(CH₂CH₂CH₃)₂. According to a specific embodiment R^{L} is N(CH(CH₃)₂)₂. According to a specific embodiment R^{L} is N(CH₂CH₂CH₂CH₃)₂. According to a specific embodiment R^{L} is NH(C(CH₃)₃)₂.

According to one another embodiment R^{L} is NH(C₃-C₈-cycloalkyl) preferably NH(C₃-C₆-cycloalkyl). According to a specific embodiment R^{L} is NH(cyclopropyl). According to a specific embodiment R^{L} is NH(cyclobutyl). According to a specific embodiment R^{L} is NH(cyclopentyl). According to a specific embodiment R^{L} is NH(cyclohexyl).

According to one another embodiment R^{L} is N(C₃-C₈-cycloalkyl)₂ preferably N(C₃-C₆-cycloalkyl)₂. According to a specific embodiment R^{L} is N(cyclopropyl)₂. According to a specific embodiment R^{L} is N(cyclobutyl)₂. According to a specific embodiment R^{L} is N(cyclopentyl)₂. According to a specific embodiment R^{L} is N(cyclohexyl)₂.

According to still a further embodiment, R^{L} is selected from C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)(N(C₃-C₆-cycloalkyl)₂), in particular selected from C(=O)(C₁-C₂-alkyl), C(=O)(OH), C(=O)(O-C₁-C₂-alkyl), C(=O)(NH(C₁-C₂-alkyl)), C(=O)(N(C₁-C₂-alkyl)₂), C(=O)(NH(C₃-C₆-cycloalkyl)) and C(=O)(N(C₃-C₆-cycloalkyl)₂). According to one specific embodiment thereof, R^{L} is C(=O)(OH) or C(=O)(O-C₁-C₄-alkyl), in particular C(=O)(OCH₃).

According to one another embodiment R^{L} is C(=O)(-C₁-C₄-alkyl). According to a specific embodiment R^{L} is C(=O)CH₃. According to a further specific embodiment R^{L} is C(=O)CH₂CH₃. According to a further specific embodiment R^{L} is C(=O)CH₂CH₂CH₃. According to a further specific embodiment R^{L} is C(=O)CH(CH₃)₂. According to a further specific embodiment R^{L} is C(=O)C(CH₃)₃. According to one another embodiment R^{L} is C(=O)OH.

According to one another embodiment R^{L} is C(=O)(-O-C₁-C₄-alkyl). According to a specific embodiment R^{L} is C(=O)OCH₃. According to a further specific embodiment R^{L} is C(=O)OCH₂CH₃. According to a further specific embodiment R^{L} is C(=O)OCH₂CH₂CH₃. According to a further specific embodiment R^{L} is C(=O)OCH(CH₃)₂. According to a further specific embodiment R^{L} is C(=O)OC(CH₃)₃.

According to one another embodiment R^{L} is C(=O)-NH(C₁-C₄-alkyl). According to a specific embodiment R^{L} is C(=O)NHCH₃. According to a further specific embodiment R^{L} is C(=O)NHCH₂CH₃. According to a further specific embodiment R^{L} is C(=O)NHCH₂CH₂CH₃. According to a further specific embodiment R^{L} is C(=O)NHCH(CH₃)₂. According to a further specific embodiment R^{L} is C(=O)NHC(CH₃)₃.

According to one another embodiment R^{L} is C(=O)-N(C₁-C₄-alkyl)₂. According to a specific embodiment R^{L} is C(=O)N(CH₃)₂. According to a further specific embodiment R^{L} is C(=O)N(CH₂CH₃)₂. According to a further specific embodiment R^{L} is C(=O)N(CH₂CH₂CH₃)₂. According to a further specific embodiment R^{L} is C(=O)N(CH(CH₃)₂)₂. According to a further specific embodiment R^{L} is C(=O)N(C(CH₃)₃)₂.

According to one another embodiment R^{L} is C(=O)-NH(C₃-C₆-cycloalkyl). According to a specific embodiment R^{L} is C(=O)NH(cyclopropyl). According to a further specific embodiment R^{L} is C(=O)NH(cyclobutyl) According to a further specific embodiment R^{L} is C(=O)NH(cyclopentyl).According to a further specific embodiment R^{L} is C(=O)NH(cyclohexyl).

According to one another embodiment R^{L} is C(=O)-N(C₃-C₆-cycloalkyl)₂. According to a specific embodiment R^{L} is C(=O)N(cyclopropyl)₂. According to a further specific embodiment R^{L} is C(=O)N(cyclobutyl)₂. According to a further specific embodiment R^{L} is C(=O)N(cyclopentyl)₂. According to a further specific embodiment R^{L} is C(=O)N(cyclohexyl)₂.

According to still a further embodiment, R^{L} is selected from S(C₁-C₂-alkyl), S(O)(C₁-C₂-alkyl) and S(O)₂(C₁-C₂-alkyl), in particular SCH₃, S(O)(CH₃) and S(O)₂(CH₃). According to a specific embodiment R^{L} is selected from S(C₁-C₂-haloalkyl), S(O)(C₁-C₂-haloalkyl) and S(O)₂(C₁-C₂-haloalkyl), such as SO₂CF₃.

Particularly preferred embodiments of R^{L} according to the invention are in Table PL below, wherein each line of lines PL-1 to PL-16 corresponds to one particular embodiment of the invention, wherein PL-1 to PL-16 are also in any combination with one another a preferred embodiment of the present invention. Thereby, for every R^{L} that is present in the inventive compounds, these specific embodiments and preferences apply independently of the meaning of any other R^{L} that may be present in the phenyl ring:

**Table PL:**

| **No.** | **R^{L}** | | **No.** | **R^{L}** | | **No.** | **R^{L}** |
|---|---|---|---|---|---|---|---|
| PL-1 | Cl | | PL-7 | CF₃ | | PL-13 | SCH₃ |
| PL-2 | F | | PL-8 | CHF₂ | | PL-14 | SOCH₃ |
| PL-3 | CN | | PL-9 | OCH₃ | | PL-15 | SO₂CH₃ |
| PL-4 | NO₂ | | PL-10 | OCH₂CH₃ | | PL-16 | CO₂CH₃ |
| PL-5 | CH₃ | | PL-11 | OCF₃ | | | |
| PL-6 | CH₂CH₃ | | PL-12 | OCHF₂ | | | |

Particularly preferred embodiments of (R^{L})ₘ if Z is phenyl according to the invention are in Table P4 below, wherein each line of lines P4-1 to P4-155 corresponds to one particular embodiment of the invention, wherein P4-1 to P4-155 are also in any combination a preferred embodiment of the present invention.

**Table P4**

| **No.** | **(R^{L})ₘ** | | **No.** | **(R^{L})ₘ** | | **No.** | **(R^{L})ₘ** |
|---|---|---|---|---|---|---|---|
| P4-1 | * | | P4-40 | 3-F-4-Cl | | P4-79 | 3-OCF₃ |
| P4-2 | 2-Cl | | P4-41 | 2-F-6-Cl | | P4-80 | 4-OCF₃ |
| P4-3 | 3-Cl | | P4-42 | 2-Cl-3-F | | P4-81 | 2-OCHF₂ |
| P4-4 | 4-Cl | | P4-43 | 2-Cl-4-F | | P4-82 | 3-OCHF₂ |
| P4-5 | 2-F | | P4-44 | 3-Cl-4-F | | P4-83 | 4-OCHF₂ |
| P4-6 | 3-F | | P4-45 | 2,3,4-Cl₃ | | P4-84 | 2,3-(CH₃)₂ |
| P4-7 | 4-F | | P4-46 | 2,4,5-Cl₃ | | P4-85 | 2,4-(CH₃)₂ |
| P4-8 | 2-CN | | P4-47 | 3,4,5-Cl₃ | | P4-86 | 3,4-(CH₃)₂ |
| P4-9 | 3-CN | | P4-48 | 2,4,6-Cl₃ | | P4-87 | 2,6-(CH₃)₂ |
| P4-10 | 4-CN | | P4-49 | 2,3,4-F₃ | | P4-88 | 2,3-(CH₂CH₃)₂ |
| P4-11 | 2-NO₂ | | P4-50 | 2,4,5-F₃ | | P4-89 | 2,4-(CH₂CH₃)₂ |
| P4-12 | 3-NO₂ | | P4-51 | 3,4,5-F₃ | | P4-90 | 3,4-(CH₂CH₃)₂ |
| P4-13 | 4-NO₂ | | P4-52 | 2,4,6-F₃ | | P4-91 | 2,6-(CH₂CH₃)₂ |
| P4-14 | 2-SCH₃ | | P4-53 | 2,3-4-F₃ | | P4-92 | 2,3-(CF₃)₂ |
| P4-15 | 3-SCH₃ | | P4-54 | 2,4-F₂-3-Cl | | P4-93 | 2,4-(CF₃)₂ |
| P4-16 | 4-SCH₃ | | P4-55 | 2,6-F₂-4-Cl | | P4-94 | 3,4-(CF₃)₂ |
| P4-17 | 2-SOCH₃ | | P4-56 | 2,5-F₂-4-Cl | | P4-95 | 2,6-(CF₃)₂ |
| P4-18 | 3-SOCH₃ | | P4-57 | 2,4-Cl₂-3-F | | P4-96 | 2,3-(CHF₂)₂ |
| P4-19 | 4-SOCH₃ | | P4-58 | 2,6-Cl₂-4-F | | P4-97 | 2,4-(CHF₂)₂ |
| P4-20 | 2-SO₂CH₃ | | P4-59 | 2,5-Cl₂-4-F | | P4-98 | 3,4-(CHF₂)₂ |
| P4-21 | 3-SO₂CH₃ | | P4-60 | 2-CH₃ | | P4-99 | 2,6-(CHF₂)₂ |
| P4-22 | 4-SO₂CH₃ | | P4-61 | 3-CH₃ | | P4-100 | 2,3-(OCH₃)₂ |
| P4-23 | 2-CO₂CH₃ | | P4-62 | 4-CH₃ | | P4-101 | 2,4-(OCH₃)₂ |
| P4-24 | 3-CO₂CH₃ | | P4-63 | 2-CH₂CH₃ | | P4-102 | 3,4-(OCH₃)₂ |
| P4-25 | 4-CO₂CH₃ | | P4-64 | 3-CH₂CH₃ | | P4-103 | 2,6-(OCH₃)₂ |
| P4-26 | 2,3-Cl₂ | | P4-65 | 4-CH₂CH₃ | | P4-104 | 2,3-(OCH₂CH₃)₂ |
| P4-27 | 2,4-Cl₂ | | P4-66 | 2-CF₃ | | P4-105 | 2,4-(OCH₂CH₃)₂ |
| P4-28 | 2,5-Cl₂ | | P4-67 | 3-CF₃ | | P4-106 | 3,4-(OCH₂CH₃)₂ |
| P4-29 | 3,4-Cl₂ | | P4-68 | 4-CF₃ | | P4-107 | 2,6-(OCH₂CH₃)₂ |
| P4-30 | 3,5-Cl₂ | | P4-69 | 2-CHF₂ | | P4-108 | 2,3-(OCF₃)₂ |
| P4-31 | 2,6-Cl₂ | | P4-70 | 3-CHF₂ | | P4-109 | 2,4-(OCF₃)₂ |
| P4-32 | 2,3-F₂ | | P4-71 | 4-CHF₂ | | P4-110 | 3,4-(OCF₃)₂ |
| P4-33 | 2,4-F₂ | | P4-72 | 2-OCH₃ | | P4-111 | 2,6-(OCF₃)₂ |
| P4-34 | 2,5-F₂ | | P4-73 | 3-OCH₃ | | P4-112 | 2,3-(OCHF₂)₂ |
| P4-35 | 3,4-F₂ | | P4-74 | 4-OCH₃ | | P4-113 | 2,4-(OCHF₂)₂ |
| P4-36 | 3,5-F₂ | | P4-75 | 2-OCH₂CH₃ | | P4-114 | 3,4-(OCHF₂)₂ |
| P4-37 | 2,6-F₂ | | P4-76 | 3-OCH₂CH₃ | | P4-115 | 2,6-(OCHF₂)₂ |
| P4-38 | 2-F-3-Cl | | P4-77 | 4-OCH₂CH₃ | | P4-116 | 2,3,4-(CH₃)₃ |
| P4-39 | 2-F-4-Cl | | P4-78 | 2-OCF₃ | | P4-117 | 2,4,5-(CH₃)₃ |
| P4-118 | 3,4,5-(CH₃)₃ | | P4-131 | 2,4,6-(CHF₂)₃ | | P4-144 | 2,3,4-(OCHF₂)₃ |
| P4-119 | 2,4,6-(CH₃)₃ | | P4-132 | 2,3,4-(OCH₃)₃ | | P4-145 | 2,4,5-(OCHF₂)₃ |
| P4-120 | 2,3,4-(CH₂CH₃)₃ | | P4-133 | 2,4,5-(OCH₃)₃ | | P4-146 | 3,4,5-(OCHF₂)₃ |
| P4-121 | 2,4,5-(CH₂CH₃)₃ | | P4-134 | 3,4,5-(OCH₃)₃ | | P4-147 | 2,4,6-(OCHF₂)₃ |
| P4-122 | 3,4,5-(CH₂CH₃)₃ | | P4-135 | 2,4,6-(OCH₃)₃ | | P4-148 | 2-CF₃-4-Cl |
| P4-123 | 2,4,6-(CH₂CH₃)₃ | | P4-136 | 2,3,4-(OCH₂CH₃)₃ | | P4-149 | 2-CF₃-4-F |
| P4-124 | 2,3,4-(CF₃)₃ | | P4-137 | 2,4,5-(OCH₂CH₃)₃ | | P4-150 | 2-Cl-4-CF₃ |
| P4-125 | 2,4,5-(CF₃)₃ | | P4-138 | 3,4,5-(OCH₂CH₃)₃ | | P4-151 | 2-F-4-CF₃ |
| P4-126 | 3,4,5-(CF₃)₃ | | P4-139 | 2,4,6-(OCH₂CH₃)₃ | | P4-152 | 2-CN-4-Cl |
| P4-127 | 2,4,6-(CF₃)₃ | | P4-140 | 2,3,4-(OCF₃)₃ | | P4-153 | 2-CN-4-F |
| P4-128 | 2,3,4-(CHF₂)₃ | | P4-141 | 2,4,5-(OCF₃)₃ | | P4-154 | 2-Cl-4-CN |
| P4-129 | 2,4,5-(CHF₂)₃ | | P4-142 | 3,4,5-(OCF₃)₃ | | P4-155 | 2-F-4-CN |
| P4-130 | 3,4,5-(CHF₂)₃ | | P4-143 | 2,4,6-(OCF₃)₃ | | | |

| | | | | | | | |
|---|---|---|---|---|---|---|---|
| * means that m=0 | | | | | | | |

The following provisos apply:
if Z is phenyl, Y is a direct bond, n = 0 and D=H, the phenyl is substituted by (R^{L})ₘ with m=1, 2, 3 or 4; and
if Z is phenyl and Y is a divalent group selected from the group consisting of -O-, -S-, - NH- and -N(C₁-C₄-alkyl)-, Z-Y is not in the para-position in relation to the attachment of the phenyl group in formula I to the remaining backbone structure;

In another embodiment Z is a five- or six-membered heteroaryl that contains 1, 2, 3 or 4 heteroatoms selected from the group consisting of O, N and S, wherein the heteroaryl is unsubstituted (m=0) or substituted by (R^{L})ₘ. According to one embodiment thereof, Z is a five-membered heteroaryl which is unsubstituted or carries one, two or three independently selected radicals R^{L} as defined or preferably defined below. According to a further embodiment thereof, Z is a six-membered heteroaryl ahich is unsubstituted or carries one, two or three independently selected radicals R^{L} as defined or preferably defined below.

According to one embodiment thereof, Z is selected from the group consisting of pyrimidin-2-yl, pyrimidin-3-yl, pyrimidin-4-yl, pyridin-2-yl, pyridin-3-yl, pyridin-4-yl, thiazol-2-yl, thiazol-4-yl, thiazol-5-yl, isothiazol-3-yl, isothiazol-4-yl, isothiazol-5-yl, pyrazin-2-yl, pyridazin-3-yl, 1,3,5-triazin-2-yl and 1,2,4-triazin-3-yl; wherein said heteroaryl is unsubstituted or carrie one, two, three or four independently selected radicals R^{L} as defined or preferably defined below.

According to one specific embodiment of the invention Z is selected from the group consisting of pyrimidin-2-yl, pyrimidin-3-yl, pyrimidin-4-yl, pyridin-2-yl, pyridin-3-yl, pyridin-4-yl, thiazol-2-yl, pyrazin-2-yl, pyridazin-3-yl, 1,3,5-triazin-2-yl, and 1,2,4-triazin-3-yl; preferably Z is pyrimidin-2-yl, pyridin-2-yl, pyridin-3-yl, pyridin-4-yl and thiazol-2-yl, that are unsubstituted or carry one, two, three or four independently selected radicals R^{L} as defined or preferably defined below.

According to the invention, there can be zero, one, two, three, four or five R^{L} present, namely for m is 0, 1, 2, 3, 4 or 5. The number of m also depends on the kind of heteroaryl. In particular, m is 0, 1, 2 or 3. According to one embodiment, m is 0. According to a further embodiment, m is 1, 2 or 3, in particular 1 or 2. According to one specific embodiment thereof, m is 1, according to a further specific embodiment, m is 2.

For every R^{L} that is present in the inventive compounds, the following embodiments and preferences apply independently of the meaning of any other R^{L} that may be present in the heteroaryl ring. Furthermore, the particular embodiments and preferences given herein for R^{L} apply independently for each of m=1, m=2, m=3, m= 4 and m=5.

Each R^{L} is independently selected from halogen, CN, NO₂, OH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyl-C₁-C₄-alkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl and phenyl-C₁-C₄-alkyl, wherein the aliphatic, alicyclic and aromatic moieties of R^{L} are unsubstituted or substituted by one, two, three or four or up to the maximum possible number of R^{La}; wherein R^{La} is independently selected from halogen, CN, NO₂, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio and C₁-C₆-haloalkylthio.

According to one embodiment, R^{L} is independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl) (p=0, 1 or 2), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂); wherein each of R^{L} is unsubstituted or further substituted by one, two, three or four independently selected R^{La}, wherein R^{La} is as defined and preferably defined herein.

According to a further embodiment, R^{L} is independently selected from halogen, CN, NO₂, C₁-C₄-alkyl, C₁-C₄-alkoxy, C₂-C₄-alkenyl, C₂-C₄-alkynyl, C₃-C₆-cycloalkyl, C₃-C₆-cycloalkyloxy, NH₂, NH(C₁-C₄₂-alkyl), N(C₁-C₂-alkyl)₂, S(C₁-C₂-alkyl), S(O)(C₁-C₂-alkyl), S(O)₂(C₁-C₂-alkyl), C(=O)(C₁-C₂-alkyl), C(=O)(OH) and C(=O)(O-C₁-C₂-alkyl), wherein each of R^{L} is unsubstituted or further substituted by one, two, three or four independently selected R^{La}, wherein R^{La} is as defined and preferably defined herein. According to a further embodiment, R^{L} is independently selected from halogen, CN, NO₂, C₁-C₄-alkyl, C₁-C₄-haloalkyl, C₁-C₄-alkoxy, C₁-C₄-haloalkoxy, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, S(C₁-C₂-alkyl), S(O)(C₁-C₂-alkyl), S(O)₂(C₁-C₂-alkyl), C(=O)(C₁-C₂-alkyl), C(=O)(OH) and C(=O)(O-C₁-C₂-alkyl).

According to a further embodiment, R^{L} is independently selected from halogen, CN, NO₂, C₁-C₂-alkyl, C₁-C₂-haloalkyl, C₁-C₂-alkoxy, C₁-C₂-haloalkoxy, S(C₁-C₂-alkyl), S(O)(C₁-C₂-alkyl), S(O)₂(C₁-C₂-alkyl), C(=O)(OH) and C(=O)(O-C₁-C₂-alkyl). According to a further embodiment, R^{L} is independently selected from F, Cl, Br, CN, C₁-C₄-alkyl, C₁-C₄-haloalkyl, C₁-C₄-alkoxy, C₁-C₄-haloalkoxy, S(C₁-C₄-alkyl), S(O)(C₁-C₄-alkyl) and S(O)₂(C₁-C₄-alkyl).

According to still a further specific embodiment, R^{L} is independently selected from halogen, in particular from Br, F and Cl, more specifically from F and Cl.

According to a further specific embodiment, R^{L} is CN.
According to one further embodiment R^{L} is NO₂.
According to one further embodiment R^{L} is OH.
According to one further embodiment R^{L} is SH.

According to a further specific embodiment, R^{L} is C₁-C₆-alkyl, in particular C₁-C₄-alkyl, such as CH₃. Further appropriate alkyls are ethyl, n-propyl, i-propyl, n-butyl, i-butyl and t-butyl.

According to a further specific embodiment, R^{L} is C₁-C₆-haloalkyl, in particular C₁-C₄-haloalkyl, such as CF₃, CHF₂, CH₂F, CCHl₃, CHCl₂ or CH₂Cl.

According to a further specific embodiment R^{L} is C₁-C₆-alkyl, preferably C₁-C₄-alkyl, substituted by OH, more preferably CH₂OH, CH₂CH₂OH, CH₂CH₂CH₂OH, CH(CH₃)CH₂OH, CH₂CH(CH₃)OH, CH₂CH₂CH₂CH₂OH. In a special embodiment R^{L} is CH₂OH. According to a further specific embodiment R^{L} is C₁-C₆-alkyl, preferably C₁-C₄-alkyl substituted by CN, more preferably CH₂CN, CH₂CH₂CN, CH₂CH₂CH₂CN, CH(CH₃)CH₂CN, CH₂CH(CH₃)CN, CH₂CH₂CH₂CH₂CN. In a special embodiment R^{L} is CH₂CH₂CN. In a further special embodiment R⁴ is CH(CH₃)CN. According to a further specific embodiment R^{L} is C₁-C₄-alkoxy-C₁-C₆-alkyl, more preferably C₁-C₄-alkoxy-C₁-C₄-alkyl. In a special embodiment R^{L} is CH₂OCH₃. In a further special embodiment R^{L} is CH₂CH₂OCH₃. In a further special embodiment R^{L} is CH(CH₃)OCH₃. In a further special embodiment R^{L} is CH(CH₃)OCH₂CH₃. In a further special embodiment R^{L} is CH₂CH₂OCH₂CH₃. According to a further specific embodiment R^{L} is C₁-C₄-haloalkoxy-C₁-C₆-alkyl, more preferably C₁-C₄-alkoxy-C₁-C₄-alkyl. In a special embodiment R^{L} is CH₂OCF₃. In a further special embodiment R^{L} is CH₂CH₂OCF₃. In a further special embodiment R^{L} is CH₂OCCl₃. In a further special embodiment R^{L} is CH₂CH₂OCCl₃. According to a further specific embodiment, R^{L} is C₁-C₆-alkoxy, in particular C₁-C₄-alkoxy, more specifically C₁-C₂-alkoxy such as OCH₃ or OCH₂CH₃.

According to a further specific embodiment, R^{L} is C₁-C₆-haloalkoxy, in particular C₁-C₄-haloalkoxy, more specifically C₁-C₂-haloalkoxy such as OCF₃, OCHF₂, OCH₂F, OCCl₃, OCHCl₂ or OCH₂Cl, in particular OCF₃, OCHF₂, OCCl₃ or OCHCl₂.

According to still a further embodiment, R^{L} is C₂-C₆-alkenyl or C₂-C₆-haloalkenyl, in particular C₂-C₄-alkenyl or C₂-C₄-haloalkenyl, such as CH=CH₂, CH₂CH=CH₂, CH=CHCH₃ or C(CH₃)=CH₂.

According to a further specific embodiment R^{L} is C₂-C₆-alkenyl, preferably C₂-C₄-alkenyl, substituted by OH, more preferably, CH=CHOH, CH=CHCH₂OH, C(CH₃)=CHOH, CH=C(CH₃)OH. In a special embodiment R^{L} is CH=CHOH. In a further special embodiment R^{L} is CH=CHCH₂OH. According to a further specific embodiment R^{L} is C₁-C₄-alkoxy-C₂-C₆-alkenyl, more preferably C₁-C₄-alkoxy-C₂-C₄-alkenyl. In a special embodiment R^{L} is CH=CHOCH₃. In a further special embodiment R^{L} is CH=CHCH₂OCH₃. According to a further specific embodiment R^{L} is C₁-C₄-haloalkoxy-C₂-C₆-alkenyl, more preferably C₁-C₄-haloalkoxy-C₂-C₄-alkenyl. In a special embodiment R^{L} is CH=CHOCF₃. In a further special embodiment R^{L} is CH=CHCH₂OCF₃. In a further special embodiment R^{L} is CH=CHOCCl₃. In a further special embodiment R^{L} is CH=CHCH₂OCCl₃. According to a further specific embodiment R^{L} is C₃-C₈-cycloalkyl-C₂-C₆-alkenyl, preferably C₃-C₆-cycloalkyl-C₂-C₄-alkenyl. According to a further specific embodiment R^{L} is C₃-C₆-halocycloalkyl-C₂-C₄-alkenyl, preferably C₃-C₈-halocycloalkyl-C₂-C₆-alkenyl.

According to still a further embodiment, R^{L} is C₂-C₆-alkynyl or C₂-C₆-haloalkynyl, in particular C₂-C₄-alkynyl or C₂-C₄-haloalkynyl, such as C-CH, CH₂CCH or CH₂CCCH₃.

According to a further specific embodiment R^{L} is C₂-C₆-alkynyl, preferably C₂-C₄-alkynyl, substituted by OH, more preferably, CCOH, CH₂CCOH. In a special embodiment R^{L} is CCOH. In a further special embodiment R^{L} is CH₂CCOH. According to a further specific embodiment R^{L} is C₁-C₄-alkoxy-C₂-C₆-alkynyl, more preferably C₁-C₄-alkoxy-C₂-C₄-alkynyl. In a special embodiment R^{L} is CCOCH₃. In a further special embodiment R^{L} is CH₂CCOCH₃. According to a further specific embodiment R^{L} is C₁-C₄-haloalkoxy-C₂-C₆-alkynyl, more preferably C₁-C₄-haloalkoxy-C₂-C₄-alkynyl. In a special embodiment R^{L} is CCOCF₃. In a further special embodiment R^{L} is CH₂CCOCF₃. In a further special embodiment R^{L} is CCOCCl₃. In a further special embodiment R^{L} is CH₂CCOCCl₃. According to a further specific embodiment R^{L} is C₃-C₈-cycloalkyl-C₂-C₆-alkynyl, preferably C₃-C₆-cycloalkyl-C₂-C₄-alkynyl. According to a further specific embodiment R^{L} is C₃-C₆-halocycloalkyl-C₂-C₄-alkynyl, preferably C₃-C₈-halocycloalkyl-C₂-C₆-alkynyl.

According to one another embodiment R^{L} is C₃-C₈-cycloalkyl, preferably cyclopropyl, cyclobutyl, cyclopentyl or cyclohexyl, in particular cyclopropyl or cyclobutyl. In a special embodiment R^{L} is cyclopropyl. In a further special embodiment R^{L} is cyclobutyl. In a further special embodiment R⁴ is cyclopentyl. In a further special embodiment R^{L} is cyclohexyl.

According to one another embodiment R^{L} is C₃-C₈-cycloalkoxy, preferably C₃-C₆-cycloalkoxy. In a special embodiment R^{L} is O-cyclopropyl.

According to a specific embodiment R^{L} is C₃-C₈-halocycloalkyl, more preferably fully or partially halogenated C₃-C₆-cycloalkyl. In a special embodiment R^{L} is fully or partially halogenated cyclopropyl. In a further special embodiment R^{L} is 1-Cl-cyclopropyl. In a further special embodiment R^{L} is 2-Cl-cyclopropyl. In a further special embodiment R^{L} is 1-F-cyclopropyl. In a further special embodiment R^{L} is 2-F-cyclopropyl. In a further special embodiment R^{L} is fully or partially halogenated cyclobutyl. In a further special embodiment R^{L} is 1-Cl-cyclobutyl. In a further special embodiment R^{L} is 1-F-cyclobutyl. In a further special embodiment R^{L} is 3,3-Cl₂-cyclobutyl. In a further special embodiment R^{L} is 3,3-F₂-cyclobutyl. According to a specific embodiment R^{L} is C₃-C₈-cycloalkyl substituted by C₁-C₄-alkyl, more preferably is C₃-C₆-cycloalkyl substituted by C₁-C₄-alkyl. In a special embodiment R^{L} is 1-CH₃-cyclopropyl. According to a specific embodiment R^{L} is C₃-C₈-cycloalkyl substituted by CN, more preferably is C₃-C₆-cycloalkyl substituted by CN. In a special embodiment R^{L} is 1-CN-cyclopropyl.According to a further specific embodiment R^{L} is C₃-C₈-cycloalkyl-C₃-C₈-cycloalkyl, preferably C₃-C₆-cycloalkyl-C₃-C₆-cycloalkyl. In a special embodiment R^{L} is cyclopropyl-cyclopropyl. In a special embodiment R^{L} is 2-cyclopropyl-cyclopropyl. According to a further specific embodiment R^{L} is C₃-C₈-cycloalkyl-C₃-C₈-halocycloalkyl, preferably C₃-C₆-cycloalkyl-C₃-C₆-halocycloalkyl.

According to one another embodiment R^{L} is C₃-C₈-cydoalkyl-C₁-C₄-alkyl, preferably C₃-C₆-cycloalkyl-C₁-C₄-alkyl. In a special embodiment R^{L} is CH(CH₃)(cyclopropyl). In a further special embodiment R^{L} is CH₂-(cyclopropyl).

According to a further preferred embodiment R^{L} is C₃-C₈-cycloalkyl-C₁-C₄-alkyl wherein the alkyl moiety can be substituted by one, two, three or up to the maximum possible number of identical or different groups R^{a} as defined and preferably herein and the cycloalkyl moiety can be substituted by one, two, three or up to the maximum possible number of identical or different groups R^{b} as defined and preferably herein.

According to a specific embodiment R^{L} is C₃-C₈-cycoalkyl-C₁-C₄-haloalkyl, C₃-C₆-cycloalkyl-C₁-C₄-haloalkyl. According to a specific embodiment R^{L} is C₃-C₈-halocycloalkyl-C₁-C₄-alkyl, C₃-C₆-halocycloalkyl-C₁-C₄-alkyl. In a special embodiment R^{L} is fully or partially halogenated cyclopropyl-C₁-C₄-alkyl. In a further special embodiment R^{L} is 1-Cl-cyclopropyl-C₁-C₄-alkyl. In a further special embodiment R^{L} is 1-F-cyclopropyl-C₁-C₄-alkyl.

According to one another embodiment R^{L} is NH₂.
According to one another embodiment R^{L} is NH(C₁-C₄-alkyl). According to a specific embodiment R^{L} is NH(CH₃). According to a specific embodiment R^{L} is NH(CH₂CH₃). According to a specific embodiment R^{L} is NH(CH₂CH₂CH₃). According to a specific embodiment R^{L} is NH(CH(CH₃)₂). According to a specific embodiment R^{L} is NH(CH₂CH₂CH₂CH₃). According to a specific embodiment R^{L} is NH(C(CH₃)₃).

According to one another embodiment R^{L} is N(C₁-C₄-alkyl)₂. According to a specific embodiment R^{L} is N(CH₃)₂. According to a specific embodiment R^{L} is N(CH₂CH₃)₂. According to a specific embodiment R^{L} is N(CH₂CH₂CH₃)₂. According to a specific embodiment R^{L} is N(CH(CH₃)₂)₂. According to a specific embodiment R^{L} is N(CH₂CH₂CH₂CH₃)₂. According to a specific embodiment R^{L} is NH(C(CH₃)₃)₂.

According to one another embodiment R^{L} is NH(C₃-C₈-cycloalkyl) preferably NH(C₃-C₆-cycloalkyl). According to a specific embodiment R^{L} is NH(cyclopropyl). According to a specific embodiment R^{L} is NH(cyclobutyl). According to a specific embodiment R^{L} is NH(cyclopentyl). According to a specific embodiment R^{L} is NH(cyclohexyl).

According to one another embodiment R^{L} is N(C₃-C₈-cycloalkyl)₂ preferably N(C₃-C₆-cycloalkyl)₂. According to a specific embodiment R^{L} is N(cyclopropyl)₂. According to a specific embodiment R^{L} is N(cyclobutyl)₂. According to a specific embodiment R^{L} is N(cyclopentyl)₂. According to a specific embodiment R^{L} is N(cyclohexyl)₂.

According to still a further embodiment, R^{L} is selected from C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)(N(C₃-C₆-cycloalkyl)₂), in particular selected from C(=O)(C₁-C₂-alkyl), C(=O)(OH), C(=O)(O-C₁-C₂-alkyl), C(=O)(NH(C₁-C₂-alkyl)), C(=O)(N(C₁-C₂-alkyl)₂), C(=O)(NH(C₃-C₆-cycloalkyl)) and C(=O)(N(C₃-C₆-cycloalkyl)₂). According to one specific embodiment thereof, R^{L} is C(=O)(OH) or C(=O)(O-C₁-C₄-alkyl), in particular C(=O)(OCH₃).

According to one another embodiment R^{L} is C(=O)(-C₁-C₄-alkyl). According to a specific embodiment R^{L} is C(=O)CH_{3.}. According to a further specific embodiment R^{L} is C(=O)CH₂CH_{3.}According to a further specific embodiment R^{L} is C(=O)CH₂CH₂CH_{3.}According to a further specific embodiment R^{L} is C(=O)CH(CH₃)_{2.} According to a further specific embodiment R^{L} is C(=O)C(CH₃)₃.

According to one another embodiment R^{L} is C(=O)OH.

According to one another embodiment R^{L} is C(=O)(-O-C₁-C₄-alkyl). According to a specific embodiment R^{L} is C(=O)OCH₃.. According to a further specific embodiment R^{L} is C(=O)OCH₂CH₃. According to a further specific embodiment R^{L} is C(=O)OCH₂CH₂CH₃. According to a further specific embodiment R^{L} is C(=O)OCH(CH₃)₂. According to a further specific embodiment R^{L} is C(=O)OC(CH₃)_{3.}

According to one another embodiment R^{L} is C(=O)-NH(C₁-C₄-alkyl). According to a specific embodiment R^{L} is C(=O)NHCH₃.. According to a further specific embodiment R^{L} is C(=O)NHCH₂CH_{3.}According to a further specific embodiment R^{L} is C(=O)NHCH₂CH₂CH_{3.}According to a further specific embodiment R^{L} is C(=O)NHCH(CH₃)_{2.}According to a further specific embodiment R^{L} is C(=O)NHC(CH₃)_{3.}

According to one another embodiment R^{L} is C(=O)-N(C₁-C₄-alkyl)₂. According to a specific embodiment R^{L} is C(=O)N(CH₃)₂. According to a further specific embodiment R^{L} is C(=O)N(CH₂CH₃)₂. According to a further specific embodiment R^{L} is C(=O)N(CH₂CH₂CH₃)_{2.} According to a further specific embodiment R^{L} is C(=O)N(CH(CH₃)₂)₂. According to a further specific embodiment R^{L} is C(=O)N(C(CH₃)₃)₂.

According to one another embodiment R^{L} is C(=O)-NH(C₃-C₆-cycloalkyl). According to a specific embodiment R^{L} is C(=O)NH(cyclopropyl).. According to a further specific embodiment R^{L} is C(=O)NH(cyclobutyl). According to a further specific embodiment R^{L} is C(=O)NH(cyclopentyl).According to a further specific embodiment R^{L} is C(=O)NH(cyclohexyl).

According to one another embodiment R^{L} is C(=O)-N(C₃-C₆-cycloalkyl)₂. According to a specific embodiment R^{L} is C(=O)N(cyclopropyl)₂. According to a further specific embodiment R^{L} is C(=O)N(cyclobutyl)₂. According to a further specific embodiment R^{L} is C(=O)N(cyclopentyl)₂.According to a further specific embodiment R^{L} is C(=O)N(cyclohexyl)₂.

According to still a further embodiment, R^{L} is selected from S(C₁-C₂-alkyl), S(O)(C₁-C₂-alkyl) and S(O)₂(C₁-C₂-alkyl), in particular SCH₃, S(O)(CH₃) and S(O)₂(CH₃). According to a specific embodiment R^{L} is selected from S(C₁-C₂-haloalkyl), S(O)(C₁-C₂-haloalkyl) and S(O)₂(C₁-C₂-haloalkyl), such as SO₂CF_{3.}

Particularly preferred embodiments of R^{L} present in the heteroaryl according to the invention are in Table PL above, wherein each line of lines PL-1 to PL-16 corresponds to one particular embodiment of the invention, wherein PL-1 to PL-16 are also in any combination with one another a preferred embodiment of the present invention. Thereby, for every R^{L} that is present in the inventive compounds, these specific embodiments and preferences apply independently of the meaning of any other R^{L} that may be present in the heteroaryl ring.

Particularly preferred embodiments of of Z with (R^{L})ₘ if Z is heteroaryl according to the invention are in Table H below, wherein each line of lines H-1 to H-155 corresponds to one particular embodiment of the invention, wherein H-1 to H-155 are also in any combination a preferred embodiment of the present invention.

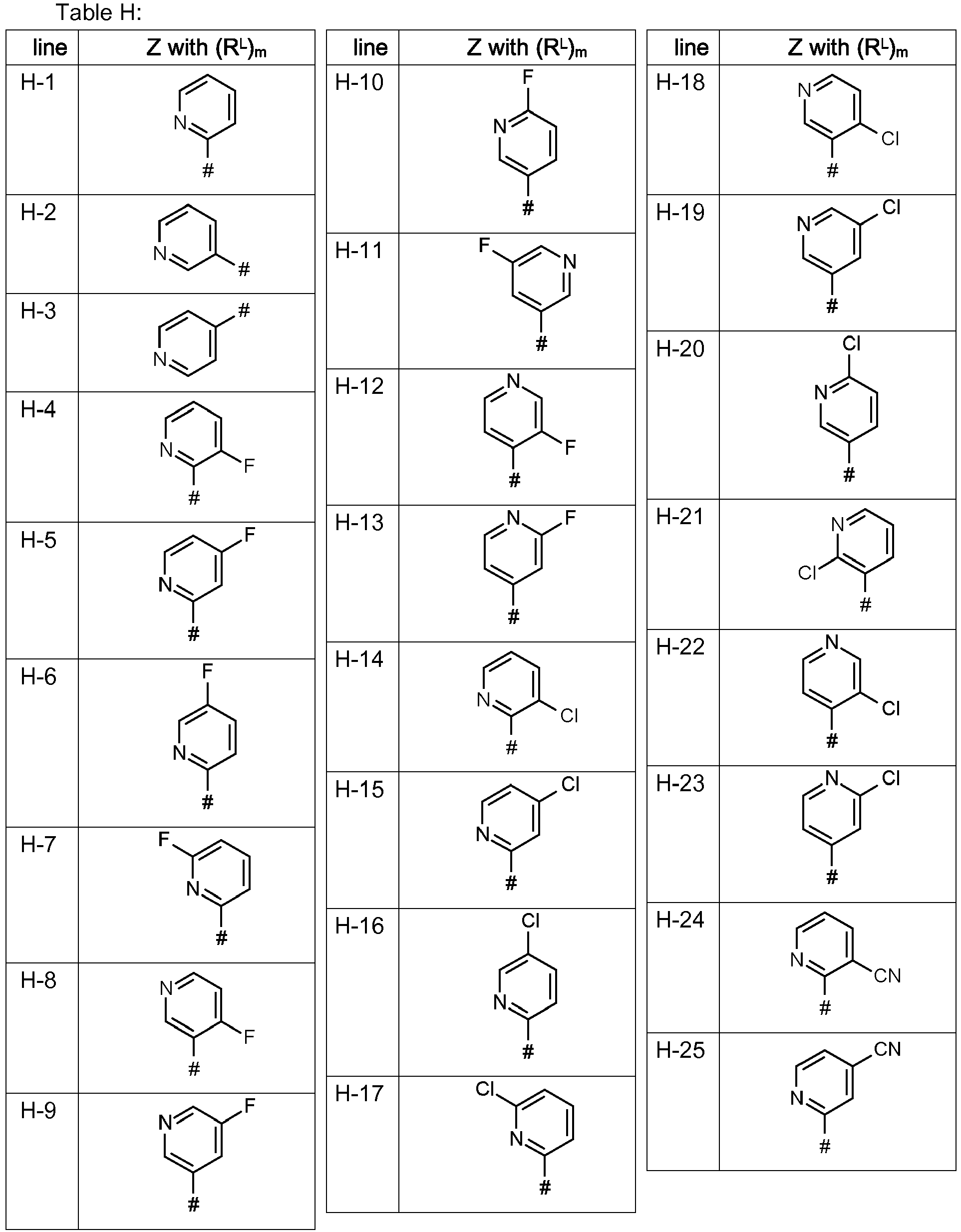

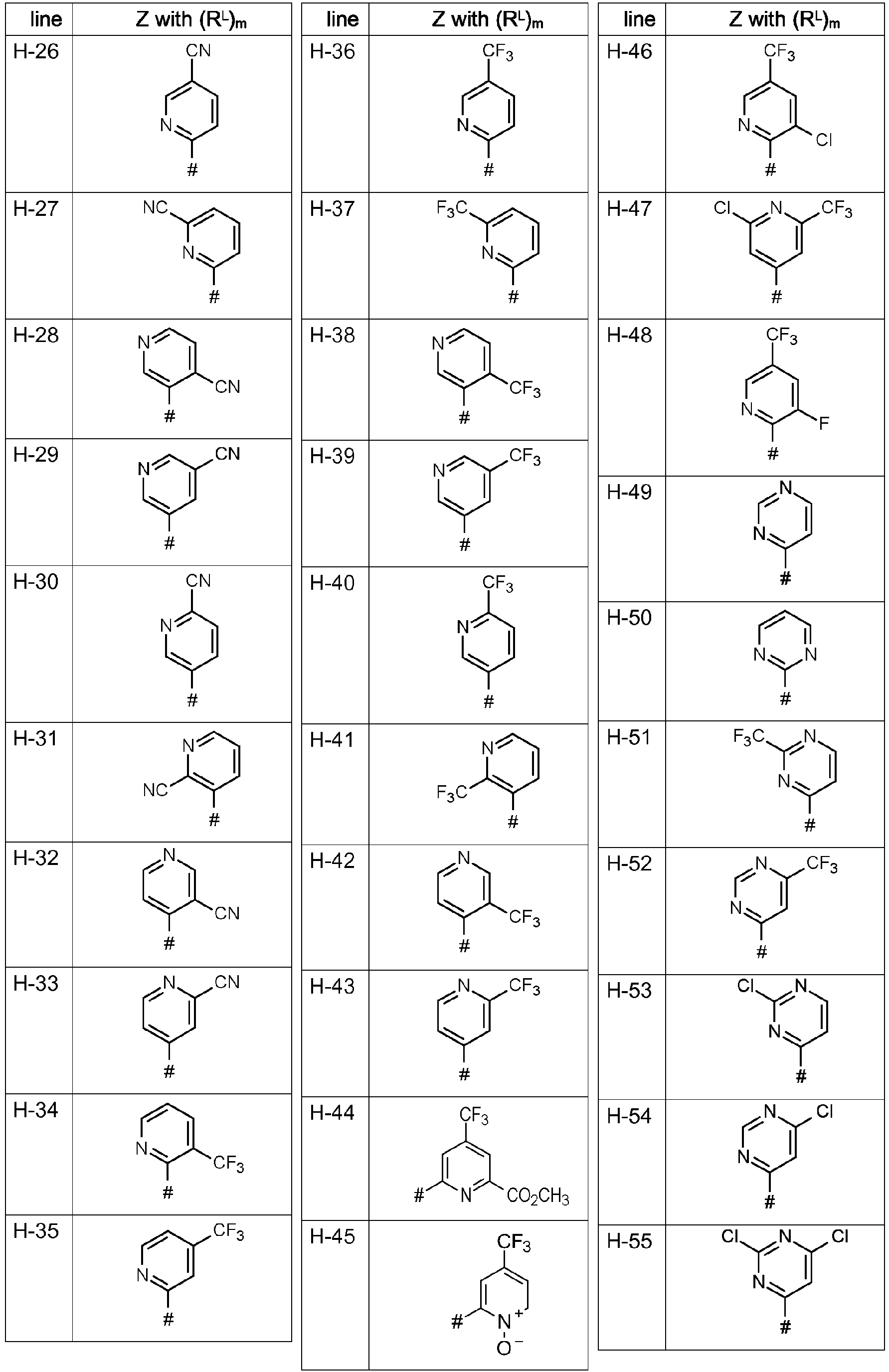

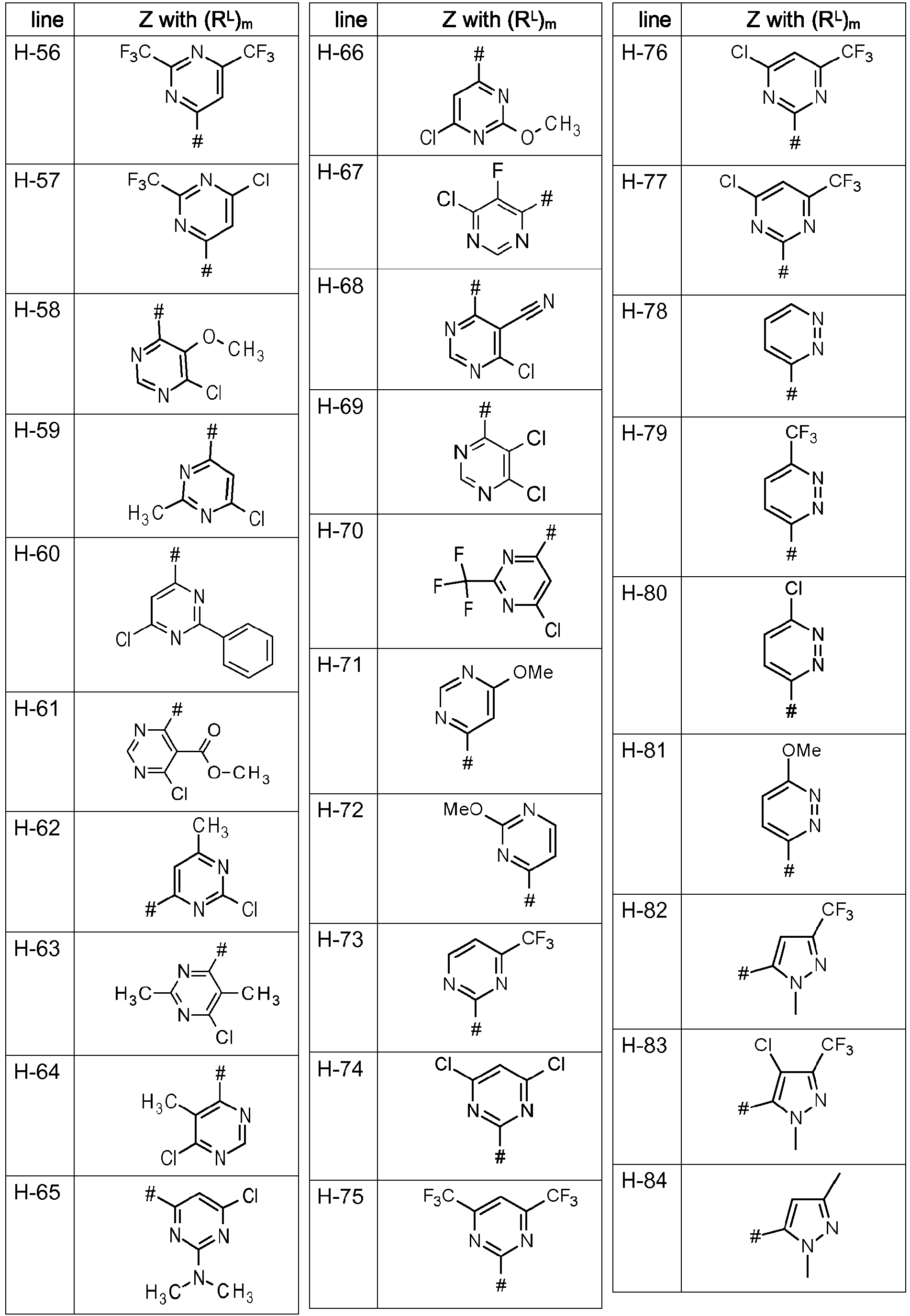

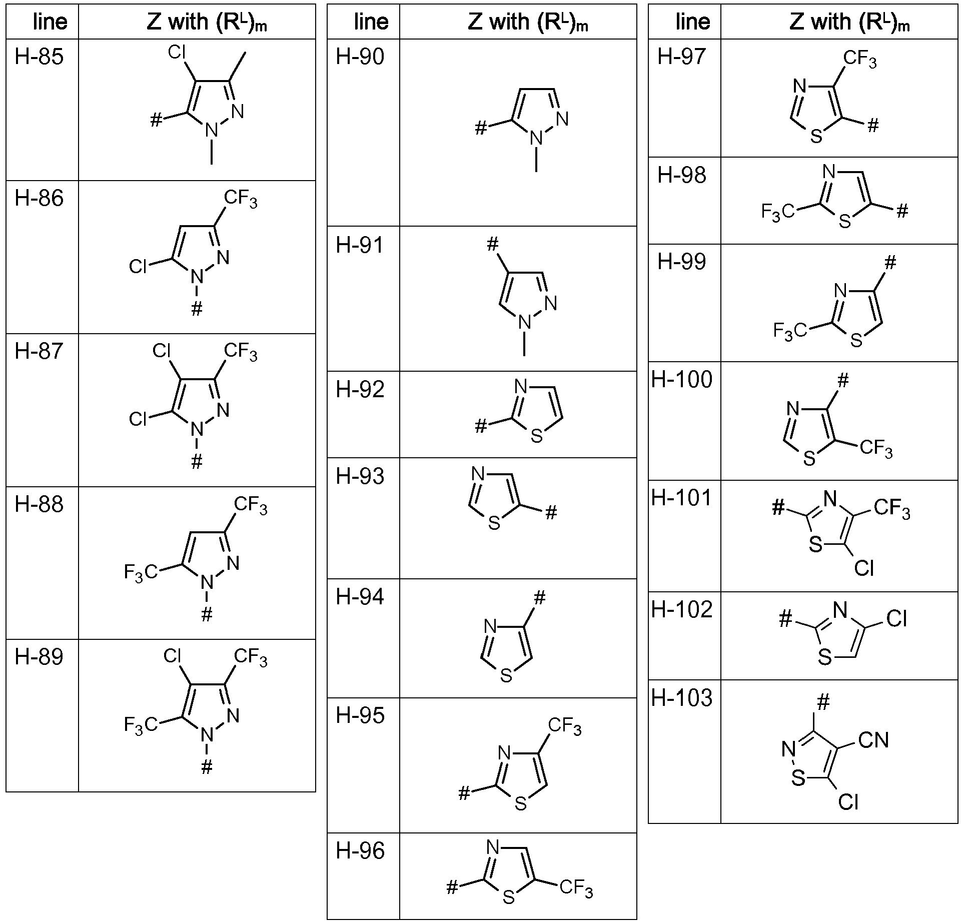

In which # indicates the point of attachment of yhe grop Y.

Each R⁴ according to the present invention is independently selected from halogen, CN, NO₂, OH, SH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₆-cycloalkyl, C₃-C₆-cycloalkyl, C₁-C₄-alkyl, C₁-C₄-alkyl, C₃-C₆-cycloalkyloxy), phenyl, phenoxy, a 5- or 6-membered heteroaryl, a 5- or 6-membered heteroaryloxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-aromatic moieties of R⁴ are unsubstituted or substituted by one, two, three or four or up to the maximum possible nimber of R^{4a}; wherein R^{4a} is independently selected from halogen, CN, NO₂, OH, C₁-C₄-alkyl, C₁-C₄-haloalkyl, C₃-C₅-cycloalkyl, C₃-C₅-halocycloalkyl, C₁-C₄-alkoxy and C₁-C₄-halogenalkoxy.

According to the invention, there can be zero, one, two, three or four R⁴ present, namely for n is 0, 1, 2, 3 or 4.

According to one embodiment, n is 0. According to a further embodiment, n is 1.
According to a further embodiment, n is 0 or 2.
According to a further embodiment, n is 2 or 3. According to one specific embodiment thereof, n is 2, according to a further specific embodiment, n is 3.
According to one embodiment of the invention, one R⁴ is attached to the 2-position (R⁴¹).
According to one specific embodiment thereof, n is 1, according to a further specific embodiment, n is 2.
According to one embodiment of the invention, one R⁴ is attached to the 3-position (R⁴²).
According to one specific embodiment thereof, n is 1, according to a further specific embodiment, n is 2.
According to a further embodiment of the invention, one R⁴ is attached to the 4-position (R⁴³).
According to one specific embodiment thereof, n is 1, according to a further specific embodiment, n is 2.
According to a further embodiment of the invention, one R⁴ is attached to the 5-position (R⁴⁴).
According to one specific embodiment thereof, n is 1, according to a further specific embodiment, n is 2.
According to still a further embodiment, n is 1, 2 or 3 and one R⁴ is in 2- or 6-position.
According to a further embodiment of the invention, one R⁴ is attached to the 6-position (R⁴⁵).
According to one specific embodiment thereof, n is 1, according to a further specific embodiment, n is 2.
According to a further embodiment of the invention, two R⁴ are attached in 2,3-position.
According to one specific embodiment thereof, n is 2, according to a further specific embodiment, n is 3.
According to a further embodiment of the invention, two R⁴ are attached in 2,4-position.
According to one specific embodiment thereof, n is 2, according to a further specific embodiment, n is 3.
According to a further embodiment of the invention, two R⁴ are attached in 2,5-position.
According to one specific embodiment thereof, n is 2, according to a further specific embodiment, n is 3.
According to a further embodiment of the invention, two R⁴ are attached in 2,6-position.
According to one specific embodiment thereof, n is 2, according to a further specific embodiment, n is 3.
According to a further embodiment of the invention, two R⁴ are attached in 3,4-position.
According to one specific embodiment thereof, n is 2, according to a further specific embodiment, n is 3.
According to a further embodiment of the invention, two R⁴ are attached in 3,5-position.
According to one specific embodiment thereof, n is 2, according to a further specific embodiment, n is 3.
According to a further embodiment of the invention, two R³ are attached in 3,6-position.
According to one specific embodiment thereof, n is 2, according to a further specific embodiment, n is 3.

For every R⁴ (or R⁴¹, R⁴², R⁴³, R⁴⁴, R⁴⁵, respectively) that is present in the inventive compounds, the following embodiments and preferences apply independently of the meaning of any other R⁴ (or R⁴¹, R⁴², R⁴³, R⁴⁴, R⁴⁵, respectively) that may be present in the phenyl ring. Furthermore, the particular embodiments and preferences given herein for R⁴ (or R⁴¹, R⁴², R⁴³, R⁴⁴, R⁴⁵, respectively) apply independently for each of n=1, n=2, n=3 and n=4.

According to one embodiment, R⁴ is independently selected from halogen, CN, NO₂, C₁-C₄-alkyl, C₁-C₄-haloalkyl, C₁-C₄-alkoxy, C₁-C₄-haloalkoxy, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, S(C₁-C₂-alkyl), S(O)(C₁-C₂-alkyl), S(O)₂(C₁-C₂-alkyl), C(=O)(C₁-C₂-alkyl), C(=O)(OH) and C(=O)(O-C₁-C₂-alkyl).

According to a further embodiment, R⁴ is independently selected from halogen, CN, NO₂, OH, SH, C₁-C₆-alkyl, C₁-C₆-alkoxy, C₂-C₆-alkenyl, C₂-C₆-alkynyl, C₃-C₈-cycloalkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, S(O)ₚ(C₁-C₄-alkyl) (p=0, 1 or 2), C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)-(N(C₃-C₆-cycloalkyl)₂); wherein each of R⁴ is unsubstituted or further substituted by one, two, three or four R^{4a}, wherein R^{4a} is as defined and preferably defined herein.

According to still a further embodiment, R⁴ is independently selected from halogen, CN, NO₂, C₁-C₄-alkyl, C₁-C₄-haloalkyl, C₁-C₄-alkoxy, C₁-C₄-haloalkoxy, C₂-C₄-alkenyl, C₂-C₄-haloalkenyl, C₂-C₄-alkynyl, C₂-C₄-haloalkynyl, C₃-C₆-cycloalkyl, C₃-C₆-halocycloalkyl, S(C₁-C₂-alkyl), S(O)(C₁-C₂-alkyl), S(O)₂(C₁-C₂-alkyl), C(=O)(C₁-C₂-alkyl), C(=O)(OH) and C(=O)(O-C₁-C₂-alkyl).

According to still a further embodiment, R⁴ is independently selected from F, Cl, Br, CN, C₁-C₄-alkyl, C₁-C₄-haloalkyl, C₁-C₄-alkoxy, C₁-C₄-haloalkoxy, S(C₁-C₄-alkyl), S(O)(C₁-C₄-alkyl) and S(O)₂(C₁-C₄-alkyl).

According to one specific embodiment, R⁴ is halogen, in particular Br, F or Cl, more specifically F or Cl.

According to a further specific embodiment, R⁴ is CN.
According to a further specific embodiment, R⁴ is C₁-C₆-alkyl, in particular C₁-C₄-alkyl, such as CH₃.

According to a further specific embodiment, R⁴ is C₁-C₆-haloalkyl, in particular C₁-C₄-haloalkyl, such as CF₃, CHF₂, CH₂F, CCl₃, CHCl₂ or CH₂Cl.

According to a further specific embodiment, R⁴ is C₁-C₆-alkoxy, in particular C₁-C₄-alkoxy, more specifically C₁-C₂-alkoxy such as OCH₃ or OCH₂CH₃.

According to a further specific embodiment, R⁴ is C₁-C₆-haloalkoxy, in particular C₁-C₄-haloalkoxy, more specifically C₁-C₂-haloalkoxy such as OCF₃, OCHF₂, OCH₂F, OCCl₃, OCHCl₂ or OCH₂Cl, in particular OCF₃, OCHF₂, OCCl₃ or OCHCl₂.

According to still a further embodiment, R⁴ is C₂-C₆-alkenyl or C₂-C₆-haloalkenyl, in particular C₂-C₄-alkenyl or C₂-C₄-haloalkenyl, such as CH=CH₂.

According to still a further embodiment, R⁴ is C₂-C₆-alkynyl or C₂-C₆-haloalkynyl, in particular C₂-C₄-alkynyl or C₂-C₄-haloalkynyl, such as CΞCH.

According to still a further embodiment, R⁴ is selected from C(=O)(C₁-C₄-alkyl), C(=O)(OH), C(=O)(O-C₁-C₄-alkyl), C(=O)(NH(C₁-C₄-alkyl)), C(=O)(N(C₁-C₄-alkyl)₂), C(=O)(NH(C₃-C₆-cycloalkyl)) and C(=O)(N(C₃-C₆-cycloalkyl)₂), in particular selected from C(=O)(C₁-C₂-alkyl), C(=O)(OH), C(=O)(O-C₁-C₂-alkyl), C(=O)(NH(C₁-C₂-alkyl)), C(=O)(N(C₁-C₂-alkyl)₂), C(=O)(NH(C₃-C₆-cycloalkyl)) and C(=O)(N(C₃-C₆-cycloalkyl)₂). According to one specific embodiment thereof, R⁴ is C(=O)(OH) or C(=O)(O-C₁-C₄-alkyl), in particular C(=O)(OCH₃).

According to still a further embodiment, R⁴ is selected from S(C₁-C₂-alkyl), S(O)(C₁-C₂-alkyl) and S(O)₂(C₁-C₂-alkyl), in particular SCH₃, S(O)(CH₃) and S(O)₂(CH₃).

According to still a further embodiment, R⁴ is unsubstituted phenyl or phenyl that is substituted by one, two, three or four R^{4a}, as defined herein.

According to still a further embodiment, R⁴ is unsubstituted phenoxy or phenoxy that is substituted by one, two, three or four R^{4a}, as defined herein.

According to still a further embodiment, R⁴ is unsubstituted 5- or 6-membered heteroaryl. According to still a further embodiment, R⁴ is 5- or 6-membered heteroaryl that is substituted by one, two or three R^{4a}, as defined herein. According to one specific embodiment, the heteroaryl in each case is 5-membered such as . According to a further specific embodiment, the heteroaryl in each case is 6-membered such as .

According to still a further embodiment, R⁴ is unsubstituted 5- or 6-membered heteroaryloxy. According to still a further embodiment, R⁴ is 5- or 6-membered heteroaryloxy that is substituted by one, two or three R^{4a}, as defined herein. According to one specific embodiment, the heteroaryloxy in each case is 5-membered. According to a further specific embodiment, the heteroaryloxy in each case is 6-membered.

R^{4a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-halogenalkoxy, in particular selected from halogen, CN, C₁-C₂-alkyl, C₁-C₂-haloalkyl, C₃-C₆-cycloalkyl, C₃-C₆-halocycloalkyl, C₁-C₂-alkoxy and C₁-C₂-halogenalkoxy. Specifically, R^{4a} is independently selected from F, Cl, CN, OH, CH₃, halomethyl, cyclopropyl, halocyclopropyl, OCH₃ and halogenmethoxy.

According to a specific embodiment, Z is phenyl and m+n = 2, 3, 4, 5 or 6.
Particularly preferred embodiments of R⁴ according to the invention are in Table P5 below, wherein each line of lines P5-1 to P5-16 corresponds to one particular embodiment of the invention, wherein P5-1 to P5-16 are also in any combination with one another a preferred embodiment of the present invention. Thereby, for every R⁴ that is present in the inventive compounds, these specific embodiments and preferences apply independently of the meaning of any other R⁴ that may be present in the phenyl ring:

**Table P5:**

| No. | R4 | | No. | R4 | | No. | R4 |
|---|---|---|---|---|---|---|---|
| P5-1 | Cl | | P5-7 | CF₃ | | P5-13 | SCH₃ |
| P5-2 | F | | P5-8 | CHF₂ | | P5-14 | SOCH₃ |
| P5-3 | CN | | P5-9 | OCH₃ | | P5-15 | SO₂CH₃ |
| P5-4 | NO₂ | | P5-10 | OCH₂CH₃ | | P5-16 | CO₂CH₃ |
| P5-5 | CH₃ | | P5-11 | OCF₃ | | | |
| P5-6 | CH₂CH₃ | | P5-12 | OCHF₂ | | | |

Particularly preferred embodiments of (R⁴)ₙ according to the invention are in Table P6 below, wherein each line of lines P6-1 to P6-155 corresponds to one particular embodiment of the invention, wherein P6-1 to P6-155 are also in any combination a preferred embodiment of the present invention.

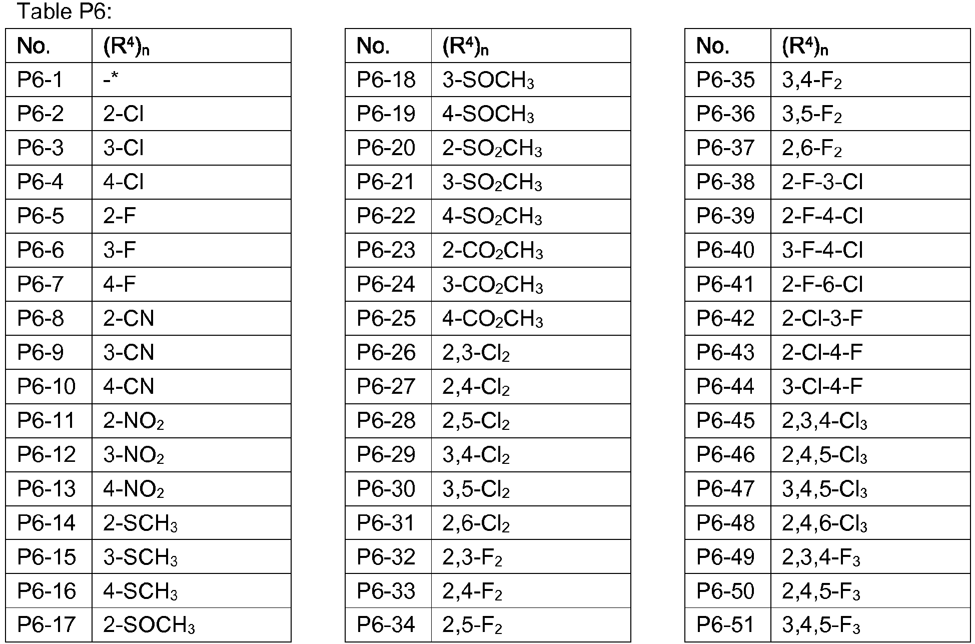

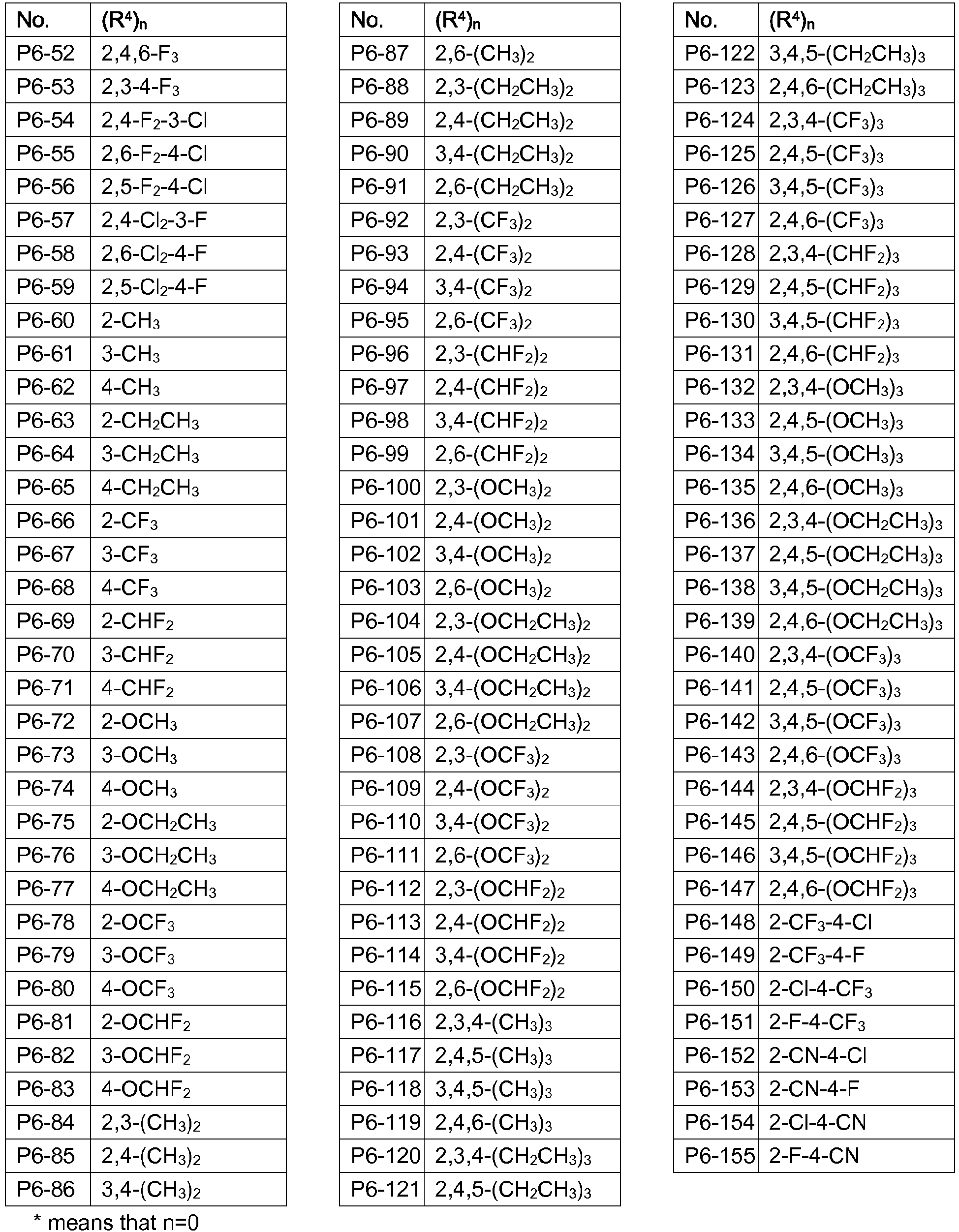

One particular embodiment relates compounds I, wherein Z-Y is Z¹-Y¹, wherein
- Y¹: is a direct bond;
- Z¹: is five or six-membered heteroaryl or phenyl, wherein the heteroaryl contains 1, 2, 3 or 4 heteroatoms selected from the group consisting of O, N and S, and wherein the heteroaryl is unsubstituted (m=0) or substituted by (R^{L})ₘ and wherein the phenyl is substituted by (R^{L})ₘ, wherein R^{L} is defined and preferably defined herein; and wherein any preferred definition for the Z applies to Z¹, where applicable, and wherein the remaining substituents are as defined and preferably defined herein. In a particular embodiment thereof, Z¹ is phenyl that is substituted by 1 or 2 R^{L} as defined and preferably defined herein, wherein one R^{L} is in 4-position.

One further particular embodiment relates compounds I, wherein Z-Y is Z²-Y², wherein
- Y²: is a direct bond or a divalent group selected from the group consisting of -O-, -S-, -NH-, - N(C₁-C₄-alkyl)-, -CR¹²R¹³-CR¹⁴R¹⁵-, -CR¹⁶=CR¹⁷-and -C≡C-; wherein R¹²,R¹³, R¹⁴,R¹⁵,R¹⁶,R¹⁷ are defined below;
- Z²: is five or six-membered heteroaryl, wherein the heteroaryl contains 1, 2, 3 or 4 heteroatoms selected from the group consisting of O, N and S, and wherein the heteroaryl is unsubstituted or substituted by 1, 2 or 3 R^{L}; wherein R^{L} is defined below; and wherein any preferred definition for the Z applies to Z², where applicable, and wherein the remaining substituents are as defined and preferably defined herein.

Another further particular embodiment relates compounds I, wherein Z-Y is Z³-Y³, that is in the meta-position in relation to the attachment of the phenyl group in formula I to the remaining backbone structure; wherein
- Y³: is a direct bond or a divalent group selected from the group consisting of -O-, -S-, -NH-, - N(C₁-C₄-alkyl)-, -CR¹²R¹³-CR¹⁴R¹⁵-, -CR¹⁶=CR¹⁷-and -C≡C-; wherein R¹²,R¹³, R¹⁴,R¹5,R¹⁶,R¹⁷ are defined below;
- Z³: is five or six-membered heteroaryl, wherein the heteroaryl contains 1, 2, 3 or 4 heteroatoms selected from the group consisting of O, N and S, and wherein the heteroaryl is unsubstituted or substituted by 1, 2 or 3 R^{L}; or phenyl that is unsubstituted or substituted by 1, 2 or 3 R^{L}; wherein R^{L} is defined below; and wherein any preferred definition for the Z applies to Z³, where applicable, and wherein the remaining substituents are as defined and preferably defined herein.

Another further particular embodiment relates compounds I, wherein Z-Y is Z⁴-Y⁴, wherein
- Y⁴: is a divalent group selected from the group consisting of -CR¹²R¹³-CR¹⁴R¹⁵-, -CR¹⁶=CR¹⁷-and -C≡C-; wherein
- R¹²,R^{13,} R¹⁴,R¹⁵,R¹⁶,R¹⁷: are independently selected from hydrogen, halogen and C₁-C₄-alkyl.
- Z⁴: is five or six-membered heteroaryl, wherein the heteroaryl contains 1, 2, 3 or 4 heteroatoms selected from the group consisting of O, N and S, and wherein the heteroaryl is unsubstituted or substituted by 1, 2 or 3 R^{L}; or phenyl that is unsubstituted or substituted by 1, 2 or 3 R^{L}; wherein
- m: is 1, 2, 3 or 4; and wherein
- R^{L}: is independently selected from halogen, CN, NO₂, OH, C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyl-C₁-C₄-alkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl and phenyl-C₁-C₄-alkyl, wherein the aliphatic, alicyclic and aromatic moieties of R^{L} are unsubstituted or substituted by one, two, three or four or up to the maximum possible number of R^{La}; wherein
- R^{La}: is independently selected from halogen, CN, NO₂, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio and C₁-C₆-haloalkylthio;
and wherein any preferred definition for the Z applies to Z⁴, where applicable, and wherein the remaining substituents are as defined and preferably defined herein.

Specific embodiment of the invention are compounds I.A1 (D=H) and I.A2 (D=SH)

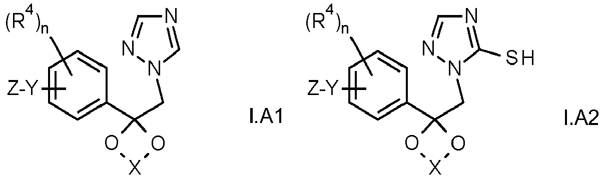

More specific embodiments are compounds I.Ab, I.Ac and I.Ad:

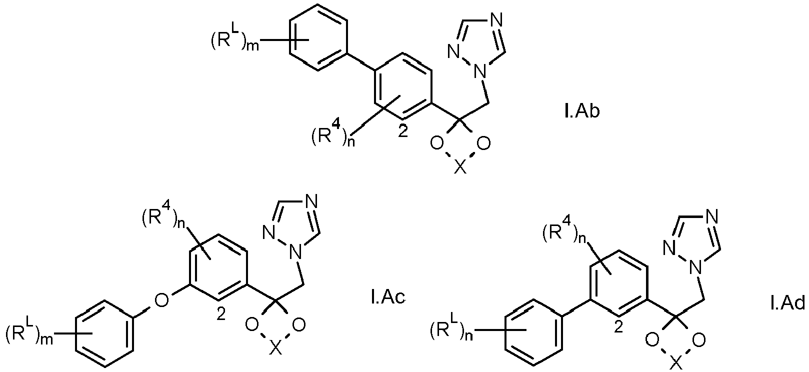

In particular with a view to their use, according to one embodiment, preference is given to the compounds of the formula I.Ab that are compiled in the Tables 1 a to 38a below. Each of the groups mentioned for a substituent in the tables is furthermore per se, independently of the combination in which it is mentioned, a particularly preferred aspect of the substituent in question.
- Table 1 a: Compounds of the formula I.Ab in which X corresponds to line Q-1 of Table Q and the meaning for the combination of (R⁴)ₙ and (R^{L})ₘ for each individual compound corresponds in each case to one line of Table D (compounds I.Ab.Q1.D1 to I.Ab.Q1.D220).
- Table 2a: Compounds of the formula I.Ab in which X corresponds to line Q-2 of Table Q and the meaning for the combination of (R4)n and (RL)m for each individual compound corresponds in each case to one line of Table D (compounds I.Ab.Q2.D1 to I.Ab.Q2.D220).
- Table 3a: Compounds of the formula I.Ab in which X corresponds to line Q-3 of Table Q and the meaning for the combination of (R4)n and (RL)m for each individual compound corresponds in each case to one line of Table D (compounds I.Ab.Q3.D1 to I.Ab.Q3.D220).
- Table 4a: Compounds of the formula I.Ab in which X corresponds to line Q-4 of Table Q and the meaning for the combination of (R4)n and (RL)m for each individual compound corresponds in each case to one line of Table D (compounds I.Ab.Q4.D1 to I.Ab.Q4.D220).
- Table 5a: Compounds of the formula I.Ab in which X corresponds to line Q-5 of Table Q and the meaning for the combination of (R4)n and (RL)m for each individual compound corresponds in each case to one line of Table D (compounds I.Ab.Q5.D1 to I.Ab.Q5.D220).
- Table 6a: Compounds of the formula I.Ab in which X corresponds to line Q-6 of Table Q and the meaning for the combination of (R4)n and (RL)m for each individual compound corresponds in each case to one line of Table D (compounds I.Ab.Q6.D1 to I.Ab.Q6.D220).
- Table 7a: Compounds of the formula I.Ab in which X corresponds to line Q-7 of Table Q and the meaning for the combination of (R4)n and (RL)m for each individual compound corresponds in each case to one line of Table D (compounds I.Ab.Q7.D1 to I.Ab.Q7.D220).
- Table 8a: Compounds of the formula I.Ab in which X corresponds to line Q-8 of Table Q and the meaning for the combination of (R4)n and (RL)m for each individual compound corresponds in each case to one line of Table D (compounds I.Ab.Q8.D1 to I.Ab.Q8.D220).
- Table 9a: Compounds of the formula I.Ab in which X corresponds to line Q-9 of Table Q and the meaning for the combination of (R4)n and (RL)m for each individual compound corresponds in each case to one line of Table D (compounds I.Ab.Q9.D1 to I.Ab.Q9.D220).
- Table: 10aCompounds of the formula I.Ab in which X corresponds to line Q-10 of Table Q and the meaning for the combination of (R⁴)ₙ and (R^{L})ₘ for each individual compound corresponds in each case to one line of Table D (compounds I.Ab.Q10.D1 to I.Ab.Q10.D220).
- Table: 11aCompounds of the formula I.Ab in which X corresponds to line Q-11 of Table Q and the meaning for the combination of (R4)n and (RL)m for each individual compound corresponds in each case to one line of Table D (compounds I.Ab.Q11.D1 to I.Ab.Q11.D220).
- Table: 12aCompounds of the formula I.Ab in which X corresponds to line Q-12 of Table Q and the meaning for the combination of (R4)n and (RL)m for each individual compound corresponds in each case to one line of Table D (compounds I.Ab.Q12.D1 to I.Ab.Q12.D220).
- Table: 13aCompounds of the formula I.Ab in which X corresponds to line Q-13 of Table Q and the meaning for the combination of (R4)n and (RL)m for each individual compound corresponds in each case to one line of Table D (compounds I.Ab.Q13.D1 to I.Ab.Q13.D220).
- Table 14a: Compounds of the formula I.Ab in which X corresponds to line Q-14 of Table Q and the meaning for the combination of (R4)n and (RL)m for each individual compound corresponds in each case to one line of Table D (compounds I.Ab.Q14.D1 to I.Ab.Q14.D220).
- Table 15a: Compounds of the formula I.Ab in which X corresponds to line Q-15 of Table Q and the meaning for the combination of (R4)n and (RL)m for each individual compound corresponds in each case to one line of Table D (compounds I.Ab.Q15.D1 to I.Ab.Q15.D220).
- Table 16a: Compounds of the formula I.Ab in which X corresponds to line Q-16 of Table Q and the meaning for the combination of (R4)n and (RL)m for each individual compound corresponds in each case to one line of Table D (compounds I.Ab.Q16.D1 to I.Ab.Q16.D220).
- Table 17a: Compounds of the formula I.Ab in which X corresponds to line Q-17 of Table Q and the meaning for the combination of (R4)n and (RL)m for each individual compound corresponds in each case to one line of Table D (compounds I.Ab.Q17.D1 to I.Ab.Q17.D220).
- Table 18a: Compounds of the formula I.Ab in which X corresponds to line Q-18 of Table Q and the meaning for the combination of (R4)n and (RL)m for each individual compound corresponds in each case to one line of Table D (compounds I.Ab.Q18.D1 to I.Ab.Q18.D220).
- Table 19a: Compounds of the formula I.Ab in which X corresponds to line Q-19 of Table Q and the meaning for the combination of (R4)n and (RL)m for each individual compound corresponds in each case to one line of Table D (compounds I.Ab.Q19.D1 to I.Ab.Q19.D220).
- Table 20a: Compounds of the formula I.Ab in which X corresponds to line Q-20 of Table Q and the meaning for the combination of (R4)n and (RL)m for each individual compound corresponds in each case to one line of Table D (compounds I.Ab.Q20.D1 to I.Ab.Q20.D220).
- Table 21a: Compounds of the formula I.Ab in which X corresponds to line Q-21 of Table Q and the meaning for the combination of (R4)n and (RL)m for each individual compound corresponds in each case to one line of Table D (compounds I.Ab.Q21.D1 to I.Ab.Q21.D220).
- Table 22a: Compounds of the formula I.Ab in which X corresponds to line Q-22 of Table Q and the meaning for the combination of (R4)n and (RL)m for each individual compound corresponds in each case to one line of Table D (compounds I.Ab.Q22.D1 to I.Ab.Q22.D220).
- Table 23a: Compounds of the formula I.Ab in which X corresponds to line Q-23 of Table Q and the meaning for the combination of (R4)n and (RL)m for each individual compound corresponds in each case to one line of Table D (compounds I.Ab.Q23.D1 to I.Ab.Q23.D220).
- Table 24a: Compounds of the formula I.Ab in which X corresponds to line Q-24 of Table Q and the meaning for the combination of (R4)n and (RL)m for each individual compound corresponds in each case to one line of Table D (compounds I.Ab.Q24.D1 to I.Ab.Q24.D220).
- Table 25a: Compounds of the formula I.Ab in which X corresponds to line Q-25 of Table Q and the meaning for the combination of (R4)n and (RL)m for each individual compound corresponds in each case to one line of Table D (compounds I.Ab.Q25.D1 to I.Ab.Q25.D220).
- Table 26a: Compounds of the formula I.Ab in which X corresponds to line Q-26 of Table Q and the meaning for the combination of (R4)n and (RL)m for each individual compound corresponds in each case to one line of Table D (compounds I.Ab.Q26.D1 to I.Ab.Q26.D220).
- Table 27a: Compounds of the formula I.Ab in which X corresponds to line Q-27 of Table Q and the meaning for the combination of (R4)n and (RL)m for each individual compound corresponds in each case to one line of Table D (compounds I.Ab.Q27.D1 to I.Ab.Q27.D220).
- Table 28a: Compounds of the formula I.Ab in which X corresponds to line Q-28 of Table Q and the meaning for the combination of (R4)n and (RL)m for each individual compound corresponds in each case to one line of Table D (compounds I.Ab.Q28.D1 to I.Ab.Q28.D220).
- Table 29a: Compounds of the formula I.Ab in which X corresponds to line Q-29 of Table Q and the meaning for the combination of (R4)n and (RL)m for each individual compound corresponds in each case to one line of Table D (compounds I.Ab.Q29.D1 to I.Ab.Q29.D220).
- Table 30a: Compounds of the formula I.Ab in which X corresponds to line Q-30 of Table Q and the meaning for the combination of (R4)n and (RL)m for each individual compound corresponds in each case to one line of Table D (compounds I.Ab.Q30.D1 to I.Ab.Q30.D220).
- Table 31a: Compounds of the formula I.Ab in which X corresponds to line Q-31 of Table Q and the meaning for the combination of (R4)n and (RL)m for each individual compound corresponds in each case to one line of Table D (compounds I.Ab.Q31.D1 to I.Ab.Q31.D220).
- Table 32a: Compounds of the formula I.Ab in which X corresponds to line Q-32 of Table Q and the meaning for the combination of (R4)n and (RL)m for each individual compound corresponds in each case to one line of Table D (compounds I.Ab.Q32.D1 to I.Ab.Q32.D220).
- Table 33a: Compounds of the formula I.Ab in which X corresponds to line Q-33 of Table Q and the meaning for the combination of (R4)n and (RL)m for each individual compound corresponds in each case to one line of Table D (compounds I.Ab.Q33.D1 to I.Ab.Q33.D220).
- Table 34a: Compounds of the formula I.Ab in which X corresponds to line Q-34 of Table Q and the meaning for the combination of (R4)n and (RL)m for each individual compound corresponds in each case to one line of Table D (compounds I.Ab.Q34.D1 to I.Ab.Q34.D220).

- Table 35a: Compounds of the formula I.Ab in which X corresponds to line Q-35 of Table Q and the meaning for the combination of (R4)n and (RL)m for each individual compound corresponds in each case to one line of Table D (compounds I.Ab.Q35.D1 to I.Ab.Q35.D220).
- Table 36a: Compounds of the formula I.Ab in which X corresponds to line Q-36 of Table Q and the meaning for the combination of (R4)n and (RL)m for each individual compound corresponds in each case to one line of Table D (compounds I.Ab.Q36.D1 to I.Ab.Q36.D220).
- Table 37a: Compounds of the formula I.Ab in which X corresponds to line Q-37 of Table Q and the meaning for the combination of (R4)n and (RL)m for each individual compound corresponds in each case to one line of Table D (compounds I.Ab.Q37.D1 to I.Ab.Q37.D220).
- Table 38a: Compounds of the formula I.Ab in which X corresponds to line Q-38 of Table Q and the meaning for the combination of (R4)n and (RL)m for each individual compound corresponds in each case to one line of Table D (compounds I.Ab.Q38.D1 to I.Ab.Q38.D220).

According to a further embodiment, preference is given to the compounds of the formula I.Ac and I.Ad that are compiled in the Tables 1 b to 38b and Tables 1 c to 38c below. Each of the groups mentioned for a substituent in the tables is furthermore per se, independently of the combination in which it is mentioned, a particularly preferred aspect of the substituent in question.
- Table 1 b: Compounds of the formula I.Ac in which X corresponds to line Q-1 of Table Q and the meaning for the combination of (R⁴)ₙ and (R^{L})ₘ for each individual compound corresponds in each case to one line of Table E (compounds I.Ac.Q1.E1 to I.Ac.Q1.E220).
- Table 2b: Compounds of the formula I.Ac in which X corresponds to line Q-2 of Table Q and the meaning for the combination of (R4)n and (RL)m for each individual compound corresponds in each case to one line of Table E (compounds I.Ac.Q2.E1 to I.Ac.Q2.E220).
- Table 3b: Compounds of the formula I.Ac in which X corresponds to line Q-3 of Table Q and the meaning for the combination of (R4)n and (RL)m for each individual compound corresponds in each case to one line of Table E (compounds I.Ac.Q3.E1 to I.Ac.Q3.E220).
- Table 4b: Compounds of the formula I.Ac in which X corresponds to line Q-4 of Table Q and the meaning for the combination of (R4)n and (RL)m for each individual compound corresponds in each case to one line of Table E (compounds I.Ac.Q4.E1 to I.Ac.Q4.E220).
- Table 5b: Compounds of the formula I.Ac in which X corresponds to line Q-5 of Table Q and the meaning for the combination of (R4)n and (RL)m for each individual compound corresponds in each case to one line of Table E (compounds I.Ac.Q5.E1 to I.Ac.Q5.E220).
- Table 6b: Compounds of the formula I.Ac in which X corresponds to line Q-6 of Table Q and the meaning for the combination of (R4)n and (RL)m for each individual compound corresponds in each case to one line of Table E (compounds I.Ac.Q6.E1 to I.Ac.Q6.E220).
- Table 7b: Compounds of the formula I.Ac in which X corresponds to line Q-7 of Table Q and the meaning for the combination of (R4)n and (RL)m for each individual compound corresponds in each case to one line of Table E (compounds I.Ac.Q7.E1 to I.Ac.Q7.E220).
- Table 8b: Compounds of the formula I.Ac in which X corresponds to line Q-8 of Table Q and the meaning for the combination of (R4)n and (RL)m for each individual compound corresponds in each case to one line of Table E (compounds I.Ac.Q8.E1 to I.Ac.Q8.E220).
- Table 9b: Compounds of the formula I.Ac in which X corresponds to line Q-9 of Table Q and the meaning for the combination of (R4)n and (RL)m for each individual compound corresponds in each case to one line of Table E (compounds I.Ac.Q9.E1 to I.Ac.Q9.E220).
- Table 10b: Compounds of the formula I.Ac in which X corresponds to line Q-10 of Table Q and the meaning for the combination of (R4)n and (RL)m for each individual compound corresponds in each case to one line of Table E (compounds I.Ac.Q10.E1 to I.Ac.Q10.E220).
- Table 11b: Compounds of the formula I.Ac in which X corresponds to line Q-11 of Table Q and the meaning for the combination of (R4)n and (RL)m for each individual compound corresponds in each case to one line of Table E (compounds I.Ac.Q11.E1 to I.Ac.Q11.E220).
- Table 12b: Compounds of the formula I.Ac in which X corresponds to line Q-12 of Table Q and the meaning for the combination of (R4)n and (RL)m for each individual compound corresponds in each case to one line of Table E (compounds I.Ac.Q12.E1 to I.Ac.Q12.E220).
- Table: 13bCompounds of the formula I.Ac in which X corresponds to line Q-13 of Table Q and the meaning for the combination of (R4)n and (RL)m for each individual compound corresponds in each case to one line of Table E (compounds I.Ac.Q13.E1 to I.Ac.Q13.E220).
- Table 14b: Compounds of the formula I.Ac in which X corresponds to line Q-14 of Table Q and the meaning for the combination of (R4)n and (RL)m for each individual compound corresponds in each case to one line of Table E (compounds I.Ac.Q14.E1 to I.Ac.Q14.E220).
- Table 15b: Compounds of the formula I.Ac in which X corresponds to line Q-15 of Table Q and the meaning for the combination of (R4)n and (RL)m for each individual compound corresponds in each case to one line of Table E (compounds I.Ac.Q15.E1 to I.Ac.Q15.E220).
- Table 16b: Compounds of the formula I.Ac in which X corresponds to line Q-16 of Table Q and the meaning for the combination of (R4)n and (RL)m for each individual compound corresponds in each case to one line of Table E (compounds I.Ac.Q16.E1 to I.Ac.Q16.E220).
- Table 17b: Compounds of the formula I.Ac in which X corresponds to line Q-17 of Table Q and the meaning for the combination of (R4)n and (RL)m for each individual compound corresponds in each case to one line of Table E (compounds I.Ac.Q17.E1 to I.Ac.Q17.E220).
- Table 18b: Compounds of the formula I.Ac in which X corresponds to line Q-18 of Table Q and the meaning for the combination of (R4)n and (RL)m for each individual compound corresponds in each case to one line of Table E (compounds I.Ac.Q18.E1 to I.Ac.Q18.E220).
- Table 19b: Compounds of the formula I.Ac in which X corresponds to line Q-19 of Table Q and the meaning for the combination of (R4)n and (RL)m for each individual compound corresponds in each case to one line of Table E (compounds I.Ac.Q19.E1 to I.Ac.Q19.E220).
- Table 20b: Compounds of the formula I.Ac in which X corresponds to line Q-20 of Table Q and the meaning for the combination of (R4)n and (RL)m for each individual compound corresponds in each case to one line of Table E (compounds I.Ac.Q20.E1 to I.Ac.Q20.E220).
- Table 21 b: compounds of the formula I.Ac in which X corresponds to line Q-21 of Table Q and the meaning for the combination of (R4)n and (RL)m for each individual compound corresponds in each case to one line of Table E (compounds I.Ac.Q21.E1 to I.Ac.Q21.E220).
- Table 22b: Compounds of the formula I.Ac in which X corresponds to line Q-22 of Table Q and the meaning for the combination of (R4)n and (RL)m for each individual compound corresponds in each case to one line of Table E (compounds I.Ac.Q22.E1 to I.Ac.Q22.E220).
- Table: 23bCompounds of the formula I.Ac in which X corresponds to line Q-23 of Table Q and the meaning for the combination of (R4)n and (RL)m for each individual compound corresponds in each case to one line of Table E (compounds I.Ac.Q23.E1 to I.Ac.Q23.E220).
- Table: 24bCompounds of the formula I.Ac in which X corresponds to line Q-24 of Table Q and the meaning for the combination of (R4)n and (RL)m for each individual compound corresponds in each case to one line of Table E (compounds I.Ac.Q24.E1 to I.Ac.Q24.E220).
- Table: 25bCompounds of the formula I.Ac in which X corresponds to line Q-25 of Table Q and the meaning for the combination of (R4)n and (RL)m for each individual compound corresponds in each case to one line of Table E (compounds I.Ac.Q25.E1 to I.Ac.Q25.E220).
- Table 26b: Compounds of the formula I.Ac in which X corresponds to line Q-26 of Table Q and the meaning for the combination of (R4)n and (RL)m for each individual compound corresponds in each case to one line of Table E (compounds I.Ac.Q26.E1 to I.Ac.Q26.E220).
- Table 27b: Compounds of the formula I.Ac in which X corresponds to line Q-27 of Table Q and the meaning for the combination of (R4)n and (RL)m for each individual compound corresponds in each case to one line of Table E (compounds I.Ac.Q27.E1 to I.Ac.Q27.E220).
- Table 28b: Compounds of the formula I.Ac in which X corresponds to line Q-28 of Table Q and the meaning for the combination of (R4)n and (RL)m for each individual compound corresponds in each case to one line of Table E (compounds I.Ac.Q28.E1 to I.Ac.Q28.E220).
- Table 29b: Compounds of the formula I.Ac in which X corresponds to line Q-29 of Table Q and the meaning for the combination of (R4)n and (RL)m for each individual compound corresponds in each case to one line of Table E (compounds I.Ac.Q29.E1 to I.Ac.Q29.E220).
- Table 30b: Compounds of the formula I.Ac in which X corresponds to line Q-30 of Table Q and the meaning for the combination of (R4)n and (RL)m for each individual compound corresponds in each case to one line of Table E (compounds I.Ac.Q30.E1 to I.Ac.Q30.E220).
- Table 31: bcompounds of the formula I.Ac in which X corresponds to line Q-31 of Table Q and the meaning for the combination of (R4)n and (RL)m for each individual compound corresponds in each case to one line of Table E (compounds I.Ac.Q31.E1 to I.Ac.Q31.E220).
- Table 32b: Compounds of the formula I.Ac in which X corresponds to line Q-32 of Table Q and the meaning for the combination of (R4)n and (RL)m for each individual compound corresponds in each case to one line of Table E (compounds I.Ac.Q32.E1 to I.Ac.Q32.E220).
- Table 33b: Compounds of the formula I.Ac in which X corresponds to line Q-33 of Table Q and the meaning for the combination of (R4)n and (RL)m for each individual compound corresponds in each case to one line of Table E (compounds I.Ac.Q33.E1 to I.Ac.Q33.E220).
- Table 34b: Compounds of the formula I.Ac in which X corresponds to line Q-34 of Table Q and the meaning for the combination of (R4)n and (RL)m for each individual compound corresponds in each case to one line of Table E (compounds I.Ac.Q34.E1 to I.Ac.Q34.E220).
- Table 35b: Compounds of the formula I.Ac in which X corresponds to line Q-35 of Table Q and the meaning for the combination of (R4)n and (RL)m for each individual compound corresponds in each case to one line of Table E (compounds I.Ac.Q35.E1 to I.Ac.Q35.E220).
- Table: 36bCompounds of the formula I.Ac in which X corresponds to line Q-36 of Table Q and the meaning for the combination of (R4)n and (RL)m for each individual compound corresponds in each case to one line of Table E (compounds I.Ac.Q36.E1 to I.Ac.Q36.E220).
- Table 37b: Compounds of the formula I.Ac in which X corresponds to line Q-37 of Table Q and the meaning for the combination of (R4)n and (RL)m for each individual compound corresponds in each case to one line of Table E (compounds I.Ac.Q37.E1 to I.Ac.Q37.E220).
- Table 38b: Compounds of the formula I.Ac in which X corresponds to line Q-38 of Table Q and the meaning for the combination of (R⁴)ₙ and (R^{L})ₘ for each individual compound corresponds in each case to one line of Table E (compounds I.Ac.Q38.E1 to I.Ac.Q38.E220).
- Table 1c: Compounds of the formula I.Ad in which X corresponds to line Q-1 of Table Q and the meaning for the combination of (R⁴)ₙ and (R^{L})ₘ for each individual compound corresponds in each case to one line of Table E (compounds I.Ad.Q1.E1 to I.Ad.Q1.E220).
- Table 2c: Compounds of the formula I.Ad in which X corresponds to line Q-2 of Table Q and the meaning for the combination of (R⁴)ₙ and (R^{L})ₘ for each individual compound corresponds in each case to one line of Table E (compounds I.Ad.Q2.E1 to I.Ad.Q2.E220).
- Table 3c: Compounds of the formula I.Ad in which X corresponds to line Q-3 of Table Q and the meaning for the combination of (R⁴)ₙ and (R^{L})ₘ for each individual compound corresponds in each case to one line of Table E (compounds I.Ad.Q3.E1 to I.Ad.Q3.E220).
- Table 4c: Compounds of the formula I.Ad in which X corresponds to line Q-4 of Table Q and the meaning for the combination of (R⁴)ₙ and (R^{L})ₘ for each individual compound corresponds in each case to one line of Table E (compounds I.Ad.Q4.E1 to I.Ad.Q4.E220).
- Table 5c: Compounds of the formula I.Ad in which X corresponds to line Q-5 of Table Q and the meaning for the combination of (R⁴)ₙ and (R^{L})ₘ for each individual compound corresponds in each case to one line of Table E (compounds I.Ad.Q5.E1 to I.Ad.Q5.E220).
- Table 6c: Compounds of the formula I.Ad in which X corresponds to line Q-6 of Table Q and the meaning for the combination of (R⁴)ₙ and (R^{L})ₘ for each individual compound corresponds in each case to one line of Table E (compounds I.Ad.Q6.E1 to I.Ad.Q6.E220).
- Table 7c: Compounds of the formula I.Ad in which X corresponds to line Q-7 of Table Q and the meaning for the combination of (R⁴)ₙ and (R^{L})ₘ for each individual compound corresponds in each case to one line of Table E (compounds I.Ad.Q7.E1 to I.Ad.Q7.E220).
- Table 8c: Compounds of the formula I.Ad in which X corresponds to line Q-8 of Table Q and the meaning for the combination of (R⁴)ₙ and (R^{L})ₘ for each individual compound corresponds in each case to one line of Table E (compounds I.Ad.Q8.E1 to I.Ad.Q8.E220).
- Table 9c: Compounds of the formula I.Ad in which X corresponds to line Q-9 of Table Q and the meaning for the combination of (R⁴)ₙ and (R^{L})ₘ for each individual compound corresponds in each case to one line of Table E (compounds I.Ad.Q9.E1 to I.Ad.Q9.E220).
- Table: 10cCompounds of the formula I.Ad in which X corresponds to line Q-10 of Table Q and the meaning for the combination of (R⁴)ₙ and (R^{L})ₘ for each individual compound corresponds in each case to one line of Table E (compounds I.Ad.Q10.E1 to I.Ad.Q10.E220).
- Table: 11 cCompounds of the formula I.Ad in which X corresponds to line Q-11 of Table Q and the meaning for the combination of (R⁴)ₙ and (R^{L})ₘ for each individual compound corresponds in each case to one line of Table E (compounds I.Ad.Q11.E1 to I.Ad.Q11.E220).
- Table: 12cCompounds of the formula I.Ad in which X corresponds to line Q-12 of Table Q and the meaning for the combination of (R⁴)ₙ and (R^{L})ₘ for each individual compound corresponds in each case to one line of Table E (compounds I.Ad.Q12.E1 to I.Ad.Q12.E220).
- Table: 13cCompounds of the formula I.Ad in which X corresponds to line Q-13 of Table Q and the meaning for the combination of (R⁴)ₙ and (R^{L})ₘ for each individual compound corresponds in each case to one line of Table E (compounds I.Ad.Q13.E1 to I.Ad.Q13.E220).
- Table 14c: Compounds of the formula I.Ad in which X corresponds to line Q-14 of Table Q and the meaning for the combination of (R⁴)ₙ and (R^{L})ₘ for each individual compound corresponds in each case to one line of Table E (compounds I.Ad.Q14.E1 to I.Ad.Q14.E220).
- Table 15c: Compounds of the formula I.Ad in which X corresponds to line Q-15 of Table Q and the meaning for the combination of (R⁴)ₙ and (R^{L})ₘ for each individual compound corresponds in each case to one line of Table E (compounds I.Ad.Q15.E1 to I.Ad.Q15.E220).
- Table 16c: Compounds of the formula I.Ad in which X corresponds to line Q-16 of Table Q and the meaning for the combination of (R⁴)ₙ and (R^{L})ₘ for each individual compound corresponds in each case to one line of Table E (compounds I.Ad.Q16.E1 to I.Ad.Q16.E220).
- Table 17c: Compounds of the formula I.Ad in which X corresponds to line Q-17 of Table Q and the meaning for the combination of (R⁴)ₙ and (R^{L})ₘ for each individual compound corresponds in each case to one line of Table E (compounds I.Ad.Q17.E1 to I.Ad.Q17.E220).
- Table 18c: Compounds of the formula I.Ad in which X corresponds to line Q-18 of Table Q and the meaning for the combination of (R⁴)ₙ and (R^{L})ₘ for each individual compound corresponds in each case to one line of Table E (compounds I.Ad.Q18.E1 to I.Ad.Q18.E220).
- Table 19c: Compounds of the formula I.Ad in which X corresponds to line Q-19 of Table Q and the meaning for the combination of (R⁴)ₙ and (R^{L})ₘ for each individual compound corresponds in each case to one line of Table E (compounds I.Ad.Q19.E1 to I.Ad.Q19.E220).
- Table 20c: Compounds of the formula I.Ad in which X corresponds to line Q-20 of Table Q and the meaning for the combination of (R⁴)ₙ and (R^{L})ₘ for each individual compound corresponds in each case to one line of Table E (compounds I.Ad.Q20.E1 to I.Ad.Q20.E220).
- Table 21c: Compounds of the formula I.Ad in which X corresponds to line Q-21 of Table Q and the meaning for the combination of (R⁴)ₙ and (R^{L})ₘ for each individual compound corresponds in each case to one line of Table E (compounds I.Ad.Q21.E1 to I.Ad.Q21.E220).
- Table 22c: Compounds of the formula I.Ad in which X corresponds to line Q-22 of Table Q and the meaning for the combination of (R⁴)ₙ and (R^{L})ₘ for each individual compound corresponds in each case to one line of Table E (compounds I.Ad.Q22.E1 to I.Ad.Q22.E220).
- Table 23c: Compounds of the formula I.Ad in which X corresponds to line Q-23 of Table Q and the meaning for the combination of (R⁴)ₙ and (R^{L})ₘ for each individual compound corresponds in each case to one line of Table E (compounds I.Ad.Q23.E1 to I.Ad.Q23.E220).
- Table 24c: Compounds of the formula I.Ad in which X corresponds to line Q-24 of Table Q and the meaning for the combination of (R⁴)ₙ and (R^{L})ₘ for each individual compound corresponds in each case to one line of Table E (compounds I.Ad.Q24.E1 to I.Ad.Q24.E220).
- Table 25c: Compounds of the formula I.Ad in which X corresponds to line Q-25 of Table Q and the meaning for the combination of (R⁴)ₙ and (R^{L})ₘ for each individual compound corresponds in each case to one line of Table E (compounds I.Ad.Q25.E1 to I.Ad.Q25.E220).
- Table 26c: Compounds of the formula I.Ad in which X corresponds to line Q-26 of Table Q and the meaning for the combination of (R⁴)ₙ and (R^{L})ₘ for each individual compound corresponds in each case to one line of Table E (compounds I.Ad.Q26.E1 to I.Ad.Q26.E220).
- Table 27c: Compounds of the formula I.Ad in which X corresponds to line Q-27 of Table Q and the meaning for the combination of (R⁴)ₙ and (R^{L})ₘ for each individual compound corresponds in each case to one line of Table E (compounds I.Ad.Q27.E1 to I.Ad.Q27.E220).
- Table 28c: Compounds of the formula I.Ad in which X corresponds to line Q-28 of Table Q and the meaning for the combination of (R⁴)ₙ and (R^{L})ₘ for each individual compound corresponds in each case to one line of Table E (compounds I.Ad.Q28.E1 to I.Ad.Q28.E220).
- Table 29c: Compounds of the formula I.Ad in which X corresponds to line Q-29 of Table Q and the meaning for the combination of (R⁴)ₙ and (R^{L})ₘ for each individual compound corresponds in each case to one line of Table E (compounds I.Ad.Q29.E1 to I.Ad.Q29.E220).
- Table 30c: Compounds of the formula I.Ad in which X corresponds to line Q-30 of Table Q and the meaning for the combination of (R⁴)ₙ and (R^{L})ₘ for each individual compound corresponds in each case to one line of Table E (compounds I.Ad.Q30.E1 to I.Ad.Q30.E220).
- Table 31 c: Compounds of the formula I.Ad in which X corresponds to line Q-31 of Table Q and the meaning for the combination of (R⁴)ₙ and (R^{L})ₘ for each individual compound corresponds in each case to one line of Table E (compounds I.Ad.Q31.E1 to I.Ad.Q31.E220).
- Table 32c: Compounds of the formula I.Ad in which X corresponds to line Q-32 of Table Q and the meaning for the combination of (R⁴)ₙ and (R^{L})ₘ for each individual compound corresponds in each case to one line of Table E (compounds I.Ad.Q32.E1 to I.Ad.Q32.E220).
- Table 33c: Compounds of the formula I.Ad in which X corresponds to line Q-33 of Table Q and the meaning for the combination of (R⁴)ₙ and (R^{L})ₘ for each individual compound corresponds in each case to one line of Table E (compounds I.Ad.Q33.E1 to I.Ad.Q33.E220).
- Table 34c: Compounds of the formula I.Ad in which X corresponds to line Q-34 of Table Q and the meaning for the combination of (R⁴)ₙ and (R^{L})ₘ for each individual compound corresponds in each case to one line of Table E (compounds I.Ad.Q34.E1 to I.Ad.Q34.E220).
- Table 35c: Compounds of the formula I.Ad in which X corresponds to line Q-35 of Table Q and the meaning for the combination of (R⁴)ₙ and (R^{L})ₘ for each individual compound corresponds in each case to one line of Table E (compounds I.Ad.Q35.E1 to I.Ad.Q35.E220).
- Table 36c: Compounds of the formula I.Ad in which X corresponds to line Q-36 of Table Q and the meaning for the combination of (R⁴)ₙ and (R^{L})ₘ for each individual compound corresponds in each case to one line of Table E (compounds I.Ad.Q36.E1 to I.Ad.Q36.E220).
- Table 37c: Compounds of the formula I.Ad in which X corresponds to line Q-37 of Table Q and the meaning for the combination of (R⁴)ₙ and (R^{L})ₘ for each individual compound corresponds in each case to one line of Table E (compounds I.Ad.Q37.E1 to I.Ad.Q37.E220).
- Table 38c: Compounds of the formula I.Ad in which X corresponds to line Q-38 of Table Q and the meaning for the combination of (R⁴)ₙ and (R^{L})ₘ for each individual compound corresponds in each case to one line of Table E (compounds I.Ad.Q38.E1 to I.Ad.Q38.E220).

**Table Q:**

| **line** | | **X** | | **line** | | **X** |
|---|---|---|---|---|---|---|
| Q-1 | | -CH₂-CH₂- | | Q-20 | | -CH₂CH₂CH₂- |
| Q-2 | | -CH(CH₃)-CH₂- | | Q-21 | | -CH₂CH(CH₃)CH₂- |
| Q-3 | | -C(CH₃)₂-CH₂- | | Q-22 | | -CH₂C(CH₃)₂CH₂- |
| Q-4 | | -CH(CH₃)-CH(CH₃)- | | Q-23 | | -CH(CH₃)CH₂CH₂- |
| Q-5 | | -C(CH₃)₂-C(CH₃)₂- | | Q-24 | | -CH(CH₃)CH(CH₃)CH₂- |
| Q-6 | | -CH(C₂H₅)-CH₂- | | Q-25 | -C(CH₃)₂CH₂CH₂- | |
| Q-7 | | -CH(C₂H₅)-CH(C₂H₅)- | | Q-26 | | -CH(CH₃)CH₂CH(CH₃)- |
| Q-8 | -CH(n-C₃H₇)-CH₂- | | | Q-27 | | -CH(C₂H₅)CH₂CH₂- |
| Q-9 | -CH(n-C₃H₇)-CH(n-C₃H₇)- | | | Q-28 | -CH₂CH(C₂H₅)CH₂- | |
| Q-10 | | -CH(i-C₃H₇)-CH₂- | | Q-29 | | -CH(C₂H₅)CH₂CH(C₂H₅)- |
| Q-11 | | -CH(i-C₃H₇)-CH(i-C₃H₇)- | | Q-30 | -CH(CH₃)CH(C₂H₅)CH₂- | |
| Q-12 | | -CH(CH₃)-CH(C₂H₅)- | | Q-31 | | -CH(CH₃)CH₂CH(C₂H₅)- |
| Q-13 | | -CH(CH₃)-CH(n-C₃H₇)- | | Q-32 | | -CH₂CH(n-C₃H₇)CH₂- |
| Q-14 | | -CHF-CH₂- | | Q-33 | | -CH₂CH(C₆H₅)CH₂- |
| Q-15 | | -CHF-CHF- | | Q-34 | | -CH₂CHFCH₂- |
| Q-16 | | -CHCl-CH₂- | | Q-35 | | -CH₂CH(CF₃)CH₂- |
| Q-17 | | -CH(C₆H₅)-CH₂- | | Q-36 | | -CH₂CHClCH₂- |
| Q-18 | | -CH(CF₃)-CH₂- | | Q-37 | | -CHClCH₂CHCl- |
| Q-19 | | -CH(CH₃)-CH(i-C₃H₇)- | | Q-38 | | -CHClCH₂CH₂- |

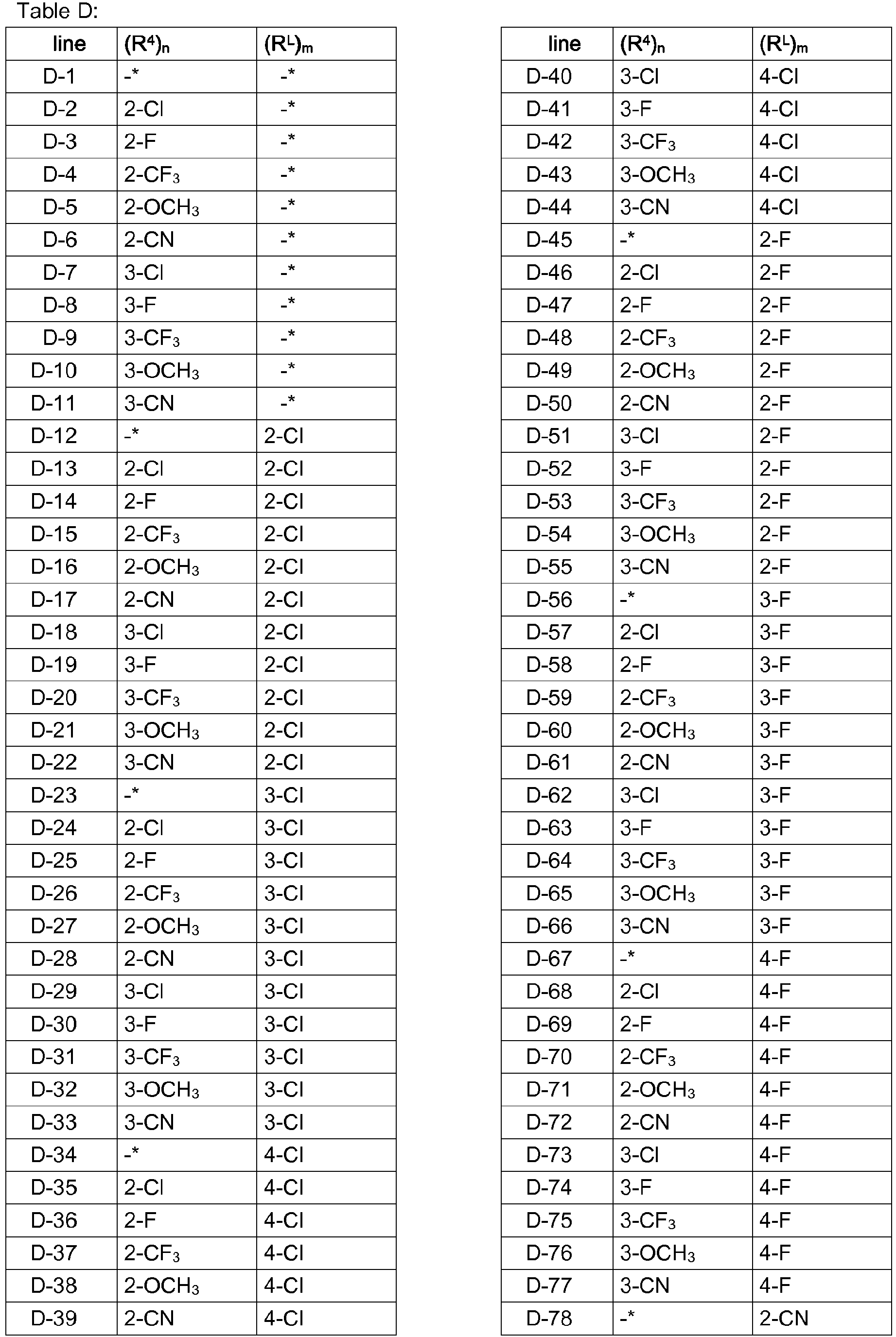

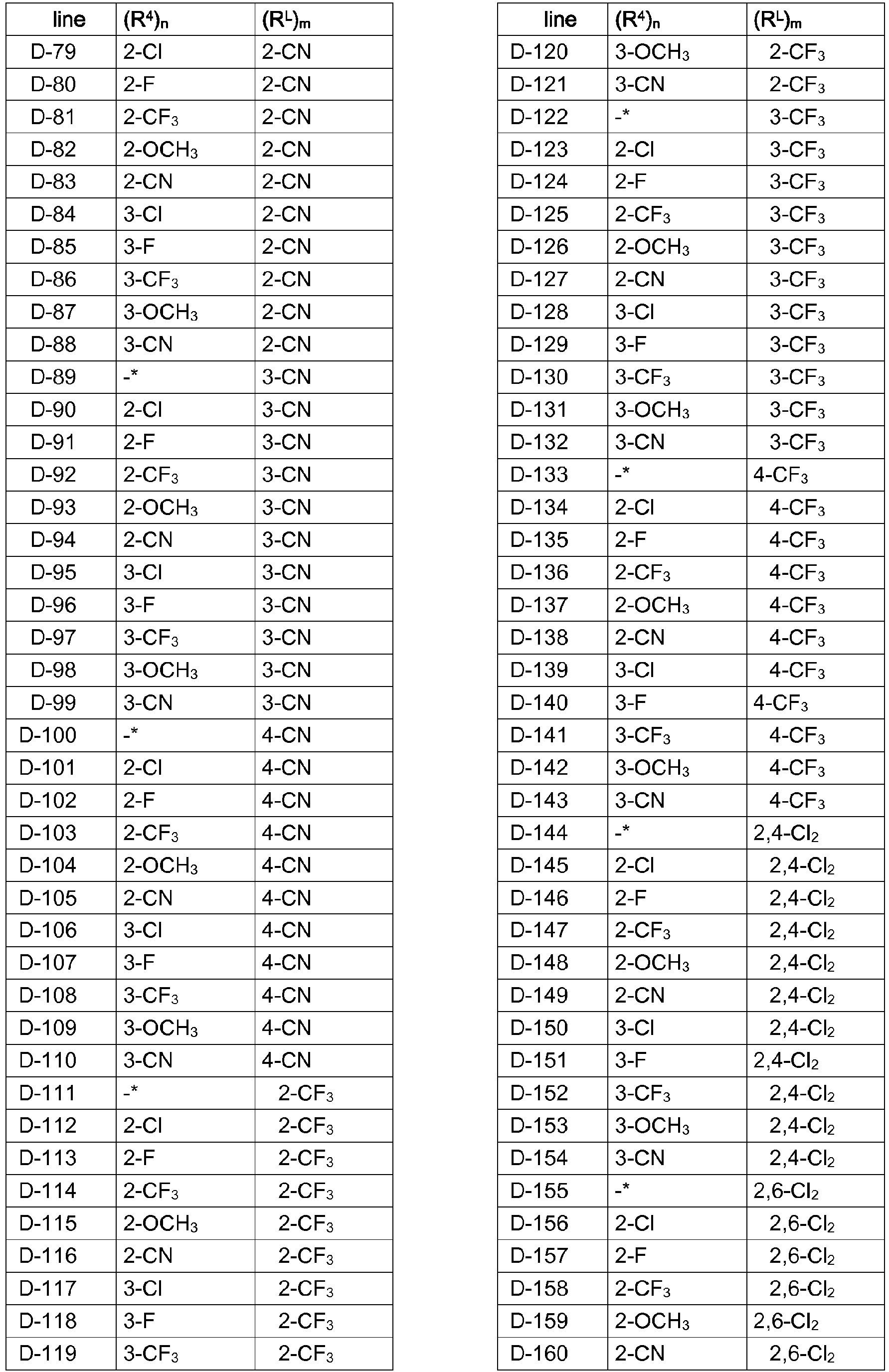

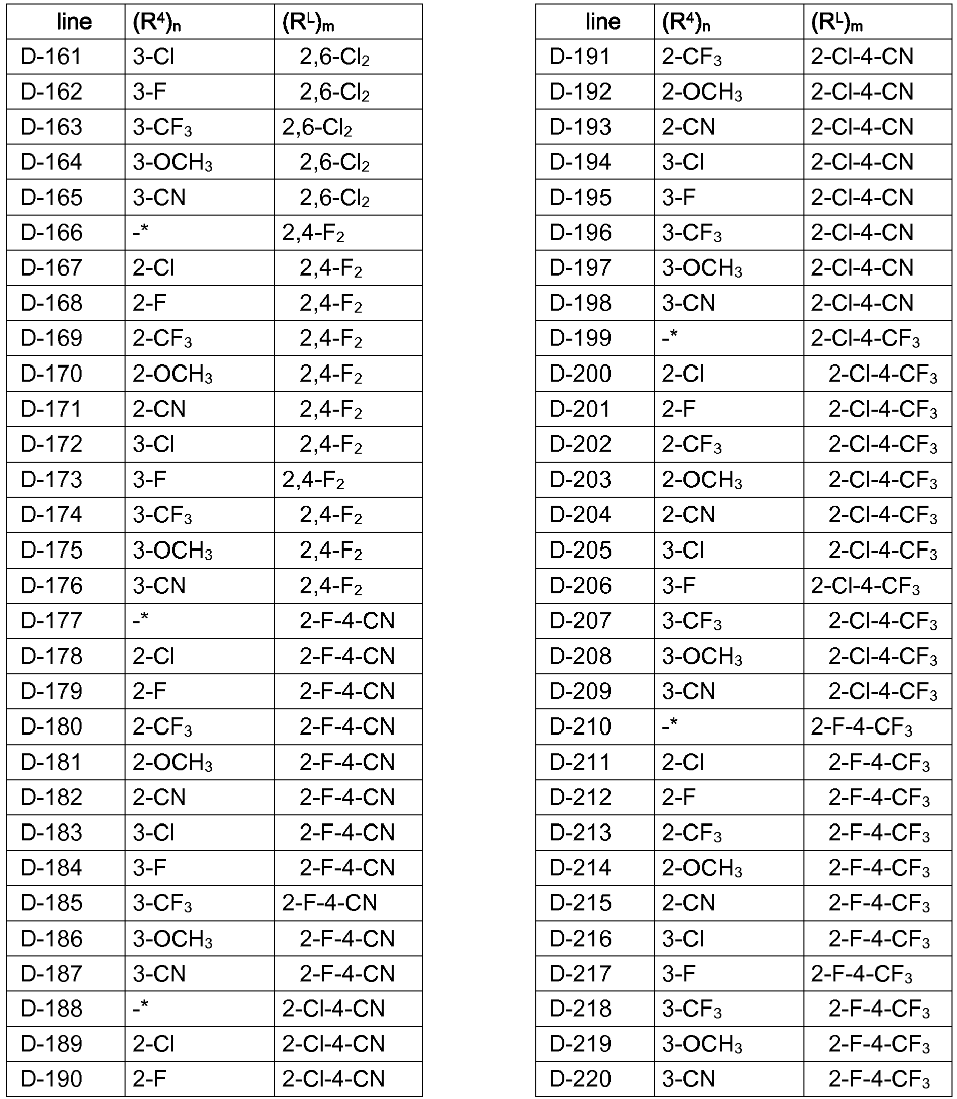

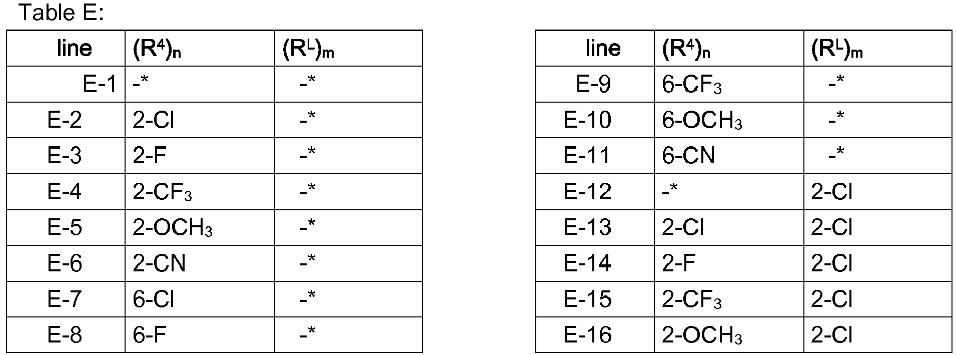

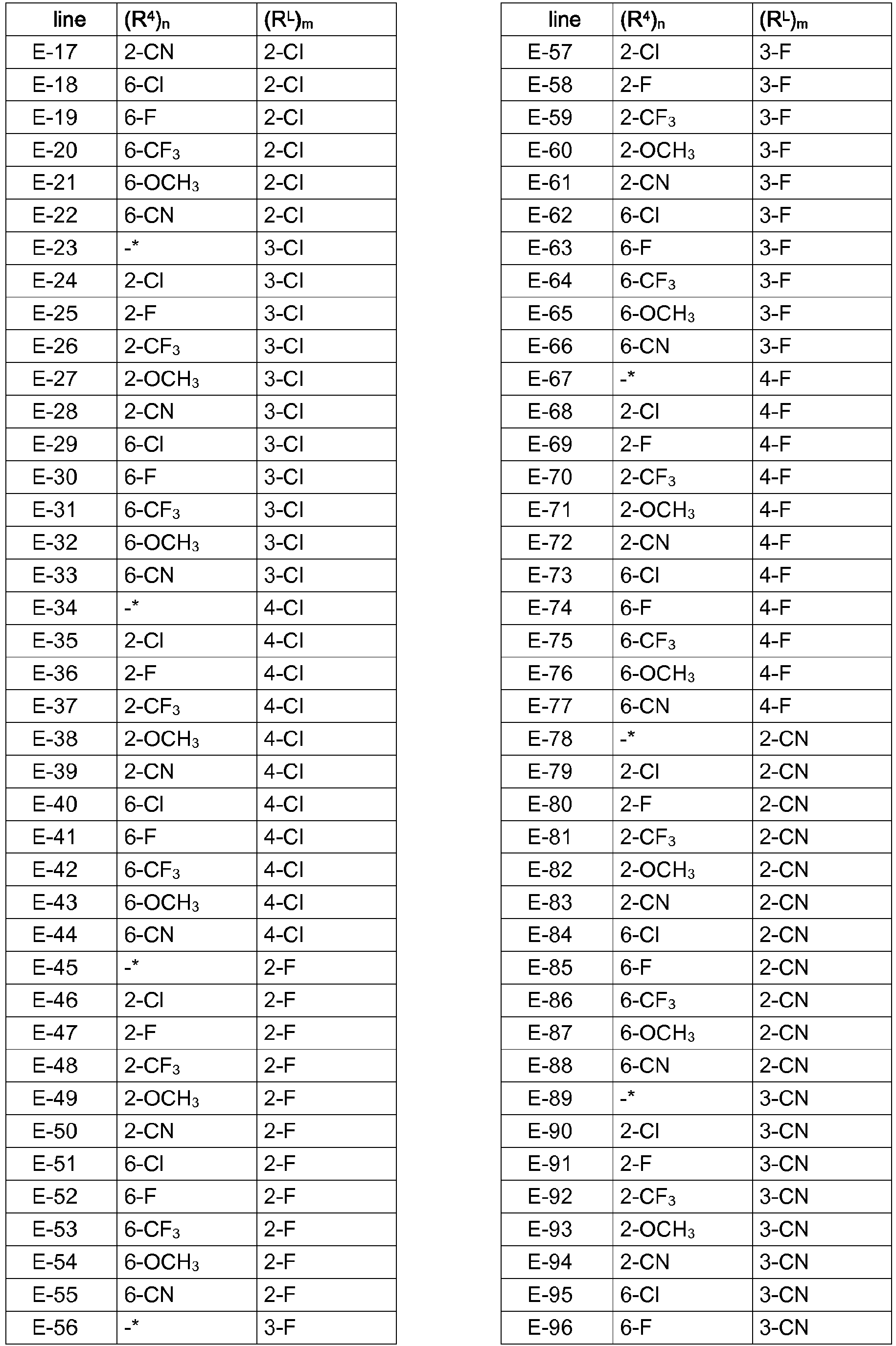

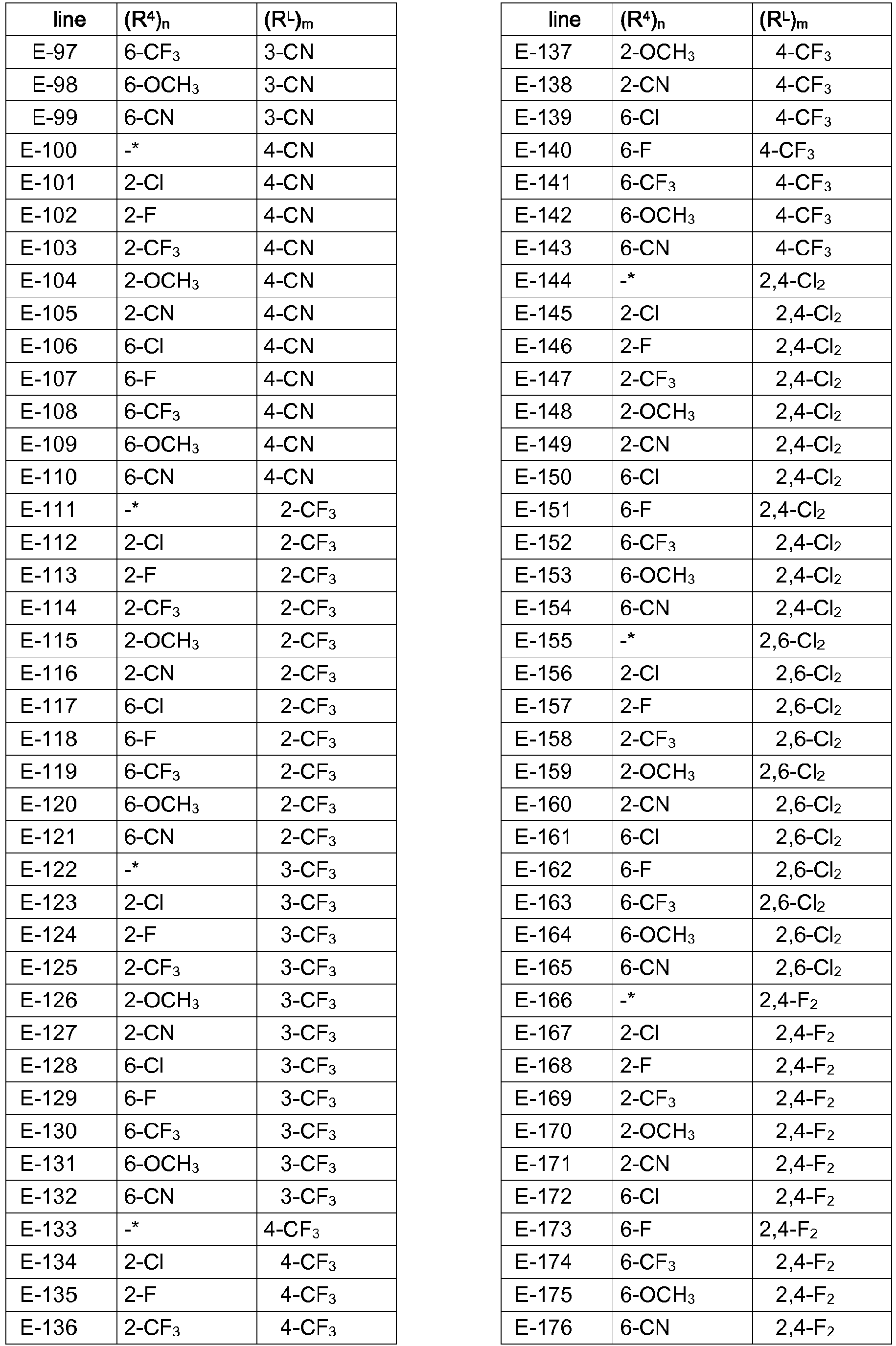

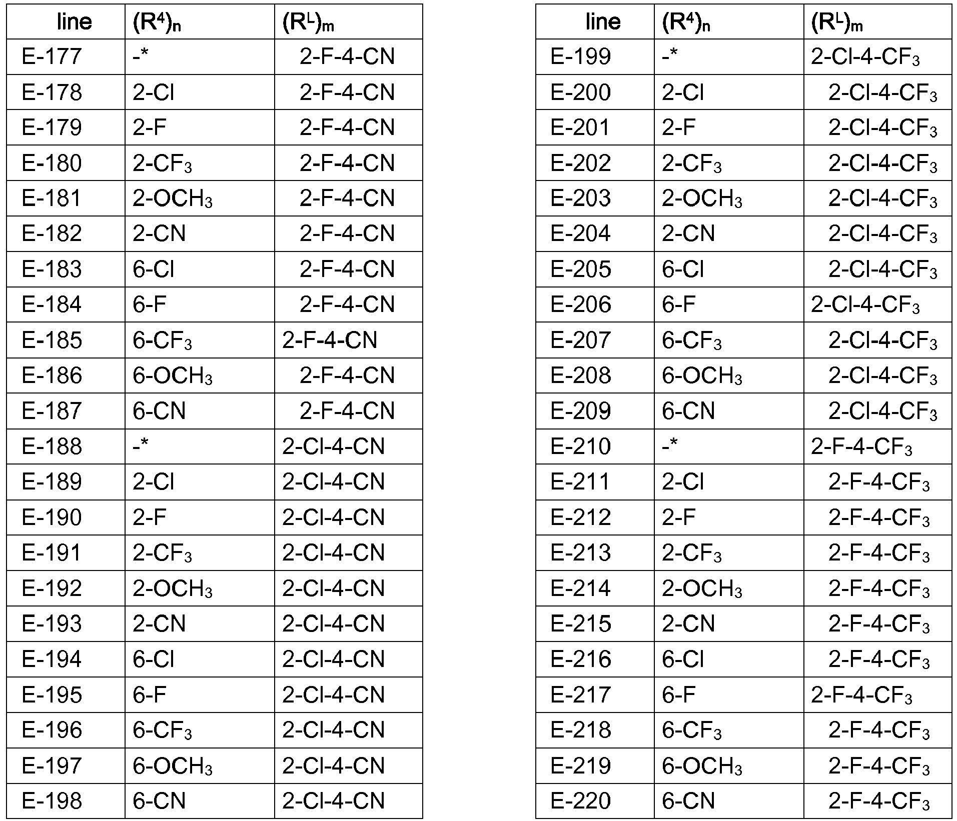

The compounds I and the compositions according to the invention, respectively, are suitable as fungicides.

Consequently, according to a further aspect, the present invention relates to the use of compounds of formula I, the N-oxides and the agriculturally acceptable salts thereof or of the compositions of the invention for combating phytopathogenic fungi.

Accordingly, the present invention also encompasses a method for combating harmful fungi, comprising treating the fungi or the materials, plants, the soil or seeds to be protected against fungal attack with an effective amount of at least one compound of formula I or with a composition comprising according to the invention.

They are distinguished by an outstanding effectiveness against a broad spectrum of phytopathogenic fungi, including soil-borne fungi, which derive especially from the classes of the Plasmo-diophoromycetes, Peronosporomycetes (syn. Oomycetes), Chytridiomycetes, Zygomycetes, Ascomycetes, Basidiomycetes and Deuteromycetes (syn. Fungi imperfecti). Some are systemically effective and they can be used in crop protection as foliar fungicides, fungicides for seed dressing and soil fungicides. Moreover, they are suitable for controlling harmful fungi, which inter alia occur in wood or roots of plants.

The compounds I and the compositions according to the invention are particularly important in the control of a multitude of phytopathogenic fungi on various cultivated plants, such as cereals, e. g. wheat, rye, barley, triticale, oats or rice; beet, e. g. sugar beet or fodder beet; fruits, such as pomes, stone fruits or soft fruits, e. g. apples, pears, plums, peaches, almonds, cherries, strawberries, raspberries, blackberries or gooseberries; leguminous plants, such as lentils, peas, alfalfa or soybeans; oil plants, such as rape, mustard, olives, sunflowers, coconut, cocoa beans, castor oil plants, oil palms, ground nuts or soybeans; cucurbits, such as squashes, cucumber or melons; fiber plants, such as cotton, flax, hemp or jute; citrus fruit, such as oranges, lemons, grapefruits or mandarins; vegetables, such as spinach, lettuce, asparagus, cabbages, carrots, onions, tomatoes, potatoes, cucurbits or paprika; lauraceous plants, such as avocados, cinnamon or camphor; energy and raw material plants, such as corn, soybean, rape, sugar cane or oil palm; corn; tobacco; nuts; coffee; tea; bananas; vines (table grapes and grape juice grape vines); hop; turf; sweet leaf (also called Stevia); natural rubber plants or ornamental and forestry plants, such as flowers, shrubs, broad-leaved trees or evergreens, e. g. conifers; and on the plant propagation material, such as seeds, and the crop material of these plants.

Preferably, compounds I and compositions thereof, respectively are used for controlling a multitude of fungi on field crops, such as potatoes sugar beets, tobacco, wheat, rye, barley, oats, rice, corn, cotton, soybeans, rape, legumes, sunflowers, coffee or sugar cane; fruits; vines; ornamentals; or vegetables, such as cucumbers, tomatoes, beans or squashes.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. These young plants may also be protected before transplantation by a total or partial treatment by immersion or pouring.

Preferably, treatment of plant propagation materials with compounds I and compositions thereof, respectively, is used for controlling a multitude of fungi on cereals, such as wheat, rye, barley and oats; rice, corn, cotton and soybeans.

The term "cultivated plants" is to be understood as including plants which have been modified by breeding, mutagenesis or genetic engineering including but not limiting to agricultural biotech products on the market or in development (cf. http://cera-gmc.org/, see GM crop database therein). Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s), oligo- or polypeptides e. g. by glycosylation or polymer additions such as prenylated, acetylated or farnesylated moieties or PEG moieties.

Plants that have been modified by breeding, mutagenesis or genetic engineering, e. g. have been rendered tolerant to applications of specific classes of herbicides, such as auxin herbicides such as dicamba or 2,4-D; bleacher herbicides such as hydroxylphenylpyruvate dioxy-genase (HPPD) inhibitors or phytoene desaturase (PDS) inhibittors; acetolactate synthase (ALS) inhibitors such as sulfonyl ureas or imidazolinones; enolpyruvylshikimate-3-phosphate synthase (EPSPS) inhibitors, such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering. Furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are e. g. described in Pest Managem. Sci. 61, 2005, 246; 61, 2005, 258; 61, 2005, 277; 61, 2005, 269; 61, 2005, 286; 64, 2008, 326; 64, 2008, 332; Weed Sci. 57, 2009, 108; Austral. J. Agricult. Res. 58, 2007, 708; Science 316, 2007, 1185; and references quoted therein. Several cultivated plants have been rendered tolerant to herbicides by conventional methods of breeding (mutagenesis), e. g. Clearfield^{®} summer rape (Canola, BASF SE, Germany) being tolerant to imidazolinones, e. g. imazamox, or ExpressSun^{®} sunflowers (DuPont, USA) being tolerant to sulfonyl ureas, e. g. tribenuron. Genetic engineering methods have been used to render cultivated plants such as soybean, cotton, corn, beets and rape, tolerant to herbicides such as glyphosate and glufosinate, some of which are commercially available under the trade names RoundupReady^{®} (glyphosate-tolerant, Monsanto, U.S.A.), Cultivance^{®} (imidazolinone tolerant, BASF SE, Germany) and LibertyLink^{®} (glufosinate-tolerant, Bayer CropScience, Germany).

Furthermore, plants are also covered that are by the use of recombinant DNA techniques capable to synthesize one or more insecticidal proteins, especially those known from the bacterial genus *Bacillus,* particularly from *Bacillus thuringiensis,* such as δ-endotoxins, e. g. CrylA(b), CryIA(c), CryIF, CryIF(a2), CryIIA(b), CryIIIA, CryIIIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278,
WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of athropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard^{®} (corn cultivars producing the Cry1Ab toxin), YieldGard^{®} Plus (corn cultivars producing Cry1Ab and Cry3Bb1 toxins), Starlink^{®} (corn cultivars producing the Cry9c toxin), Herculex^{®} RW (corn cultivars producing Cry34Ab1, Cry35Ab1 and the enzyme Phosphinothricin-N-Acetyltransferase [PAT]); NuCOTN^{®} 33B (cotton cultivars producing the Cry1Ac toxin), Bollgard^{®} I (cotton cultivars producing the Cry1Ac toxin), Bollgard^{®} II (cotton cultivars producing Cry1Ac and Cry2Ab2 toxins); VIPCOT^{®} (cotton cultivars producing a VIP-toxin); NewLeaf^{®} (potato cultivars producing the Cry3A toxin); Bt-Xtra^{®}, NatureGard^{®}, KnockOut^{®}, BiteGard^{®}, Protecta^{®}, Bt11 (e. g. Agrisure^{®} CB) and Bt176 from Syngenta Seeds SAS, France, (corn cultivars producing the Cry1Ab toxin and PAT enyzme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1 F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e. g. EP-A 392 225), plant disease resistance genes (e. g. potato cultivars, which express resistance genes acting against *Phytophthora infestans* derived from the mexican wild potato *Solanum bulbocastanum*) or T4-lysozym (e. g. potato cultivars capable of synthesizing these proteins with increased resistance against bacteria such as *Erwinia amylvora*). The methods for producing such genetically modified plants are generally known to the person skilled in the art and are described, e. g. in the publications mentioned above.

Furthermore, plants are also covered that are by the use of recombinant DNA techniques capable to synthesize one or more proteins to increase the productivity (e. g. bio mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of substances of content or new substances of content, specifically to improve human or animal nutrition, e. g. oil crops that produce health-promoting long-chain omega-3 fatty acids or unsaturated omega-9 fatty acids (e. g. Nexera^{®} rape, DOW Agro Sciences, Canada).

Furthermore, plants are also covered that contain by the use of recombinant DNA techniques a modified amount of substances of content or new substances of content, specifically to improve raw material production, e. g. potatoes that produce increased amounts of amylopectin (e. g. Amflora^{®} potato, BASF SE, Germany).

The compounds I and compositions thereof, respectively, are particularly suitable for controlling the following plant diseases:
*Albugo* spp. (white rust) on ornamentals, vegetables (e. g. *A. candida*) and sunflowers (e. g. *A. tragopogonis*); *Alternaria* spp. (Alternaria leaf spot) on vegetables, rape (*A. brassicola* or *brassicae*), sugar beets (*A. tenuis*), fruits, rice, soybeans, potatoes (e. g. *A. solani* or *A. alternata*), tomatoes (e. g. *A. solani* or *A. alternata*) and wheat; *Aphanomyces* spp. on sugar beets and vegetables; *Ascochyta* spp. on cereals and vegetables, e. g. *A. tritici* (anthracnose) on wheat and *A. hordei on* barley; *Bipolaris* and *Drechslera* spp. (teleomorph: *Cochliobolus* spp.), e. g. Southern leaf blight *(D. maydis)* or Northern leaf blight *(B. zeicola)* on corn, e. g. spot blotch (*B. sorokiniana*) on cereals and e.g. *B. oryzae* on rice and turfs; *Blumeria* (formerly *Erysiphe*) *graminis* (powdery mildew) on cereals (e. g. on wheat or barley); *Botrytis cinerea* (teleomorph: *Botryotinia fuckeliana:* grey mold) on fruits and berries (e. g. strawberries), vegetables (e. g. lettuce, carrots, celery and cabbages), rape, flowers, vines, forestry plants and wheat; *Bremia lactucae* (downy mildew) on lettuce; *Ceratocystis* (syn. *Ophiostoma*) spp. (rot or wilt) on broad-leaved trees and evergreens, e. g. *C. ulmi* (*Dutch* elm disease) on elms; *Cercospora* spp. (Cercospora leaf spots) on corn (e.g. Gray leaf spot: *C. zeae-maydis*), rice, sugar beets (e. g. *C. beticola*), sugar cane, vegetables, coffee, soybeans (e. g. *C. sojina* or *C. kikuchii*) and rice; *Cladosporium* spp. on tomatoes (e. g. *C. fulvum:* leaf mold) and cereals, e. g. *C. herbarum* (black ear) on wheat; *Claviceps purpurea* (ergot) on cereals; *Cochliobolus* (anamorph: *Helminthosporium* of *Bipolaris*) spp. (leaf spots) on corn *(C. carbonum),* cereals (e. g. *C. sativus,* anamorph: *B. sorokiniana)* and rice (e. g. *C. miyabeanus,* anamorph: *H. oryzae*); *Colletotrichum* (teleomorph: *Glomerella*) spp. (anthracnose) on cotton (e. g. *C. gossypii*), corn (e. g. *C. graminicola:* Anthracnose stalk rot), soft fruits, potatoes (e. g. *C. coccodes:* black dot), beans (e. g. *C. lindemuthianum*) and soybeans (e. g. *C. truncatum* or *C. gloeosporioides*); *Corticium* spp., e. g. *C. sasakii* (sheath blight) on rice; *Corynespora cassiicola* (leaf spots) on soybeans and ornamentals; *Cycloconium* spp., e. g. *C. oleaginum* on olive trees; *Cylindrocarpon* spp. (e. g. fruit tree canker or young vine decline, teleomorph: *Nectria* or *Neonectria* spp.) on fruit trees, vines (e. g. *C. liriodendri,* teleomorph: *Neonectria liriodendri:* Black Foot Disease) and ornamentals; *Dematophora* (teleomorph: *Rosellinia*) necatrix (root and stem rot) on soybeans; *Diaporthe* spp., e. g. *D. phaseolorum* (damping off) on soybeans; *Drechslera* (*syn. Helminthosporium,* teleomorph: *Pyrenophora*) spp. on corn, cereals, such as barley (e. g. *D. teres,* net blotch) and wheat (e. g. *D. tritici-repentis:* tan spot), rice and turf; Esca (dieback, apoplexy) on vines, caused by *Formitiporia (syn. Phellinus) punctata, F. mediterranea, Phaeomoniella chlamydospora* (earlier *Phaeoacremonium chlamydosporum), Phaeoacremonium aleophilum* and/or *Botryosphaeria obtusa; Elsinoe* spp. on pome fruits *(E. pyri),* soft fruits *(E. veneta:* anthracnose) and vines *(E. ampelina:* anthracnose); *Entyloma oryzae* (leaf smut) on rice; *Epicoccum* spp. (black mold) on wheat; *Erysiphe* spp. (powdery mildew) on sugar beets *(E. betae),* vegetables (e. g. *E. pisi*), such as cucurbits (e. g. *E. cichoracearum*), cabbages, rape (e. g. *E. cruciferarum*); *Eutypa lata* (Eutypa canker or dieback, anamorph: *Cytosporina lata, syn. Libertella blepharis*) on fruit trees, vines and ornamental woods; *Exserohilum (syn. Helminthosporium)* spp. on corn (e. g. *E. turcicum*); *Fusarium* (teleomorph: *Gibberella*) spp. (wilt, root or stem rot) on various plants, such as *F. graminearum* or *F. culmorum* (root rot, scab or head blight) on cereals (e. g. wheat or barley), *F. oxysporum* on tomatoes, *F. solani* on soybeans and *F. verticillioides* on corn; *Gaeumannomyces graminis* (take-all) on cereals (e. g. wheat or barley) and corn; *Gibberella* spp. on cereals (e. g. *G. zeae*) and rice (e. g. *G. fujikuroi:* Bakanae disease); *Glomerella cingulata* on vines, pome fruits and other plants and *G. gossypii* on cotton; Grainstaining complex on rice; *Guignardia bidwellii* (black rot) on vines; *Gymnosporangium* spp. on rosaceous plants and junipers, e. g. *G. sabinae* (rust) on pears; *Helminthosporium* spp. (syn. *Drechslera,* teleomorph: *Cochliobolus*) on corn, cereals and rice; *Hemileia* spp., e. g. *H. vastatrix* (coffee leaf rust) on coffee; *Isariopsis clavispora (syn. Cladosporium vitis)* on vines; *Macrophomina phaseolina (syn. phaseoli)* (root and stem rot) on soybeans and cotton; *Microdochium (syn. Fusarium) nivale* (pink snow mold) on cereals (e. g. wheat or barley); *Microsphaera diffusa* (powdery mildew) on soybeans; *Monilinia* spp., e. g. *M. laxa, M. fructicola* and *M. fructigena* (bloom and twig blight, brown rot) on stone fruits and other rosaceous plants; *Mycosphaerella* spp. on cereals, bananas, soft fruits and ground nuts, such as e. g. *M. graminicola* (anamorph: *Septoria tritici,* Septoria blotch) on wheat or *M*. *fijiensis* (black Sigatoka disease) on bananas; *Peronospora* spp. (downy mildew) on cabbage (e. g. *P. brassicae*), rape (e. g. *P. parasitica*), onions (e. g. *P. destructor*), tobacco *(P. tabacina)* and soybeans (e. g. *P. manshurica*); *Phakopsora pachyrhizi* and *P. meibomiae* (soybean rust) on soybeans; *Phialophora* spp. e. g. on vines (e. g. *P. tracheiphila* and *P. tetraspora*) and soybeans (e. g. *P. gregata:* stem rot); *Phoma lingam* (root and stem rot) on rape and cabbage and *P. betae* (root rot, leaf spot and damping-off) on sugar beets; *Phomopsis* spp. on sunflowers, vines (e. g. *P. viticola:* can and leaf spot) and soybeans (e. g. stem rot: *P. phaseoli,* teleomorph: *Diaporthe phaseolorum*); *Physoderma maydis* (brown spots) on corn; *Phytophthora* spp. (wilt, root, leaf, fruit and stem root) on various plants, such as paprika and cucurbits (e. g. *P. capsici*), soybeans (e. g. *P. megasperma, syn. P. sojae*), potatoes and tomatoes (e. g. *P. infestans:* late blight) and broad-leaved trees (e. g. *P. ramorum:* sudden oak death); *Plasmodiophora brassicae* (club root) on cabbage, rape, radish and other plants; *Plasmopara* spp., e. g. *P. viticola* (grapevine downy mildew) on vines and *P. halstedii* on sunflowers; *Podosphaera* spp. (powdery mildew) on rosaceous plants, hop, pome and soft fruits, e. g. *P*. *leucotricha* on apples; *Polymyxa* spp., e. g. on cereals, such as barley and wheat *(P. graminis)* and sugar beets *(P. betae)* and thereby transmitted viral diseases; *Pseudocercosporella herpotrichoides* (eyespot, teleomorph: *Tapesia yallundae)* on cereals, e. g. wheat or barley; *Pseudoperonospora* (downy mildew) on various plants, e. g. *P. cubensis* on cucurbits or *P*. *humili* on hop; *Pseudopezicula tracheiphila* (red fire disease or ,rotbrenner', anamorph: *Phialophora*) on vines; *Puccinia* spp. (rusts) on various plants, e. g. *P. triticina* (brown or leaf rust), *P. striiformis* (stripe or yellow rust), *P. hordei* (dwarf rust), *P. graminis* (stem or black rust) or *P*. *recondita* (brown or leaf rust) on cereals, such as e. g. wheat, barley or rye, *P. kuehnii* (orange rust) on sugar cane and *P. asparagi* on asparagus; *Pyrenophora* (anamorph: *Drechslera*) *tritici-repentis* (tan spot) on wheat or *P*. *teres* (net blotch) on barley; *Pyricularia* spp., e. g. *P. oryzae* (teleomorph: *Magnaporthe grisea,* rice blast) on rice and *P. grisea* on turf and cereals; *Pythium* spp. (damping-off) on turf, rice, corn, wheat, cotton, rape, sunflowers, soybeans, sugar beets, vegetables and various other plants (e. g. *P. ultimum* or *P*. *aphanidermatum*); *Ramularia* spp., e. g. *R. collo-cygni* (Ramularia leaf spots, Physiological leaf spots) on barley and *R. beticola* on sugar beets; *Rhizoctonia* spp. on cotton, rice, potatoes, turf, corn, rape, potatoes, sugar beets, vegetables and various other plants, e. g. *R. solani* (root and stem rot) on soybeans, *R. solani* (sheath blight) on rice or *R*. *cerealis* (Rhizoctonia spring blight) on wheat or barley; *Rhizopus stolonifer* (black mold, soft rot) on strawberries, carrots, cabbage, vines and tomatoes; *Rhynchosporium secalis* (scald) on barley, rye and triticale; *Sarocladium oryzae* and *S. attenuatum* (sheath rot) on rice; *Sclerotinia* spp. (stem rot or white mold) on vegetables and field crops, such as rape, sunflowers (e. g. *S. sclerotiorum*) and soybeans (e. g. *S. rolfsii* or *S*. *sclerotiorum*); *Septoria* spp. on various plants, e. g. *S. glycines* (brown spot) on soybeans, *S. tritici* (Septoria blotch) on wheat and S. (syn. *Stagonospora*) nodorum (Stagonospora blotch) on cereals; *Uncinula (syn. Erysiphe) necator* (powdery mildew, anamorph: *Oidium tuckeri*) on vines; Se*tospaeria* spp. (leaf blight) on corn (e. g. *S. turcicum, syn. Helminthosporium turcicum*) and turf; *Sphacelotheca* spp. (smut) on corn, (e. g. *S. reiliana:* head smut), sorghum und sugar cane; *Sphaerotheca fuliginea* (powdery mildew) on cucurbits; *Spongospora subterranea* (powdery scab) on potatoes and thereby transmitted viral diseases; *Stagonospora* spp. on cereals, e. g. *S. nodorum* (Stagonospora blotch, teleomorph: *Leptosphaeria* [*syn. Phaeosphaeria*] *nodorum*) on wheat; *Synchytrium endobioticum* on potatoes (potato wart disease); *Taphrina* spp., e. g. *T. deformans* (leaf curl disease) on peaches and *T. pruni* (plum pocket) on plums; *Thielaviopsis* spp. (black root rot) on tobacco, pome fruits, vegetables, soybeans and cotton, e. g. *T. basicola (syn. Chalara elegans); Tilletia* spp. (common bunt or stinking smut) on cereals, such as e. g. *T. tritici* (syn. *T. caries,* wheat bunt) and *T. controversa* (dwarf bunt) on wheat; *Typhula incarnata* (grey snow mold) on barley or wheat; *Urocystis* spp., e. g. *U. occulta* (stem smut) on rye; *Uromyces* spp. (rust) on vegetables, such as beans (e. g. *U. appendiculatus, syn. U. phaseoli)* and sugar beets (e. g. *U. betae*); *Ustilago* spp. (loose smut) on cereals (e. g. *U. nuda* and *U*. *avaenae*), corn (e. g. *U. maydis:* corn smut) and sugar cane; *Venturia* spp. (scab) on apples (e. g. *V. inaequalis*) and pears; and *Verticillium* spp. (wilt) on various plants, such as fruits and ornamentals, vines, soft fruits, vegetables and field crops, e. g. *V. dahliae* on strawberries, rape, potatoes and tomatoes.

The compounds I and compositions thereof, respectively, are also suitable for controlling harmful fungi in the protection of stored products or harvest and in the protection of materials. The term "protection of materials" is to be understood to denote the protection of technical and non-living materials, such as adhesives, glues, wood, paper and paperboard, textiles, leather, paint dispersions, plastics, colling lubricants, fiber or fabrics, against the infestation and destruction by harmful microorganisms, such as fungi and bacteria. As to the protection of wood and other materials, the particular attention is paid to the following harmful fungi: Ascomycetes such as *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomycetes such as *Coniophora* spp., *Coriolus* spp., *Gloeophyllum* spp., *Lentinus* spp., *Pleurotus* spp., *Poria* spp., *Serpula* spp. and *Tyromyces* spp., Deuteromycetes such as *Aspergillus* spp., *Cladosporium* spp., *Penicillium* spp., *Trichorma* spp., *Alternaria* spp., *Paecilomyces* spp. and Zygomycetes such as *Mucor spp.,* and in addition in the protection of stored products and harvest the following yeast fungi are worthy of note: *Candida* spp. and *Saccharomyces cerevisae.*

The compounds I and compositions thereof, resepectively, may be used for improving the health of a plant. The invention also relates to a method for improving plant health by treating a plant, its propagation material and/or the locus where the plant is growing or is to grow with an effective amount of compounds I and compositions thereof, respectively.

The term "plant health" is to be understood to denote a condition of the plant and/or its products which is determined by several indicators alone or in combination with each other such as yield (e. g. increased biomass and/or increased content of valuable ingredients), plant vigor (e. g. improved plant growth and/or greener leaves ("greening effect")), quality (e. g. improved content or composition of certain ingredients) and tolerance to abiotic and/or biotic stress.The above identified indicators for the health condition of a plant may be interdependent or may result from each other.

The compounds of formula I can be present in different crystal modifications whose biological activity may differ. They are likewise subject matter of the present invention.

The compounds I are employed as such or in form of compositions by treating the fungi or the plants, plant propagation materials, such as seeds, soil, surfaces, materials or rooms to be protected from fungal attack with a fungicidally effective amount of the active substances. The application can be carried out both before and after the infection of the plants, plant propagation materials, such as seeds, soil, surfaces, materials or rooms by the fungi.

Plant propagation materials may be treated with compounds I as such or a composition comprising at least one compound I prophylactically either at or before planting or transplanting. The invention also relates to compositions comprising one compound I according to the invention. In particular, such composition further comprises an auxiliary as defined below.

The term "effective amount" used denotes an amount of the composition or of the compounds I, which is sufficient for controlling harmful fungi on cultivated plants or in the protection of materials and which does not result in a substantial damage to the treated plants. Such an amount can vary in a broad range and is dependent on various factors, such as the fungal species to be controlled, the treated cultivated plant or material, the climatic conditions and the specific compound I used.

The compounds I, their N-oxides and salts can be converted into customary types of agrochemical compositions, e. g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further compositions types are defined in the "Catalogue of pesticide formulation types and international coding system", Technical Monograph No. 2, 6^{th} Ed. May 2008, CropLife International.

The compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-subsituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compound I on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for composition types and their preparation are:
i) Water-soluble concentrates (SL, LS)
   10-60 wt% of a compound I and 5-15 wt% wetting agent (e.g. alcohol alkoxylates) are dissolved in water and/or in a water-soluble solvent (e.g. alcohols) ad 100 wt%. The active substance dissolves upon dilution with water.
ii) Dispersible concentrates (DC)
   5-25 wt% of a compound I and 1-10 wt% dispersant (e. g. polyvinylpyrrolidone) are dissolved in organic solvent (e.g. cyclohexanone) ad 100 wt%. Dilution with water gives a dispersion.
iii) Emulsifiable concentrates (EC)
   15-70 wt% of a compound I and 5-10 wt% emulsifiers (e.g. calcium dodecylbenzenesulfonate and castor oil ethoxylate) are dissolved in water-insoluble organic solvent (e.g. aromatic hydrocarbon) ad 100 wt%. Dilution with water gives an emulsion.
iv) Emulsions (EW, EO, ES)
   5-40 wt% of a compound I and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into water ad 100 wt% by means of an emulsifying machine and made into a homogeneous emulsion. Dilution with water gives an emulsion.
v) Suspensions (SC, OD, FS)
   In an agitated ball mill, 20-60 wt% of a compound I are comminuted with addition of 2-10 wt% dispersants and wetting agents (e.g. sodium lignosulfonate and alcohol ethoxylate), 0.1-2 wt% thickener (e.g. xanthan gum) and water ad 100 wt% to give a fine active substance suspension. Dilution with water gives a stable suspension of the active substance. For FS type composition up to 40 wt% binder (e.g. polyvinylalcohol) is added.
vi) Water-dispersible granules and water-soluble granules (WG, SG)
   50-80 wt% of a compound I are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound I are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.
viii) Gel (GW, GF)
   In an agitated ball mill, 5-25 wt% of a compound I are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.
iv) Microemulsion (ME)
   5-20 wt% of a compound I are added to 5-30 wt% organic solvent blend (e.g. fatty acid dimethylamide and cyclohexanone), 10-25 wt% surfactant blend (e.g. alcohol ethoxylate and arylphenol ethoxylate), and water ad 100 %. This mixture is stirred for 1 h to produce spontaneously a thermodynamically stable microemulsion.
iv) Microcapsules (CS)
   An oil phase comprising 5-50 wt% of a compound I, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. diphenylmethene-4,4'-diisocyanatae) are dispersed into an aqueous solution of a protective colloid (e.g. polyvinyl alcohol). The addition of a polyamine (e.g. hexamethylenediamine) results in the formation of polyurea microcapsules. The monomers amount to 1-10 wt%. The wt% relate to the total CS composition.
ix) Dustable powders (DP, DS)
   1-10 wt% of a compound I are ground finely and mixed intimately with solid carrier (e.g. finely divided kaolin) ad 100 wt%.
x) Granules (GR, FG)
   0.5-30 wt% of a compound I is ground finely and associated with solid carrier (e.g. silicate) ad 100 wt%. Granulation is achieved by extrusion, spray-drying or fluidized bed.
xi) Ultra-low volume liquids (UL)
   1-50 wt% of a compound I are dissolved in organic solvent (e.g. aromatic hydrocarbon) ad 100 wt%.

The compositions types i) to xi) may optionally comprise further auxiliaries, such as 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and in particular between 0.5 and 75%, by weight of active substance. The active substances are employed in a purity of from 90% to 100%, preferably from 95% to 100% (according to NMR spectrum).

Solutions for seed treatment (LS), Suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The compositions in question give, after two-to-tenfold dilution, active substance concentrations of from 0.01 to 60% by weight, preferably from 0.1 to 40%, in the ready-to-use preparations. Application can be carried out before or during sowing. Methods for applying compound I and compositions thereof, respectively, on to plant propagation material, especially seeds include dressing, coating, pelleting, dusting, soaking and in-furrow application methods of the propagation material. Preferably, compound I or the compositions thereof, respectively, are applied on to the plant propagation material by a method such that germination is not induced, e. g. by seed dressing, pelleting, coating and dusting.

When employed in plant protection, the amounts of active substances applied are, depending on the kind of effect desired, from 0.001 to 2 kg per ha, preferably from 0.005 to 2 kg per ha, more preferably from 0.05 to 0.9 kg per ha, and in particular from 0.1 to 0.75 kg per ha.

In treatment of plant propagation materials such as seeds, e. g. by dusting, coating or drenching seed, amounts of active substance of from 0.1 g to 10 kg, in particular 0.1 to 1000 g, more particularly from 1 to 1000 g, specificaly from 1 to 100 g and most specificaly from 5 to 100 g, per 100 kilogram of plant propagation material (preferably seeds) are generally required.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Various types of oils, wetters, adjuvants, fertilizer, or micronutrients, and further pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition according to the invention such as parts of a kit or parts of a composition comprising two or three active ingredients, may be mixed by the user himself in a spray tank and further auxiliaries may be added, if appropriate.

Mixing the compounds I or the compositions comprising them in the use form as fungicides with other fungicides results in many cases in an expansion of the fungicidal spectrum of activity being obtained or in a prevention of fungicide resistance development. Furthermore, in many cases, synergistic effects are obtained.

The following list of active substances, in conjunction with which the compounds I can be used, is intended to illustrate the possible combinations but does not limit them:
A) Respiration inhibitors
   - Inhibitors of complex III at Qₒ site (e.g. strobilurins): azoxystrobin, coumethoxystrobin, coumoxystrobin, dimoxystrobin, enestroburin, fenaminstrobin, fenoxy-strobin/flufenoxystrobin, fluoxastrobin, kresoxim-methyl, metominostrobin, orysastrobin, picoxystrobin, pyraclostrobin, pyrametostrobin, pyraoxystrobin, trifloxystrobin, 2-[2-(2,5-dimethyl-phenoxymethyl)-phenyl]-3-methoxy-acrylic acid methyl ester and 2-(2-(3-(2,6-dichlorophenyl)-1-methyl-allylideneaminooxymethyl)-phenyl)-2-methoxyimino-N-methyl-acetamide, pyribencarb, triclopyricarb/chlorodincarb, famoxadone, fenamidone;
   - inhibitors of complex III at Qᵢ site: cyazofamid, amisulbrom, [(3S,6S,7R,8R)-8-benzyl-3-[(3-acetoxy-4-methoxy-pyridine-2-carbonyl)amino]-6-methyl-4,9-dioxo-1,5-dioxonan-7-yl] 2-methylpropanoate, [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl] 2-methylpropanoate, [(3S,6S,7R,8R)-8-benzyl-3-[(3-isobutoxycarbonyloxy-4-methoxy-pyridine-2-carbonyl)amino]-6-methyl-4,9-dioxo-1,5-dioxonan-7-yl] 2-methylpropanoate, [(3S,6S,7R,8R)-8-benzyl-3-[[3-(1,3-benzodioxol-5-ylmethoxy)-4-methoxy-pyridine-2-carbonyl]amino]-6-methyl-4,9-dioxo-1,5-dioxonan-7-yl] 2-methylpropanoate; (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate
   - inhibitors of complex II (e. g. carboxamides): benodanil, benzovindiflupyr, bixafen, boscalid, carboxin, fenfuram, fluopyram, flutolanil, fluxapyroxad, furametpyr, isopyrazam, mepronil, oxycarboxin, penflufen, penthiopyrad, sedaxane, tecloftalam, thifluzamide, N-(4'-trifluoromethylthiobiphenyl-2-yl)-3-difluoromethyl-1-methyl-1H-pyrazole-4-carboxamide, N-(2-(1,3,3-trimethyl-butyl)-phenyl)-1,3-dimethyl-5-fluoro-1H-pyrazole-4-carboxamide, 3-(difluoromethyl)-1-methyl-N-(1,1,3-trimethylindan-4-yl)pyrazole-4-carboxamide, 3-(trifluoromethyl)-1-methyl-N-(1,1,3-trimethylindan-4-yl)pyrazole-4-carboxamide, 1,3-dimethyl-N-(1,1,3-trimethylindan-4-yl)pyrazole-4-carboxamide, 3-(trifluoromethyl)-1,5-dimethyl-N-(1,1,3-trimethylindan-4-yl)pyrazole-4-carboxamide, 3-(difluoromethyl)-1,5-dimethyl-N-(1,1,3-trimethylindan-4-yl)pyrazole-4-carboxamide, 1,3,5-trimethyl-N-(1,1,3-trimethylindan-4-yl)pyrazole-4-carboxamide;
   - other respiration inhibitors (e.g. complex I, uncouplers): diflumetorim, (5,8-difluoroquinazoiin-4-yl)-{2-[2-fluoro-4-(4-trifluoromethylpyridin-2-yloxy)-phenyl]-ethyl}-amine; nitrophenyl derivates: binapacryl, dinobuton, dinocap, fluazinam; ferimzone; organometal compounds: fentin salts, such as fentin-acetate, fentin chloride or fentin hydroxide; ametoctradin; and silthiofam;
B) Sterol biosynthesis inhibitors (SBI fungicides)
   - C14 demethylase inhibitors (DMI fungicides): triazoles: azaconazole, bitertanol, bromuconazole, cyproconazole, difenoconazole, diniconazole, diniconazole-M, epoxiconazole, fenbuconazole, fluquinconazole, flusilazole, flutriafol, hexaconazole, imibenconazole, ipconazole, metconazole, myclobutanil, oxpoconazole, paclobutrazole, penconazole, propiconazole, prothioconazole, simeconazole, tebuconazole, tetraconazole, triadimefon, triadimenol, triticonazole, uniconazole, 1-[*rel*-(2*S*;3*R*)-3-(2-chlorophenyl)-2-(2,4-difluorophenyl)-oxiranylmethyl]-5-thiocyanato-1H-[1,2,4]triazole, 2-[*rel-*(2S;3*R*)-3-(2-chlorophenyl)-2-(2,4-difluorophenyl)-oxiranylmethyl]-2H-[1,2,4]triazole-3-thiol; imidazoles: imazalil, pefurazoate, prochloraz, triflumizol; pyrimidines, pyridines and piperazines: fenarimol, nuarimol, pyrifenox, triforine;
   - Delta14-reductase inhibitors: aldimorph, dodemorph, dodemorph-acetate, fenpropimorph, tridemorph, fenpropidin, piperalin, spiroxamine;
   - Inhibitors of 3-keto reductase: fenhexamid;
C) Nucleic acid synthesis inhibitors
   - phenylamides or acyl amino acid fungicides: benalaxyl, benalaxyl-M, kiralaxyl, metalaxyl, metalaxyl-M (mefenoxam), ofurace, oxadixyl;
   - others: hymexazole, octhilinone, oxolinic acid, bupirimate, 5-fluorocytosine, 5-fluoro-2-(p-tolylmethoxy)pyrimidin-4-amine, 5-fluoro-2-(4-fluorophenylmethoxy)pyrimidin-4-amine;
D) Inhibitors of cell division and cytoskeleton
   - tubulin inhibitors, such as benzimidazoles, thiophanates: benomyl, carbendazim, fuberidazole, thiabendazole, thiophanate-methyl; triazolopyrimidines: 5-chloro-7-(4-methyl-piperidin-1-yl)-6-(2,4,6-trifluorophenyl)-[1,2,4]triazolo[1,5-a]pyrimidine
   - other cell division inhibitors: diethofencarb, ethaboxam, pencycuron, fluopicolide, zoxamide, metrafenone, pyriofenone;
E) Inhibitors of amino acid and protein synthesis
   - methionine synthesis inhibitors (anilino-pyrimidines): cyprodinil, mepanipyrim, pyrimethanil;
   - protein synthesis inhibitors: blasticidin-S, kasugamycin, kasugamycin hydrochloride-hydrate, mildiomycin, streptomycin, oxytetracyclin, polyoxine, validamycin A;
F) Signal transduction inhibitors
   - MAP / histidine kinase inhibitors: fluoroimid, iprodione, procymidone, vinclozolin, fenpiclonil, fludioxonil;
   - G protein inhibitors: quinoxyfen;
G) Lipid and membrane synthesis inhibitors
   - Phospholipid biosynthesis inhibitors: edifenphos, iprobenfos, pyrazophos, isoprothiolane;
   - lipid peroxidation: dicloran, quintozene, tecnazene, tolclofos-methyl, biphenyl, chloroneb, etridiazole;
   - phospholipid biosynthesis and cell wall deposition: dimethomorph, flumorph, mandipropamid, pyrimorph, benthiavalicarb, iprovalicarb, valifenalate and N-(1-(1-(4-cyano-phenyl)-ethanesulfonyl)-but-2-yl) carbamic acid-(4-fluorophenyl) ester;
   - compounds affecting cell membrane permeability and fatty acides: propamocarb, propamocarb-hydrochlorid
   - fatty acid amide hydrolase inhibitors: 1-[4-[4-[5-(2,6-difluorophenyl)-4,5-dihydro-3-isoxazolyl]-2-thiazolyl]-1-piperidinyl]-2-[5-methyl-3-(trifluoromethyl)-1H-pyrazol-1-yl]ethanone
H) Inhibitors with Multi Site Action
   - inorganic active substances: Bordeaux mixture, copper acetate, copper hydroxide, copper oxychloride, basic copper sulfate, sulfur;
   - thio- and dithiocarbamates: ferbam, mancozeb, maneb, metam, metiram, propineb, thiram, zineb, ziram;
   - organochlorine compounds (e.g. phthalimides, sulfamides, chloronitriles): anilazine, chlorothalonil, captafol, captan, folpet, dichlofluanid, dichlorophen, hexachlorobenzene, pentachlorphenole and its salts, phthalide, tolylfluanid, N-(4-chloro-2-nitro-phenyl)-N-ethyl-4-methyl-benzenesulfonamide;
   - guanidines and others: guanidine, dodine, dodine free base, guazatine, guazatine-acetate, iminoctadine, iminoctadine-triacetate, iminoctadine-tris(albesilate), dithianon, 2,6-dimethyl-1H,5H-[1,4]dithiino[2,3-c:5,6-c']dipyrrole-1,3,5,7(2H,6H)-tetraone;
I) Cell wall synthesis inhibitors
   - inhibitors of glucan synthesis: validamycin, polyoxin B; melanin synthesis inhibitors: pyroquilon, tricyclazole, carpropamid, dicyclomet, fenoxanil;
J) Plant defence inducers
   - acibenzolar-S-methyl, probenazole, isotianil, tiadinil, prohexadione-calcium; phosphonates: fosetyl, fosetyl-aluminum, phosphorous acid and its salts;
K) Unknown mode of action
   - bronopol, chinomethionat, cyflufenamid, cymoxanil, dazomet, debacarb, diclomezine, difenzoquat, difenzoquat-methylsulfate, diphenylamin, fenpyrazamine, flumetover, flusulfamide, flutianil, methasulfocarb, nitrapyrin, nitrothal-isopropyl, oxathiapiprolin, oxin-copper, proquinazid, tebufloquin, tecloftalam, triazoxide, 2-butoxy-6-iodo-3-propylchromen-4-one, N-(cyclopropylmethoxyimino-(6-difluoro-methoxy-2,3-difluoro-phenyl)-methyl)-2-phenyl acetamide, N'-(4-(4-chloro-3-trifluoromethyl-phenoxy)-2,5-dimethyl-phenyl)-N-ethyl-N-methyl formamidine, N'-(4-(4-fluoro-3-trifluoromethyl-phenoxy)-2,5-dimethyl-phenyl)-N-ethyl-N-methyl formamidine, N'-(2-methyl-5-trifluoromethyl-4-(3-trimethylsilanyl-propoxy)-phenyl)-N-ethyl-N-methyl formamidine, N'-(5-difluoromethyl-2-methyl-4-(3-trimethylsilanyl-propoxy)-phenyl)-N-ethyl-N-methyl formamidine, methoxy-acetic acid 6-tert-butyl-8-fluoro-2,3-dimethyl-quinolin-4-yl ester, 3-[5-(4-methylphenyl)-2,3-dimethyl-isoxazolidin-3-yl]-pyridine, 3-[5-(4-chloro-phenyl)-2,3-dimethyl-isoxazolidin-3-yl]-pyridine (pyrisoxazole), N-(6-methoxy-pyridin-3-yl) cyclopropanecarboxylic acid amide, 5-chloro-1-(4,6-dimethoxypyrimidin-2-yl)-2-methyl-1 H-benzoimidazole, 2-(4-chloro-phenyl)-N-[4-(3,4-dimethoxy-phenyl)-isoxazol-5-yl]-2-prop-2-ynyloxy-acetamide;
L) Antifungal biocontrol agents, plant bioactivators: *Ampelomyces quisqualis* (e.g. AQ 10^{®} from Intrachem Bio GmbH & Co. KG, Germany), *Aspergillus flavus* (e.g. AFLAGUARD^{®} from Syngenta, CH), *Aureobasidium pullulans* (e.g. BOTECTOR^{®} from bio-ferm GmbH, Germany), *Bacillus pumilus* (e.g. NRRL Accession No. B-30087 in SONATA^{®} and BALLAD^{®} Plus from AgraQuest Inc., USA), *Bacillus subtilis* (e.g. isolate NRRL-Nr. B-21661 in RHAPSODY^{®}, SERENADE^{®} MAX and SERENADE^{®} ASO from AgraQuest Inc., USA), *Bacillus subtilis* var. *amyloliquefaciens* FZB24 (e.g. TAEGRO^{®} from Novozyme Biologicals, Inc., USA), *Candida oleophila* I-82 (e.g. ASPIRE^{®} from Ecogen Inc., USA), *Candida saitoana* (e.g. BIOCURE^{®} (in mixture with lysozyme) and BIOCOAT^{®} from Micro Flo Company, USA (BASF SE) and Arysta), Chitosan (e.g. ARMOUR-ZEN from BotriZen Ltd., NZ), *Clonostachys rosea f. catenulata,* also named *Gliocladium catenulatum* (e.g. isolate J1446:
   PRESTOP^{®} from Verdera, Finland), *Coniothyrium minitans* (e.g. CONTANS^{®} from Prophyta, Germany), *Cryphonectria parasitica* (e.g. *Endothia parasitica* from CNICM, France), *Cryptococcus albidus* (e.g. YIELD PLUS^{®} from Anchor Bio-Technologies, South Africa), *Fusarium oxysporum* (e.g. BIOFOX^{®} from S.I.A.P.A., Italy, FUSACLEAN^{®} from Natural Plant Protection, France), *Metschnikowia fructicola* (e.g. SHEMER^{®} from Agrogreen, Israel), *Microdochium dimerum* (e.g. ANTIBOT^{®} from Agrauxine, France), *Phlebiopsis gigantea* (e.g. ROTSOP^{®} from Verdera, Finland), *Pseudozyma flocculosa* (e.g. SPORODEX^{®} from Plant Products Co. Ltd., Canada), *Pythium oligandrum* DV74 (e.g. POLYVERSUM^{®} from Remeslo SSRO, Biopreparaty, Czech Rep.), *Reynoutria sachlinensis* (e.g. REGALIA^{®} from Marrone Biolnnovations, USA), *Talaromyces flavus* V117b (e.g. PROTUS^{®} from Prophyta, Germany), *Trichoderma asperellum* SKT-1 (e.g. ECO-HOPE^{®} from Kumiai Chemical Industry Co., Ltd., Japan), *T. atroviride* LC52 (e.g. SENTINEL^{®} from Agrimm Technologies Ltd, NZ), *T. harzianum* T-22 (e.g. PLANTSHIELD^{®} der Firma BioWorks Inc., USA), *T. harzianum* TH 35 (e.g. ROOT PRO^{®} from Mycontrol Ltd., Israel), *T. harzianum T-*39 (e.g. TRICHODEX^{®} and TRICHODERMA 2000^{®} from Mycontrol Ltd., Israel and Makhteshim Ltd., Israel), *T. harzianum* and *T. viride* (e.g. TRICHOPEL from Agrimm Technologies Ltd, NZ), *T. harzianum* ICC012 and *T. viride* ICC080 (e.g. REMEDIER^{®} WP from Isagro Ricerca, Italy), *T. polysporum* and *T. harzianum* (e.g. BINAB^{®} from BINAB Bio-Innovation AB, Sweden), *T. stromaticum* (e.g. TRICOVAB^{®} from C.E.P.L.A.C., Brazil), *T. virens GL-21* (e.g. SOILGARD^{®} from Certis LLC, USA), *T. viride* (e.g. TRIECO^{®} from Ecosense Labs. (India) Pvt. Ltd., Indien, BIO-CURE^{®} F from T. Stanes & Co. Ltd., Indien), *T. viride* TV1 (e.g. T. viride TV1 from Agribiotec srl, Italy), *Ulocladium oudemansii* HRU3 (e.g. BOTRY-ZEN^{®} from Botry-Zen Ltd, NZ);
M) Growth regulators
   abscisic acid, amidochlor, ancymidol, 6-benzylaminopurine, brassinolide, butralin, chlormequat (chlormequat chloride), choline chloride, cyclanilide, daminozide, dikegulac, dimethipin, 2,6-dimethylpuridine, ethephon, flumetralin, flurprimidol, fluthiacet, forchlorfenuron, gibberellic acid, inabenfide, indole-3-acetic acid , maleic hydrazide, mefluidide, mepiquat (mepiquat chloride), naphthaleneacetic acid, N-6-benzyladenine, paclobutrazol, prohexadione (prohexadione-calcium), prohydrojasmon, thidiazuron, triapenthenol, tributyl phosphorotrithioate, 2,3,5-tri-iodobenzoic acid , trinexapac-ethyl and uniconazole;
N) Herbicides
   - acetamides: acetochlor, alachlor, butachlor, dimethachlor, dimethenamid, flufenacet, mefenacet, metolachlor, metazachlor, napropamide, naproanilide, pethoxamid, pretilachlor, propachlor, thenylchlor;
   - amino acid derivatives: bilanafos, glyphosate, glufosinate, sulfosate;
   - aryloxyphenoxypropionates: clodinafop, cyhalofop-butyl, fenoxaprop, fluazifop, haloxyfop, metamifop, propaquizafop, quizalofop, quizalofop-P-tefuryl;
   - Bipyridyls: diquat, paraquat;
   - (thio)carbamates: asulam, butylate, carbetamide, desmedipham, dimepiperate, eptam (EPTC), esprocarb, molinate, orbencarb, phenmedipham, prosulfocarb, pyributicarb, thiobencarb, triallate;
   - cyclohexanediones: butroxydim, clethodim, cycloxydim, profoxydim, sethoxydim, tepraloxydim, tralkoxydim;
   - dinitroanilines: benfluralin, ethalfluralin, oryzalin, pendimethalin, prodiamine, trifluralin;
   - diphenyl ethers: acifluorfen, aclonifen, bifenox, diclofop, ethoxyfen, fomesafen, lactofen, oxyfluorfen;
   - hydroxybenzonitriles: bomoxynil, dichlobenil, ioxynil;
   - imidazolinones: imazamethabenz, imazamox, imazapic, imazapyr, imazaquin, imazethapyr;
   - phenoxy acetic acids: clomeprop, 2,4-dichlorophenoxyacetic acid (2,4-D), 2,4-DB, dichlorprop, MCPA, MCPA-thioethyl, MCPB, Mecoprop;
   - pyrazines: chloridazon, flufenpyr-ethyl, fluthiacet, norflurazon, pyridate;
   - pyridines: aminopyralid, clopyralid, diflufenican, dithiopyr, fluridone, fluroxypyr, picloram, picolinafen, thiazopyr;
   - sulfonyl ureas: amidosulfuron, azimsulfuron, bensulfuron, chlorimuron-ethyl, chlorsulfuron, cinosulfuron, cyclosulfamuron, ethoxysulfuron, flazasulfuron, flucetosulfuron, flupyrsulfuron, foramsulfuron, halosulfuron, imazosulfuron, iodosulfuron, mesosulfuron, metazosulfuron, metsulfuron-methyl, nicosulfuron, oxasulfuron, primisulfuron, prosulfuron, pyrazosulfuron, rimsulfuron, sulfometuron, sulfosulfuron, thifensulfuron, triasulfuron, tribenuron, trifloxysulfuron, triflusulfuron, tritosulfuron, 1-((2-chloro-6-propyl-imidazo[1,2-b]pyridazin-3-yl)sulfonyl)-3-(4,6-dimethoxy-pyrimidin-2-yl)urea;
   - triazines: ametryn, atrazine, cyanazine, dimethametryn, ethiozin, hexazinone, metamitron, metribuzin, prometryn, simazine, terbuthylazine, terbutryn, triaziflam;
   - ureas: chlorotoluron, daimuron, diuron, fluometuron, isoproturon, linuron, methabenzthiazuron,tebuthiuron;
   - other acetolactate synthase inhibitors: bispyribac-sodium, cloransulam-methyl, diclosulam, florasulam, flucarbazone, flumetsulam, metosulam, ortho-sulfamuron, penoxsulam, propoxycarbazone, pyribambenz-propyl, pyribenzoxim, pyriftalid, pyriminobac-methyl, pyrimisulfan, pyrithiobac, pyroxasulfone, pyroxsulam;
   - others: amicarbazone, aminotriazole, anilofos, beflubutamid, benazolin, bencarbazone,benfluresate, benzofenap, bentazone, benzobicyclon, bicyclopyrone, bromacil, bromobutide, butafenacil, butamifos, cafenstrole, carfentrazone, cinidon-ethyl, chlorthal, cinmethylin, clomazone, cumyluron, cyprosulfamide, dicamba, difenzoquat, diflufenzopyr, *Drechslera monoceras,* endothal, ethofumesate, etobenzanid, fenoxasulfone, fentrazamide, flumiclorac-pentyl, flumioxazin, flupoxam, flurochloridone, flurtamone, indanofan, isoxaben, isoxaflutole, lenacil, propanil, propyzamide, quinclorac, quinmerac, mesotrione, methyl arsonic acid, naptalam, oxadiargyl, oxadiazon, oxaziclomefone, pentoxazone, pinoxaden, pyraclonil, pyraflufen-ethyl, pyrasulfotole, pyrazoxyfen, pyrazolynate, quinoclamine, saflufenacil, sulcotrione, sulfentrazone, terbacil, tefuryltrione, tembotrione, thiencarbazone, topramezone, (3-[2-chloro-4-fluoro-5-(3-methyl-2,6-dioxo-4-trifluoromethyl-3,6-dihydro-2H-pyrimidin-1-yl)-phenoxy]-pyridin-2-yloxy)-acetic acid ethyl ester, 6-amino-5-chloro-2-cyclopropyl-pyrimidine-4-carboxylic acid methyl ester, 6-chloro-3-(2-cyclopropyl-6-methylphenoxy)-pyridazin-4-ol, 4-amino-3-chloro-6-(4-chloro-phenyl)-5-fluoro-pyridine-2-carboxylic acid, 4-amino-3-chloro-6-(4-chloro-2-fluoro-3-methoxy-phenyl)-pyridine-2-carboxylic acid methyl ester, and 4-amino-3-chloro-6-(4-chloro-3-dimethylamino-2-fluoro-phenyl)-pyridine-2-carboxylic acid methyl ester.
O) Insecticides
   - organo(thio)phosphates: acephate, azamethiphos, azinphos-methyl, chlorpyrifos, chlorpyrifos-methyl, chlorfenvinphos, diazinon, dichlorvos, dicrotophos, dimethoate, disulfoton, ethion, fenitrothion, fenthion, isoxathion, malathion, methamidophos, methidathion, methyl-parathion, mevinphos, monocrotophos, oxydemeton-methyl, paraoxon, parathion, phenthoate, phosalone, phosmet, phosphamidon, phorate, phoxim, pirimiphos-methyl, profenofos, prothiofos, sulprophos, tetrachlorvinphos, terbufos, triazophos, trichlorfon;
   - carbamates: alanycarb, aldicarb, bendiocarb, benfuracarb, carbaryl, carbofuran, carbosulfan, fenoxycarb, furathiocarb, methiocarb, methomyl, oxamyl, pirimicarb, propoxur, thiodicarb, triazamate;
   - pyrethroids: allethrin, bifenthrin, cyfluthrin, cyhalothrin, cyphenothrin, cypermethrin, alpha-cypermethrin, beta-cypermethrin, zeta-cypermethrin, deltamethrin, esfenvalerate, etofenprox, fenpropathrin, fenvalerate, imiprothrin, lambda-cyhalothrin, permethrin, prallethrin, pyrethrin I and II, resmethrin, silafluofen, tau-fluvalinate, tefluthrin, tetramethrin, tralomethrin, transfluthrin, profluthrin, dimefluthrin;
   - insect growth regulators: a) chitin synthesis inhibitors: benzoylureas: chlorfluazuron, cyramazin, diflubenzuron, flucycloxuron, flufenoxuron, hexaflumuron, lufenuron, novaluron, teflubenzuron, triflumuron; buprofezin, diofenolan, hexythiazox, etoxazole, clofentazine; b) ecdysone antagonists: halofenozide, methoxyfenozide, tebufenozide, azadirachtin; c) juvenoids: pyriproxyfen, methoprene, fenoxycarb; d) lipid biosynthesis inhibitors: spirodiclofen, spiromesifen, spirotetramat;
   - nicotinic receptor agonists/antagonists compounds: clothianidin, dinotefuran, flupyradifurone, imidacloprid, thiamethoxam, nitenpyram, acetamiprid, thiacloprid, 1-2-chloro-thiazol-5-ylmethyl)-2-nitrimino-3,5-dimethyl-[1,3,5]triazinane;
   - GABA antagonist compounds: endosulfan, ethiprole, fipronil, vaniliprole, pyrafluprole, pyriprole, 5-amino-1-(2,6-dichloro-4-methyl-phenyl)-4-sulfinamoyl-1H-pyrazole-3-carbothioic acid amide;
   - macrocyclic lactone insecticides: abamectin, emamectin, milbemectin, lepimectin, spinosad, spinetoram;
   - mitochondrial electron transport inhibitor (METI) I acaricides: fenazaquin, pyridaben, tebufenpyrad, tolfenpyrad, flufenerim;
   - METI II and III compounds: acequinocyl, fluacyprim, hydramethylnon;
   - Uncouplers: chlorfenapyr;
   - oxidative phosphorylation inhibitors: cyhexatin, diafenthiuron, fenbutatin oxide, propargite;
   - moulting disruptor compounds: cryomazine;
   - mixed function oxidase inhibitors: piperonyl butoxide;
   - sodium channel blockers: indoxacarb, metaflumizone;
   - others: benclothiaz, bifenazate, cartap, flonicamid, pyridalyl, pymetrozine, sulfur, thiocyclam, flubendiamide, chlorantraniliprole, cyazypyr (HGW86), cyenopyrafen, flupyrazofos, cyflumetofen, amidoflumet, imicyafos, bistrifluron, pyrifluquinazon and 1,1'-[(3S,4R,4aR,6S,6aS,12R,12aS,12bS)-4-[[(2-cyclopropylacetyl)oxy]methyl]-1,3,4,4a,5,6,6a,12,12a,12b-decahydro-12-hydroxy-4,6a,12b-trimethyl-11-oxo-9-(3-pyridinyl)-2H,11H-naphtho[2,1-b]pyrano[3,4-e]pyran-3,6-diyl] cyclopropaneacetic acid ester.

The present invention furthermore relates to compositions comprising a compound I (component 1) and at least one further active substance useful for plant protection, e. g. selected from the groups A) to O) (component 2), in particular one further fungicide, e. g. fungicide from the groups A) to L), as described above, and if desired one suitable solvent or solid carrier. Those compositions are of particular interest, since many of them at the same application rate show higher efficiencies against harmful fungi. Furthermore, combating harmful fungi with a compristion comprising a compound I and a fungicide from groups A) to L), as described above, is more efficient than combating those fungi with individual compounds I or individual fungicides from groups A) to L). By applying compounds I together with at least one active substance from groups A) to O) a synergistic effect can be obtained, i.e. more then simple addition of the individual effects is obtained (synergistic compositions).

This can be obtained by applying the compounds I and at least one further active substance simultaneously, either jointly (e. g. as tank-mix) or seperately, or in succession, wherein the time interval between the individual applications is selected to ensure that the active substance applied first still occurs at the site of action in a sufficient amount at the time of application of the further active substance(s). The order of application is not essential for working of the present invention.

In compositions according to the invention comprising one compound I (component 1) and one further active substance (component 2), e. g. one active substance from groups A) to O), the weight ratio of component 1 and component 2 generally depends from the properties of the active substances used, usually it is in the range of from 1:100 to 100:1, regularly in the range of from 1:50 to 50:1, preferably in the range of from 1:20 to 20:1, more preferably in the range of from 1:10 to 10:1 1 and in particular in the range of from 1:3 to 3:1.

In compositions according to the invention comprising one compound I (component 1) and a first further active substance (component 2) and a second further active substance (component 3), e. g. two active substances from groups A) to O), the weight ratio of component 1 and component 2 depends from the properties of the active substances used, preferably it is in the range of from 1:50 to 50:1 and particularly in the range of from 1:10 to 10:1, and the weight ratio of component 1 and component 3 preferably is in the range of from 1:50 to 50:1 and particularly in the range of from 1:10 to 10:1.

Preference is also given to compositions comprising a compound I (component 1) and at least one active substance selected from group A) (component 2) and particularly selected from azoxystrobin, dimoxystrobin, fluoxastrobin, kresoxim-methyl, orysastrobin, picoxystrobin, pyraclostrobin, trifloxystrobin; famoxadone, fenamidone; benzovindiflupyr, bixafen, boscalid, fluopyram, fluxapyroxad, isopyrazam, penflufen, penthiopyrad, sedaxane; ametoctradin, cyazofamid, fluazinam, fentin salts, such as fentin acetate.

Preference is given to compositions comprising a compound of formula I (component 1) and at least one active substance selected from group B) (component 2) and particularly selected from cyproconazole, difenoconazole, epoxiconazole, fluquinconazole, flusilazole, flutriafol, metconazole, myclobutanil, penconazole, propiconazole, prothioconazole, triadimefon, triadimenol, tebuconazole, tetraconazole, triticonazole, prochloraz, fenarimol, triforine; dodemorph, fenpropimorph, tridemorph, fenpropidin, spiroxamine; fenhexamid.

Preference is given to compositions comprising a compound of formula I (component 1) and at least one active substance selected from group C) (component 2) and particularly selected from metalaxyl, (metalaxyl-M) mefenoxam, ofurace.

Preference is given to compositions comprising a compound of formula I (component 1) and at least one active substance selected from group D) (component 2) and particularly selected from benomyl, carbendazim, thiophanate-methyl, ethaboxam, fluopicolide, zoxamide, metrafenone, pyriofenone.

Preference is also given to compositions comprising a compound I (component 1) and at least one active substance selected from group E) (component 2) and particularly selected from cyprodinil, mepanipyrim, pyrimethanil.

Preference is also given to compositions comprising a compound I (component 1) and at least one active substance selected from group F) (component 2) and particularly selected from iprodione, fludioxonil, vinclozolin, quinoxyfen.

Preference is also given to compositions comprising a compound I (component 1) and at least one active substance selected from group G) (component 2) and particularly selected from dimethomorph, flumorph, iprovalicarb, benthiavalicarb, mandipropamid, propamocarb.

Preference is also given to compositions comprising a compound I (component 1) and at least one active substance selected from group H) (component 2) and particularly selected from copper acetate, copper hydroxide, copper oxychloride, copper sulfate, sulfur, mancozeb, metiram, propineb, thiram, captafol, folpet, chlorothalonil, dichlofluanid, dithianon.

Preference is also given to compositions comprising a compound I (component 1) and at least one active substance selected from group I) (component 2) and particularly selected from carpropamid and fenoxanil.

Preference is also given to compositions comprising a compound I (component 1) and at least one active substance selected from group J) (component 2) and particularly selected from acibenzolar-S-methyl, probenazole, tiadinil, fosetyl, fosetyl-aluminium, H₃PO₃ and salts thereof.

Preference is also given to compositions comprising a compound I (component 1) and at least one active substance selected from group K) (component 2) and particularly selected from cymoxanil, proquinazid and *N*-methyl-2-{1-[(5-methyl-3-trifluoromethyl-1H-pyrazol-1-yl)-acetyl]-piperidin-4-yl}-*N*-[(1R)-1,2,3,4-tetrahydronaphthalen-1-yl]-4-thiazoiecarboxamide.

Preference is also given to compositions comprising a compound I (component 1) and at least one active substance selected from group L) (component 2) and particularly selected from *Bacillus subtilis* strain NRRL No. B-21661, *Bacillus pumilus* strain NRRL No. B-30087 and *Ulocladium oudemansii.*

Accordingly, the present invention furthermore relates to compositions comprising one compound I (component 1) and one further active substance (component 2), which further active substance is selected from the column "Component 2" of the lines C-1 to C-370 of Table C.

A further embodiment relates to the compositions C-1 to C-370 listed in Table C, wherein one row of Table C corresponds in each case to a composition comprising one of the compounds I that are individualized compounds of formula I (component 1) and the respective further active substance from groups A) to O) (component 2) stated in the respective row. According to a preferred embodiment, the "individualized compound I" is one of the compounds as individualized in Tables 1 a to 38a, Tables 1 b to 38b and Tables 1 c to 38c. Preferably, the compositions described comprise the active substances in synergistically effective amounts.

**Table C:**

| Composition comprising one individualized compound of the present invention and one further active substance from groups A) to O) | | |
|---|---|---|
| **composition** | **Component 1** | **Component 2** |
| C-1 | one individualized compound I | Azoxystrobin |
| C-2 | one individualized compound I | Coumethoxystrobin |
| C-3 | one individualized compound I | Coumoxystrobin |
| C-4 | one individualized compound I | Dimoxystrobin |
| C-5 | one individualized compound I | Enestroburin |
| C-6 | one individualized compound I | Fenaminstrobin |
| C-7 | one individualized compound I | Fenoxystrobin/Flufenoxystrobin |
| C-8 | one individualized compound I | Fluoxastrobin |
| C-9 | one individualized compound I | Kresoxim-methyl |
| C-10 | one individualized compound I | Metominostrobin |
| C-11 | one individualized compound I | Orysastrobin |
| C-12 | one individualized compound I | Picoxystrobin |
| C-13 | one individualized compound I | Pyraclostrobin |
| C-14 | one individualized compound I | Pyrametostrobin |
| C-15 | one individualized compound I | Pyraoxystrobin |
| C-16 | one individualized compound I | Pyribencarb |
| C-17 | one individualized compound I | Trifloxystrobin |
| C-18 | one individualized compound I | Triclopyricarb/Chlorodincarb |
| C-19 | one individualized compound I | 2-[2-(2,5-dimethyl-phenoxymethyl)phenyl]-3-methoxy-acrylic acid methyl ester |
| C-20 | one individualized compound I | 2-(2-(3-(2,6-dichlorophenyl)-1-methylallylideneaminooxymethyl)-phenyl)-2-methoxyimino-N-methyl-acetamide |
| C-21 | one individualized compound I | Benalaxyl |
| C-22 | one individualized compound I | Benalaxyl-M |
| C-23 | one individualized compound I | Benodanil |
| C-24 | one individualized compound I | Benzovindiflupyr |
| C-25 | one individualized compound I | Bixafen |
| C-26 | one individualized compound I | Boscalid |
| C-27 | one individualized compound I | Carboxin |
| C-28 | one individualized compound I | Fenfuram |
| C-29 | one individualized compound I | Fenhexamid |
| C-30 | one individualized compound I | Flutolanil |
| C-31 | one individualized compound I | Fluxapyroxad |
| C-32 | one individualized compound I | Furametpyr |
| C-33 | one individualized compound I | Isopyrazam |
| C-34 | one individualized compound I | Isotianil |
| C-35 | one individualized compound I | Kiralaxyl |
| C-36 | one individualized compound I | Mepronil |
| C-37 | one individualized compound I | Metalaxyl |
| C-38 | one individualized compound I | Metalaxyl-M |
| C-39 | one individualized compound I | Ofurace |
| C-40 | one individualized compound I | Oxadixyl |
| C-41 | one individualized compound I | Oxycarboxin |
| C-42 | one individualized compound I | Penflufen |
| C-43 | one individualized compound I | Penthiopyrad |
| C-44 | one individualized compound I | Sedaxane |
| C-45 | one individualized compound I | Tecloftalam |
| C-46 | one individualized compound I | Thifluzamide |
| C-47 | one individualized compound I | Tiadinil |
| C-48 | one individualized compound I | 2-Amino-4-methyl-thiazole-5-carboxylic acid anilide |
| C-49 | one individualized compound I | N-(4'-trifluoromethylthiobiphenyl-2-yl)-3-difluoromethyl-1-methyl-1H-pyrazole-4-carboxamide |
| C-50 | one individualized compound I | N-(2-(1,3,3-trimethyl-butyl)-phenyl)-1,3-dimethyl-5-fluoro-1H-pyrazole-4-carboxamide |
| C-51 | one individualized compound I | 3-(difluoromethyl)-1-methyl-N-(1,1,3-trimethylindan-4-yl)pyrazole-4-carboxamide |
| C-52 | one individualized compound I | 3-(trifluoromethyl)-1-methyl-N-(1,1,3-trimethylindan-4-yl)pyrazole-4-carboxamide |
| C-53 | one individualized compound I | 1,3-dimethyl-N-(1,1,3-trimethylindan-4-yl)pyrazole-4-carboxamide |
| C-54 | one individualized compound I | 3-(trifluoromethyl)-1,5-dimethyl-N-(1,1,3-trimethylindan-4-yl)pyrazole-4-carboxamide |
| C-55 | one individualized compound I | 3-(difluoromethyl)-1,5-dimethyl-N-(1,1,3-trimethylindan-4-yl)pyrazole-4-carboxamide |
| C-56 | one individualized compound I | 1,3,5-trimethyl-N-(1,1,3-trimethylindan-4-yl)pyrazole-4-carboxamide |
| C-57 | one individualized compound I | Dimethomorph |
| C-58 | one individualized compound I | Flumorph |
| C-59 | one individualized compound I | Pyrimorph |
| C-60 | one individualized compound I | Flumetover |
| C-61 | one individualized compound I | Fluopicolide |
| C-62 | one individualized compound I | Fluopyram |
| C-63 | one individualized compound I | Zoxamide |
| C-64 | one individualized compound I | Carpropamid |
| C-65 | one individualized compound I | Diclocymet |
| C-66 | one individualized compound I | Mandipropamid |
| C-67 | one individualized compound I | Oxytetracyclin |
| C-68 | one individualized compound I | Silthiofam |
| C-69 | one individualized compound I | N-(6-methoxy-pyridin-3-yl) cyclopropanecarboxylic acid amide |
| C-70 | one individualized compound I | Azaconazole |
| C-71 | one individualized compound I | Bitertanol |
| C-72 | one individualized compound I | Bromuconazole |
| C-73 | one individualized compound I | Cyproconazole |
| C-74 | one individualized compound I | Difenoconazole |
| C-75 | one individualized compound I | Diniconazole |
| C-76 | one individualized compound I | Diniconazole-M |
| C-77 | one individualized compound I | Epoxiconazole |
| C-78 | one individualized compound I | Fenbuconazole |
| C-79 | one individualized compound I | Fluquinconazole |
| C-80 | one individualized compound I | Flusilazole |
| C-81 | one individualized compound I | Flutriafol |
| C-82 | one individualized compound I | Hexaconazol |
| C-83 | one individualized compound I | Imibenconazole |
| C-84 | one individualized compound I | Ipconazole |
| C-85 | one individualized compound I | Metconazole |
| C-86 | one individualized compound I | Myclobutanil |
| C-87 | one individualized compound I | Oxpoconazol |
| C-88 | one individualized compound I | Paclobutrazol |
| C-89 | one individualized compound I | Penconazole |
| C-90 | one individualized compound I | Propiconazole |
| C-91 | one individualized compound I | Prothioconazole |
| C-92 | one individualized compound I | Simeconazole |
| C-93 | one individualized compound I | Tebuconazole |
| C-94 | one individualized compound I | Tetraconazole |
| C-95 | one individualized compound I | Triadimefon |
| C-96 | one individualized compound I | Triadimenol |
| C-97 | one individualized compound I | Triticonazole |
| C-98 | one individualized compound I | Uniconazole |
| C-99 | one individualized compound I | 1-[rel-(2*S*;3*R*)-3-(2-chlorophenyl)-2-(2,4-difluorophenyl)-oxiranylmethyl]-5-thiocyanato-1H-[1,2,4]triazole, |
| C-100 | one individualized compound I | 2-[*rel*-(*2S*;3*R*)-3-(2-chlorophenyl)-2-(2,4-difluorophenyl)-oxiranylmethyl]-2H-[1,2,4]triazole-3-thiol |
| C-101 | one individualized compound I | Cyazofamid |
| C-102 | one individualized compound I | Amisulbrom |
| C-103 | one individualized compound I | Imazalil |
| C-104 | one individualized compound I | Imazalil-sulfate |
| C-105 | one individualized compound I | Pefurazoate |
| C-106 | one individualized compound I | Prochloraz |
| C-107 | one individualized compound I | Triflumizole |
| C-108 | one individualized compound I | Benomyl |
| C-109 | one individualized compound I | Carbendazim |
| C-110 | one individualized compound I | Fuberidazole |
| C-111 | one individualized compound I | Thiabendazole |
| C-112 | one individualized compound I | Ethaboxam |
| C-113 | one individualized compound I | Etridiazole |
| C-114 | one individualized compound I | Hymexazole |
| C-115 | one individualized compound I | 2-(4-Chloro-phenyl)-N-[4-(3,4-dimethoxy-phenyl)-isoxazol-5-yl]-2-prop-2-ynyloxy-acetamide |
| C-116 | one individualized compound I | Fluazinam |
| C-117 | one individualized compound I | Pyrifenox |
| C-118 | one individualized compound I | 3-[5-(4-Chloro-phenyl)-2,3-dimethyl-is-oxazolidin-3-yl]-pyridine (Pyrisoxazole) |
| C-119 | one individualized compound I | 3-[5-(4-Methyl-phenyl)-2,3-dimethylisoxazolidin-3-yl]-pyridine |
| C-120 | one individualized compound I | Bupirimate |
| C-121 | one individualized compound I | Cyprodinil |
| C-122 | one individualized compound I | 5-Fluorocytosine |
| C-123 | one individualized compound I | 5-Fluoro-2-(p-tolylmethoxy)pyrimidin-4-amine |
| C-124 | one individualized compound I | 5-Fluoro-2-(4-fluorophenylmethoxy)-pyrimidin-4-amine |
| C-125 | one individualized compound I | Diflumetorim |
| C-126 | one individualized compound I | (5,8-Difluoroquinazolin-4-yl)-{2-[2-fluoro-4-(4-trifluoromethylpyridin-2-yloxy)phenyl]-ethyl}-amine |
| C-127 | one individualized compound I | Fenarimol |
| C-128 | one individualized compound I | Ferimzone |
| C-129 | one individualized compound I | Mepanipyrim |
| C-130 | one individualized compound I | Nitrapyrin |
| C-131 | one individualized compound I | Nuarimol |
| C-132 | one individualized compound I | Pyrimethanil |
| C-133 | one individualized compound I | Triforine |
| C-134 | one individualized compound I | Fenpiclonil |
| C-135 | one individualized compound I | Fludioxonil |
| C-136 | one individualized compound I | Aldimorph |
| C-137 | one individualized compound I | Dodemorph |
| C-138 | one individualized compound I | Dodemorph-acetate |
| C-139 | one individualized compound I | Fenpropimorph |
| C-140 | one individualized compound I | Tridemorph |
| C-141 | one individualized compound I | Fenpropidin |
| C-142 | one individualized compound I | Fluoroimid |
| C-143 | one individualized compound I | Iprodione |
| C-144 | one individualized compound I | Procymidone |
| C-145 | one individualized compound I | Vinclozolin |
| C-146 | one individualized compound I | Famoxadone |
| C-147 | one individualized compound I | Fenamidone |
| C-148 | one individualized compound I | Flutianil |
| C-149 | one individualized compound I | Octhilinone |
| C-150 | one individualized compound I | Probenazole |
| C-151 | one individualized compound I | Fenpyrazamine |
| C-152 | one individualized compound I | Acibenzolar-S-methyl |
| C-153 | one individualized compound I | Ametoctradin |
| C-154 | one individualized compound I | Amisulbrom |
| C-155 | one individualized compound I | [(3S,6S,7R,8R)-8-benzyl-3-[(3-isobutyryloxymethoxy-4-methoxypyridine-2-carbonyl)amino]-6-methyl-4,9-dioxo-[1,5]dioxonan-7-yl] 2-methylpropanoate |
| C-156 | one individualized compound I | [(3S,6S,7R,8R)-8-benzyl-3-[(3-acetoxy-4-methoxy-pyridine-2-carbonyl)amino]-6-methyl-4,9-dioxo-1,5-dioxonan-7-yl] 2-methylpropanoate |
| C-157 | one individualized compound I | [(3S,6S,7R,8R)-8-benzyl-3-[[3-(acetoxymethoxy)-4-methoxy-pyridine-2-carbonyl]amino]-6-methyl-4,9-dioxo-1,5-dioxonan-7-yl] 2-methylpropanoate |
| C-158 | one individualized compound I | [(3S,6S,7R,8R)-8-benzyl-3-[(3-isobutoxycarbonyloxy-4-methoxy-pyridine-2-carbonyl)amino]-6-methyl-4,9-dioxo-1,5-dioxonan-7-yl] 2-methylpropanoate |
| C-159 | one individualized compound I | [(3S,6S,7R,8R)-8-benzyl-3-[[3-(1,3-benzodioxol-5-ylmethoxy)-4-methoxypyridine-2-carbonyl]amino]-6-methyl-4,9-dioxo-1,5-dioxonan-7-yl] 2-methylpropanoate |
| C-160 | one individualized compound I | (3S,6S,7R,8R)-3-[[(3-hydroxy-4-methoxy-2-pyridinyl)carbonyl]amino]-6-methyl-4,9-dioxo-8-(phenylmethyl)-1,5-dioxonan-7-yl 2-methylpropanoate |
| C-161 | one individualized compound I | Anilazin |
| C-162 | one individualized compound I | Blasticidin-S |
| C-163 | one individualized compound I | Captafol |
| C-164 | one individualized compound I | Captan |
| C-165 | one individualized compound I | Chinomethionat |
| C-166 | one individualized compound I | Dazomet |
| C-167 | one individualized compound I | Debacarb |
| C-168 | one individualized compound I | Diclomezine |
| C-169 | one individualized compound I | Difenzoquat, |
| C-170 | one individualized compound I | Difenzoquat-methylsulfate |
| C-171 | one individualized compound I | Fenoxanil |
| C-172 | one individualized compound I | Folpet |
| C-173 | one individualized compound I | Oxolinsäure |
| C-174 | one individualized compound I | Piperalin |
| C-175 | one individualized compound I | Proquinazid |
| C-176 | one individualized compound I | Pyroquilon |
| C-177 | one individualized compound I | Quinoxyfen |
| C-178 | one individualized compound I | Triazoxid |
| C-179 | one individualized compound I | Tricyclazole |
| C-180 | one individualized compound I | 2-Butoxy-6-iodo-3-propyl-chromen-4-one |
| C-181 | one individualized compound I | 5-Chloro-1-(4,6-dimethoxy-pyrimidin-2-yl)-2-methyl-1H-benzoimidazole |
| C-182 | one individualized compound I | 5-Ch loro-7-(4-methyl-piperidin-1-yl)-6-(2,4,6-trifluoro-phenyl)-[1,2,4]triazolo[1,5-a]pyrimidine |
| C-183 | one individualized compound I | Ferbam |
| C-184 | one individualized compound I | Mancozeb |
| C-185 | one individualized compound I | Maneb |
| C-186 | one individualized compound I | Metam |
| C-187 | one individualized compound I | Methasulphocarb |
| C-188 | one individualized compound I | Metiram |
| C-189 | one individualized compound I | Propineb |
| C-190 | one individualized compound I | Thiram |
| C-191 | one individualized compound I | Zineb |
| C-192 | one individualized compound I | Ziram |
| C-193 | one individualized compound I | Diethofencarb |
| C-194 | one individualized compound I | Benthiavalicarb |
| C-195 | one individualized compound I | Iprovalicarb |
| C-196 | one individualized compound I | Propamocarb |
| C-197 | one individualized compound I | Propamocarb hydrochlorid |
| C-198 | one individualized compound I | Valifenalate |
| C-199 | one individualized compound I | N-(1-(1-(4-cyanophenyl)ethanesulfonyl)-but-2-yl) carbamic acid-(4-fluorophenyl) ester |
| C-200 | one individualized compound I | Dodine |
| C-201 | one individualized compound I | Dodine free base |
| C-202 | one individualized compound I | Guazatine |
| C-203 | one individualized compound I | Guazatine-acetate |
| C-204 | one individualized compound I | Iminoctadine |
| C-205 | one individualized compound I | Iminoctadine-triacetate |
| C-206 | one individualized compound I | Iminoctadine-tris(albesilate) |
| C-207 | one individualized compound I | Kasugamycin |
| C-208 | one individualized compound I | Kasugamycin-hydrochloride-hydrate |
| C-209 | one individualized compound I | Polyoxine |
| C-210 | one individualized compound I | Streptomycin |
| C-211 | one individualized compound I | Validamycin A |
| C-212 | one individualized compound I | Binapacryl |
| C-213 | one individualized compound I | Dicloran |
| C-214 | one individualized compound I | Dinobuton |
| C-215 | one individualized compound I | Dinocap |
| C-216 | one individualized compound I | Nitrothal-isopropyl |
| C-217 | one individualized compound I | Tecnazen |
| C-218 | one individualized compound I | Fentin salts |
| C-219 | one individualized compound I | Dithianon |
| C-220 | one individualized compound I | 2,6-dimethyl-1H,5H-[1,4]dithiino [2,3-c:5,6-c']dipyrrole-1,3,5,7(2H,6H)-tetraone |
| C-221 | one individualized compound I | Isoprothiolane |
| C-222 | one individualized compound I | Edifenphos |
| C-223 | one individualized compound I | Fosetyl, Fosetyl-aluminium |
| C-224 | one individualized compound I | Iprobenfos |
| C-225 | one individualized compound I | Phosphorous acid (H₃PO₃) and derivatives |
| C-226 | one individualized compound I | Pyrazophos |
| C-227 | one individualized compound I | Tolclofos-methyl |
| C-228 | one individualized compound I | Chlorothalonil |
| C-229 | one individualized compound I | Dichlofluanid |
| C-230 | one individualized compound I | Dichlorophen |
| C-231 | one individualized compound I | Flusulfamide |
| C-232 | one individualized compound I | Hexachlorbenzene |
| C-233 | one individualized compound I | Pencycuron |
| C-234 | one individualized compound I | Pentachlorophenol and salts |
| C-235 | one individualized compound I | Phthalide |
| C-236 | one individualized compound I | Quintozene |
| C-237 | one individualized compound I | Thiophanate Methyl |
| C-238 | one individualized compound I | Tolylfluanid |
| C-239 | one individualized compound I | N-(4-chloro-2-nitro-phenyl)-N-ethyl-4-methyl-benzenesulfonamide |
| C-240 | one individualized compound I | Bordeaux mixture |
| C-241 | one individualized compound I | Copper acetate |
| C-242 | one individualized compound I | Copper hydroxide |
| C-243 | one individualized compound I | Copper oxychloride |
| C-244 | one individualized compound I | basic Copper sulfate |
| C-245 | one individualized compound I | Sulfur |
| C-246 | one individualized compound I | Biphenyl |
| C-247 | one individualized compound I | Bronopol |
| C-248 | one individualized compound I | Cyflufenamid |
| C-249 | one individualized compound I | Cymoxanil |
| C-250 | one individualized compound I | Diphenylamin |
| C-251 | one individualized compound I | Metrafenone |
| C-252 | one individualized compound I | Pyriofenone |
| C-253 | one individualized compound I | Mildiomycin |
| C-254 | one individualized compound I | Oxin-copper |
| C-255 | one individualized compound I | Oxathiapiprolin |
| C-256 | one individualized compound I | Prohexadione calcium |
| C-257 | one individualized compound I | Spiroxamine |
| C-258 | one individualized compound I | Tebufloquin |
| C-259 | one individualized compound I | Tolylfluanid |
| C-260 | one individualized compound I | N-(Cyclopropylmethoxyimino-(6-difluoromethoxy-2,3-difluoro-phenyl)-methyl)-2-phenyl acetamide |
| C-261 | one individualized compound I | N'-(4-(4-chloro-3-trifluoromethylphenoxy)-2,5-dimethyl-phenyl)-N-ethyl-N-methyl formamidine |
| C-262 | one individualized compound I | N'-(4-(4-fluoro-3-trifluoromethylphenoxy)-2,5-dimethyl-phenyl)-N-ethyl-N-methyl formamidine |
| C-263 | one individualized compound I | N'-(2-methyl-5-trifluoromethyl-4-(3-trimethylsilanyl-propoxy)-phenyl)-N-ethyl-N-methyl formamidine |
| C-264 | one individualized compound I | N'-(5-difluoromethyl-2-methyl-4-(3-trimethylsilanyl-propoxy)-phenyl)-N-ethyl-N-methyl formamidine |
| C-265 | one individualized compound I | Methoxy-acetic acid 6-tert-butyl-8-fluoro-2,3-dimethyl-quinolin-4-yl ester |
| C-266 | one individualized compound I | *Bacillus subtilis* NRRL No. B-21661 |
| C-267 | one individualized compound I | *Bacillus pumilus* NRRL No. B-30087 |
| C-268 | one individualized compound I | *Ulocladium oudemansii* |
| C-269 | one individualized compound I | Carbaryl |
| C-270 | one individualized compound I | Carbofuran |
| C-271 | one individualized compound I | Carbosulfan |
| C-272 | one individualized compound I | Methomylthiodicarb |
| C-273 | one individualized compound I | Bifenthrin |
| C-274 | one individualized compound I | Cyfluthrin |
| C-275 | one individualized compound I | Cypermethrin |
| C-276 | one individualized compound I | alpha-Cypermethrin |
| C-277 | one individualized compound I | zeta-Cypermethrin |
| C-278 | one individualized compound I | Deltamethrin |
| C-279 | one individualized compound I | Esfenvalerate |
| C-280 | one individualized compound I | Lambda-cyhalothrin |
| C-281 | one individualized compound I | Permethrin |
| C-282 | one individualized compound I | Tefluthrin |
| C-283 | one individualized compound I | Diflubenzuron |
| C-284 | one individualized compound I | Flufenoxuron |
| C-285 | one individualized compound I | Lufenuron |
| C-286 | one individualized compound I | Teflubenzuron |
| C-287 | one individualized compound I | Spirotetramate |
| C-288 | one individualized compound I | Clothianidin |
| C-289 | one individualized compound I | Dinotefuran |
| C-290 | one individualized compound I | Imidacloprid |
| C-291 | one individualized compound I | Thiamethoxam |
| C-292 | one individualized compound I | Flupyradifurone |
| C-293 | one individualized compound I | Acetamiprid |
| C-294 | one individualized compound I | Thiacloprid |
| C-295 | one individualized compound I | Endosulfan |
| C-296 | one individualized compound I | Fipronil |
| C-297 | one individualized compound I | Abamectin |
| C-298 | one individualized compound I | Emamectin |
| C-299 | one individualized compound I | Spinosad |
| C-300 | one individualized compound I | Spinetoram |
| C-301 | one individualized compound I | Hydramethylnon |
| C-302 | one individualized compound I | Chlorfenapyr |
| C-303 | one individualized compound I | Fenbutatin oxide |
| C-304 | one individualized compound I | Indoxacarb |
| C-305 | one individualized compound I | Metaflumizone |
| C-306 | one individualized compound I | Flonicamid |
| C-307 | one individualized compound I | Lubendiamide |
| C-308 | one individualized compound I | Chlorantraniliprole |
| C-309 | one individualized compound I | Cyazypyr (HGW86) |
| C-310 | one individualized compound I | Cyflumetofen |
| C-311 | one individualized compound I | Acetochlor |
| C-312 | one individualized compound I | Dimethenamid |
| C-313 | one individualized compound I | metolachlor |
| C-314 | one individualized compound I | Metazachlor |
| C-315 | one individualized compound I | Glyphosate |
| C-316 | one individualized compound I | Glufosinate |
| C-317 | one individualized compound I | Sulfosate |
| C-318 | one individualized compound I | Clodinafop |
| C-319 | one individualized compound I | Fenoxaprop |
| C-320 | one individualized compound I | Fluazifop |
| C-321 | one individualized compound I | Haloxyfop |
| C-322 | one individualized compound I | Paraquat |
| C-323 | one individualized compound I | Phenmedipham |
| C-324 | one individualized compound I | Clethodim |
| C-325 | one individualized compound I | Cycloxydim |
| C-326 | one individualized compound I | Profoxydim |
| C-327 | one individualized compound I | Sethoxydim |
| C-328 | one individualized compound I | Tepraloxydim |
| C-329 | one individualized compound I | Pendimethalin |
| C-330 | one individualized compound I | Prodiamine |
| C-331 | one individualized compound I | Trifluralin |
| C-332 | one individualized compound I | Acifluorfen |
| C-333 | one individualized compound I | Bromoxynil |
| C-334 | one individualized compound I | Imazamethabenz |
| C-335 | one individualized compound I | Imazamox |
| C-336 | one individualized compound I | Imazapic |
| C-337 | one individualized compound I | Imazapyr |
| C-338 | one individualized compound I | Imazaquin |
| C-339 | one individualized compound I | Imazethapyr |
| C-340 | one individualized compound I | 2,4-Dichlorophenoxyacetic acid (2,4-D) |
| C-341 | one individualized compound I | Chloridazon |
| C-342 | one individualized compound I | Clopyralid |
| C-343 | one individualized compound I | Fluroxypyr |
| C-344 | one individualized compound I | Picloram |
| C-345 | one individualized compound I | Picolinafen |
| C-346 | one individualized compound I | Bensulfuron |
| C-347 | one individualized compound I | Chlorimuron-ethyl |
| C-348 | one individualized compound I | Cyclosulfamuron |
| C-349 | one individualized compound I | Iodosulfuron |
| C-350 | one individualized compound I | Mesosulfuron |
| C-351 | one individualized compound I | Metsulfuron-methyl |
| C-352 | one individualized compound I | Nicosulfuron |
| C-353 | one individualized compound I | Rimsulfuron |
| C-354 | one individualized compound I | Triflusulfuron |
| C-355 | one individualized compound I | Atrazine |
| C-356 | one individualized compound I | Hexazinone |
| C-357 | one individualized compound I | Diuron |
| C-358 | one individualized compound I | Florasulam |
| C-359 | one individualized compound I | Pyroxasulfone |
| C-360 | one individualized compound I | Bentazone |
| C-361 | one individualized compound I | Cinidon-ethyl |
| C-362 | one individualized compound I | Cinmethylin |
| C-363 | one individualized compound I | Dicamba |
| C-364 | one individualized compound I | Diflufenzopyr |
| C-365 | one individualized compound I | Quinclorac |
| C-366 | one individualized compound I | Quinmerac |
| C-367 | one individualized compound I | Mesotrione |
| C-368 | one individualized compound I | Saflufenacil |
| C-369 | one individualized compound I | Topramezone |
| C-370 | one individualized compound I | 1,1'-[(3S,4R,4aR,6S,6aS,12R,12aS, 12bS)-4-[[(2-cyclopropylacetyl)oxy]methyl]-1,3,4,4a,5,6,6a,12,12a,12b-decahydro-12-hydroxy-4,6a,12b-trimethyl-11-oxo-9-(3-pyridinyl)-2H,11H-naphtho[2,1-b]pyrano[3,4-e]pyran-3,6-diyl] cyclopropaneacetic acid ester |

The active substances referred to as component 2, their preparation and their activity against harmful fungi is known (cf.: http://www.alanwood.net/pesticides/); these substances are commercially available. The compounds described by IUPAC nomenclature, their preparation and their fungicidal activity are also known (cf. Can. J. Plant Sci. 48(6), 587-94, 1968; EP-A 141 317; EP-A 152 031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 11/028657).

The composition of active substances can be prepared as compositions comprising besides the active ingredients at least one inert ingredient by usual means, e. g. by the means given for the compositions of compounds I. Concerning usual ingredients of such compositions reference is made to the explanations given for the compositions containing compounds I. The compositions of active substances according to the present invention are suitable as fungicides, as are the compounds of formula I. They are distinguished by an outstanding effectiveness against a broad spectrum of phytopathogenic fungi, especially from the classes of the Ascomycetes, Basidiomycetes, Deuteromycetes and Peronosporomycetes (syn. Oomycetes). In addition, it is refered to the explanations regarding the fungicidal activity of the compounds and the compositions containing compounds I, respectively.

## Claims

1. Compounds of the formula I 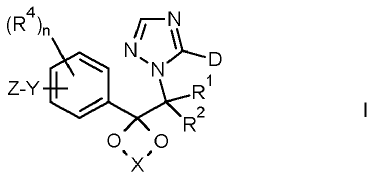 in which
D is H, halogen or SR^{D}, wherein
R^{D} is hydrogen, C₁-C₆-alkyl, C₁-C₆-haloalkyl, C₂-C₆-alkenyl,
C₂-C₆-haloalkenyl, C₂-C₆-alkynyl, C₂-C₆-haloalkynyl or CN;
R¹, R² are hydrogen;
X is -CR⁵R⁶-CR⁷R⁸- or -CR⁵R⁶-CR⁷R⁸-CR⁹R¹⁰-, wherein
R⁵,R⁶,R⁷,R⁸,R⁹,R¹⁰ are independently selected from hydrogen, halogen, C₁-C₆-alkyl, C₂-C₆-alkenyl, C₂-C₆-alkynyl, C₁-C₆-alkoxy and phenyl; wherein these substituents are unsubstituted or substituted by one, two, three or up to the maximum possible number of R^{x}; wherein
R^{x} is independently selected from halogen, CN, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio, C₁-C₆-haloalkylthio, C₁-C₄-alkoxy-C₁-C₄-alkyl, phenyl and phenoxy Y is a direct bond or a divalent group selected from the group consisting of -
O-, -S-, -SO-, -SO₂-, -NH-, -N(C₁-C₄-alkyl)-, -CR¹²R¹³-, -CR¹²R¹³-CR¹⁴R¹⁵-, -CR¹⁶=CR¹⁷-and -C≡C-; wherein
R¹²,R¹³,R¹⁴,R¹⁵,R¹⁶,R¹⁷ are independently selected from hydrogen, halogen, CN, nitro, OH, C₁-C₄-alkyl, C₁-C₄-halogenalkyl, C₁-C₄-alkoxy and C₁-C₄-halogenalkoxy;
Z is five or six-membered heteroaryl or phenyl, wherein the heteroaryl contains 1, 2, 3 or 4 heteroatoms selected from the group consisting of O, N and S, and wherein the heteroaryl or phenyl is unsubstituted (m=0) or substituted by (R^{L})ₘ, wherein
m is 0, 1, 2, 3 or 4; and wherein
R^{L} is independently selected from halogen, CN, NO₂, OH,
C₁-C₆-alkyl, C₁-C₆-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cydoalkyl, C₃-C₈-cycloalkyl-C₁-C₄-alkyl, C₃-C₈-cycloalkyloxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH, C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl), C(=O)-N(C₃-C₆-cycloalkyl)₂, phenyl and phenyl-C₁-C₄-alkyl, wherein the aliphatic, alicyclic and aromatic moieties of R^{L} are unsubstituted or substituted by one, two, three or four or up to the maximum possible number of R^{La}; wherein
R^{La} is independently selected from halogen, CN, NO₂, OH, SH, NH₂, C₁-C₆-alkyl, C₁-C₆-haloalkyl, C₃-C₈-cycloalkyl, C₃-C₈-halocycloalkyl, C₁-C₆-alkoxy, C₁-C₆-haloalkoxy, C₁-C₆-alkylthio and C₁-C₆-haloalkylthio;
R⁴ is independently selected from halogen, CN, NO₂, OH, SH, C₁-C₄-alkyl, C₁C₁-C₄-alkoxy, C₁-C₆-alkylthio, C₁-C₆-alkylsulfinyl, C₁-C₆-alkylsulfonyl, C₂-C₆-alkenyl, C₂-C₆-alkynyl, C₃-C₈-cycloalkyl, C₃-C₈-cycloalkyl-C₁-C₄-alkyl, C₃-C₈-cycloalkyloxy, phenyl, phenoxy, 5- or 6-membered heteroaryl, 5- or 6-membered heteroaryloxy, NH₂, NH(C₁-C₄-alkyl), N(C₁-C₄-alkyl)₂, NH(C₃-C₆-cycloalkyl), N(C₃-C₆-cycloalkyl)₂, C(=O)-C₁-C₄-alkyl, C(=O)OH,
C(=O)-O-C₁-C₄-alkyl, C(=O)-NH(C₁-C₄-alkyl), C(=O)-N(C₁-C₄-alkyl)₂, C(=O)-NH(C₃-C₆-cycloalkyl) and C(=O)-N(C₃-C₆-cycloalkyl)₂; wherein the aliphatic, alicyclic and aromatic moieties of R⁴ are unsubstituted or substituted by one, two, three or four or up to the maximum possible number of R^{4a}; wherein
R^{4a} is independently selected from halogen, CN, NO₂, OH, C₁-C₄-alkyl, C₁-C₄-haloalkyl, C₃-C₈-cycloalkyl, C₃-C₈-halocycloalkyl, C₁-C₄-alkoxy and C₁-C₄-halogenalkoxy;
n is 0, 1, 2, 3 or 4;
with the proviso, that
if Z is phenyl, Y is a direct bond, n = 0 and D=H, the phenyl is substituted by (R^{L})ₘ with m=1, 2, 3 or 4; and
if Z is phenyl and Y is a divalent group selected from the group consisting of -O-, -S-, -NH- and -N(C₁-C₄-alkyl)-, Z-Y is not in the para-position in relation to the attachment of the phenyl group in formula I to the remaining backbone structure;
and the N-oxides and the agriculturally acceptable salts thereof.

2. The compounds of claim 1, wherein Z-Y is Z¹-Y¹, wherein
Y¹ is a direct bond
Z¹ is five or six-membered heteroaryl or phenyl, wherein the heteroaryl contains 1, 2, 3 or 4 heteroatoms selected from the group consisting of O, N and S, and wherein the heteroaryl is unsubstituted (m=0) or substituted by (R^{L})ₘ and wherein the phenyl is substituted by (R^{L})ₘ, wherein R^{L} is defined in claim 1.

3. The compounds of claim 2, wherein Z¹ is phenyl that is substituted by 1 or 2 R^{L}, wherein one R^{L} is in 4-position.

4. The compounds of any one of claims 1 to 3, wherein n is 1, 2 or 3.

5. The compounds of any one of claims 1 to 4, wherein X is -CR⁵R⁶-CR⁷R⁸-.

6. The compounds of any one of claims 1 to 5, wherein X is -CR⁵H-CR⁷H-.

7. The compounds of any one of claims 1 to 4, wherein X is -CR⁵R⁶-CR⁷R⁸-CR⁹R¹⁰- .

8. The compounds of claim 5, wherein X is -CR⁵H-CH₂-CR⁹H-.

9. A composition, comprising one compound of formula I, as defined in any of the claims 1 to 8, an N-oxide or an agriculturally acceptable salt thereof.

10. The composition according to claim 9, comprising additionally a further active substance.

11. A use of a compound of the formula I, as defined in any of the claims 1 to 8, and/or of an agriculturally acceptable salt thereof or of the compositions, as defined in any of the claims 9 or 10, for combating phytopathogenic fungi.

12. A method for combating harmful fungi, comprising treating the fungi or the materials, plants, the soil or seeds to be protected against fungal attack with an effective amount of at least one compound of formula I, as defined in any of the claims 1 to 8, or with a composition, as defined in any of the claims 9 or 10.

13. Seed, coated with at least one compound of the formula I, as defined in any of the claims 1 to 8, and/or an agriculturally acceptable salt thereof or with a composition, as defined in any of the claims 8 or 9, in an amount of from 0.1 to 10 kg per 100 kg of seed.
